# EUROPÄISCHE PATENTSCHRIFT

(11) **EP 3 044 284 B1**
(45) Veröffentlichungstag und Bekanntmachung des Hinweises auf die Patenterteilung: **13.11.2019**
(21) Anmeldenummer: 14750447.6
(22) Anmeldetag: 12.08.2014
(51) Int. Cl.: C09K 11/06, H05B 33/10

(54) **METALLKOMPLEXE**
METAL COMPLEXES
COMPLEXES MÉTALLIQUES

(30) Priorität: 11.09.2013 EP 13004411
(43) Veröffentlichungstag der Anmeldung: 20.07.2016
(73) Patentinhaber: Merck Patent GmbH, 64293 Darmstadt (DE)
(72) Erfinder: STOESSEL, Philipp, 60389 Frankfurt am Main (DE); KOENEN, Nils, 64347 Griesheim (DE); BREUNING, Esther, 64372 Ober-Ramstadt (DE)
(86) Internationale Anmeldenummer: PCT/EP2014/002209
(87) Internationale Veröffentlichungsnummer: WO 2015/036074

(56) Entgegenhaltungen:
- WO-A1-2010/108579
- DE-A1-102009 048 791
- K.K.LO ET AL: "Synthesis, photophysical and electrochemical properties, and biological labelling studies of luminescent cyclometallated iridium(III)bipyridine-aldehyde complexes", INORGANICA CHIMICA ACTA, Bd. 357, 2004, Seiten 3109-3118, XP55145959,
- LAMANSKY S ET AL: "Synthesis and Characterization of Phosphorescent Cyclometalated Iridium Complexes", INORGANIC CHEMISTRY, AMERICAN CHEMICAL SOCIETY, EASTON, US, Bd. 40, Nr. 7, 1. Januar 2001 (2001-01-01) , Seiten 1704-1711, XP002196399, ISSN: 0020-1669, DOI: 10.1021/IC0008969

## Beschreibung

Die vorliegende Erfindung betrifft Metallkomplexe sowie elektronische Vorrichtungen, insbesondere organische Elektrolumineszenzvorrichtungen, enthaltend diese Metallkomplexe.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, jedoch immer noch Verbesserungsbedarf, insbesondere im Hinblick auf Effizienz, Betriebsspannung und Lebensdauer.

Gemäß dem Stand der Technik werden in phosphoreszierenden OLEDs als Triplettemitter insbesondere Iridiumkomplexe eingesetzt, wie zum Beispiel Iridiumkomplexe, welche als Liganden Benzo[h]chinolin-Derivate enthalten. Generell sind bei phosphoreszierenden Emittern noch weitere Verbesserungen, insbesondere hinsichtlich Effizienz, Betriebsspannung und Lebensdauer, wünschenswert. Aus DE 10 2009 048791 sind verschiedene Metallkomplexe für die Verwendung in OLEDs bekannt. Benzo-[h]chinolinkomplexe, insbesondere solche mit einem ankondensierten aliphatischen Ring, werden jedoch nicht offenbart.

Aufgabe der vorliegenden Erfindung ist daher die Bereitstellung neuer Metallkomplexe, welche sich als Emitter für die Verwendung in OLEDs eignen und dabei zu verbesserten Eigenschaften der OLED, insbesondere hinsichtlich Effizienz, Betriebsspannung und/oder Lebensdauer führen.

Überraschend wurde gefunden, dass bestimmte, unten näher beschriebene Metallchelatkomplexe, welche im Liganden einen ankondensierten aliphatischen Fünfring aufweisen, diese Aufgabe lösen und verbesserte Eigenschaften in organischen Elektrolumineszenzvorrichtung zeigen. Insbesondere zeigen diese Metallkomplexe verbesserte Effizienz und Lebensdauer gegenüber den analogen Metallkomplexen, die diesen ankondensierten aliphatischen Fünfring nicht enthalten. Diese Metallkomplexe und elektronische Vorrichtungen, insbesondere organische Elektrolumineszenzvorrichtungen, welche diese Komplexe enthalten, sind daher der Gegenstand der vorliegenden Erfindung.

Gegenstand der Erfindung ist somit eine Verbindung gemäß Formel (1),

[Ir(L)ₙ(L')ₘ] Formel (1)

wobei die Verbindung der allgemeinen Formel (1) eine Teilstruktur Ir(L)ₙ der Formel (2) enthält: 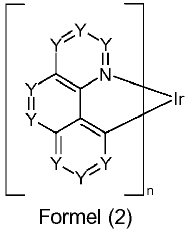 wobei für die verwendeten Symbole und Indizes gilt:
- Y: ist bei jedem Auftreten gleich oder verschieden CR oder N mit der Maßgabe, dass maximal ein Symbol Y pro Cyclus für N steht, oder zwei benachbarte Symbole Y stehen zusammen für eine Gruppe der folgenden Formel (3), 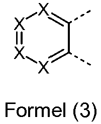 wobei die gestrichelten Bindungen die Verknüpfung dieser Gruppe im Liganden symbolisieren;
- X: ist bei jedem Auftreten gleich oder verschieden CR oder N mit der Maßgabe, dass maximal zwei Symbole X pro Ligand für N stehen;
- R: ist bei jedem Auftreten gleich oder verschieden H, D, Cl, Br, I, CN, eine geradkettige Alkyl- oder Alkoxygruppe mit 1 bis 40 C-Atomen oder eine verzweigte oder cyclische Alkyl- oder Alkoxygruppe mit 3 bis 40 C-Atomen oder ein aromatisches oder heteroaromatisches Ringsystem mit 5 bis 60 aromatischen Ringatomen, das jeweils durch einen oder mehrere Reste R¹ substituiert sein kann; dabei können zwei oder mehrere benachbarte Reste R auch miteinander ein mono- oder polycyclisches, aliphatisches Ringsystem bilden;
- R¹: ist bei jedem Auftreten gleich oder verschieden H, eine geradkettige Alkylgruppe mit 1 bis 40 C-Atomen oder eine verzweigte oder cyclische Alkylgruppe mit 3 bis 40 C-Atomen;
- L': ist gleich oder verschieden bei jedem Auftreten ein mono- oder bidentater Ligand;
- n: ist 2 oder 3;
- m: ist 0 oder 1;
dadurch gekennzeichnet, dass in der Teilstruktur der Formel (2) zwei benachbarte Gruppen Y für CR stehen und die jeweiligen Reste R zusammen mit den C-Atomen einen Ring der folgenden Formel (4) oder Formel (5) aufspannen,
und/oder dass zwei benachbarte Gruppen Y für eine Gruppe der Formel (3) stehen und in dieser Gruppe der Formel (3) zwei benachbarte Gruppen X für CR stehen und die die jeweiligen Reste R zusammen mit den C-Atomen einen Ring der folgenden Formel (4) oder Formel (5) aufspannen, 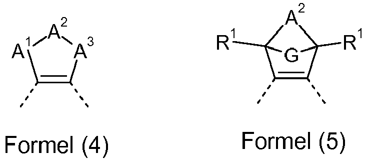 wobei R¹ und R² die oben genannten Bedeutungen aufweisen, die gestrichelten Bindungen die Verknüpfung der beiden Kohlenstoffatome im Liganden andeuten und weiterhin gilt:
- A¹, A³: ist gleich oder verschieden bei jedem Auftreten C(R³)₂ oder O;
- A²: ist C(R¹)₂;
- G: ist eine Alkylengruppe mit 2 C-Atomen;
- R³: ist gleich oder verschieden bei jedem Auftreten F, eine geradkettige Alkylgruppe mit 1 bis 10 C-Atomen, eine verzweigte oder cyclische Alkylgruppe mit 3 bis 10 C-Atomen oder ein aromatisches Ring-system mit 5 bis 24 aromatischen Ringatomen; dabei können zwei Reste R³, welche an dasselbe Kohlenstoffatom gebunden sind, miteinander ein aliphatisches Ringsystem bilden und so ein Spiro-system aufspannen.

Dabei werden die Indizes n und m so gewählt, dass die Koordinationszahl am Iridium insgesamt 6 entspricht. Dies ist insbesondere davon abhängig, wie viele Liganden L vorhanden sind und ob es sich bei den Liganden L' um mono- oder bidentate Liganden handelt.

In der folgenden Beschreibung bedeutet "benachbarte Gruppen Y" bzw. "benachbarte Gruppen X", dass die Gruppen Y bzw. X in der Struktur direkt aneinander gebunden sind.

Weiterhin bedeutet "benachbart" in der Definition der Reste, dass diese Reste an dasselbe oder an direkt aneinander gebundene C-Atome gebunden sind oder, wenn sie nicht an direkt gebundene C-Atome gebunden sind, dass es sich um die nächstmögliche Position handelt, in der ein Substituent gebunden sein kann. Dies wird anhand von einem spezifischen Liganden in der folgenden schematischen Darstellung für benachbarte Reste nochmals erläutert:

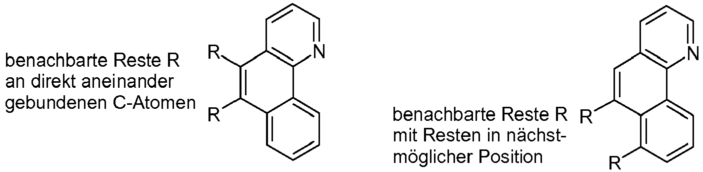

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom oder eine Carbonylgruppe, verbunden sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylengruppe oder durch eine Silylengruppe verbunden sind.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, neo-Pentyl, Cyclopentyl, n-Hexyl, s-Hexyl, tert-Hexyl, 2-Hexyl, 3-Hexyl, Cyclohexyl, 2-Methylpentyl, neo-Hexyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Trifluormethyl, Pentafluorethyl oder 2,2,2-Trifluorethyl verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die erfindungsgemäßen Komplexe können facial bzw. pseudofacial sein, oder sie können meridional bzw. pseudomeridional sein.

In einer bevorzugten Ausführungsform ist der Index n = 3, d. h. es handelt sich um einen homoleptischen Metallkomplex und der Index m = 0.

In einer weiteren bevorzugten Ausführungsform ist der Index n = 2 und m = 1, der erfindungsgemäße Komplex enthält zwei Liganden L und einen bidenten Liganden L'. Dabei ist bevorzugt, wenn der Ligand L' ein Ligand ist, der über ein Kohlenstoffatom und ein Stickstoffatom, ein Kohlenstoffatom und ein Sauerstoffatom, zwei Sauerstoffatome, zwei Stickstoffatome oder ein Sauerstoff- und ein Stickstoffatom an das Iridium koordinieren.

In einer weiteren bevorzugten Ausführungsform der Erfindung sind in den erfindungsgemäßen Verbindungen keine, eine oder zwei Gruppen der Formel (3) vorhanden. Die Teilstrukturen Ir(L)ₙ sind daher bevorzugt ausgewählt aus den folgenden Formeln (6) bis (15), 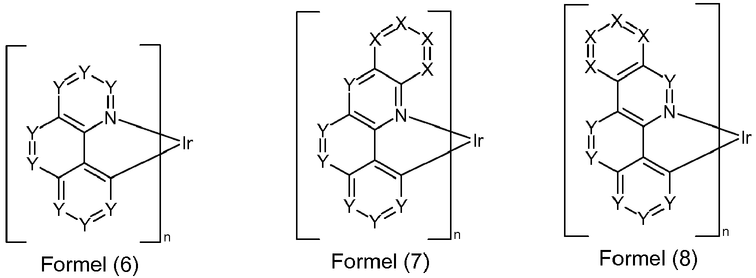 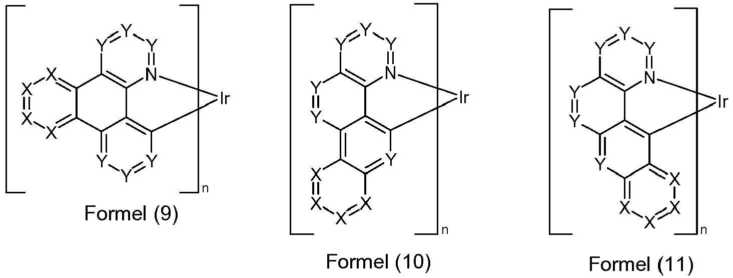 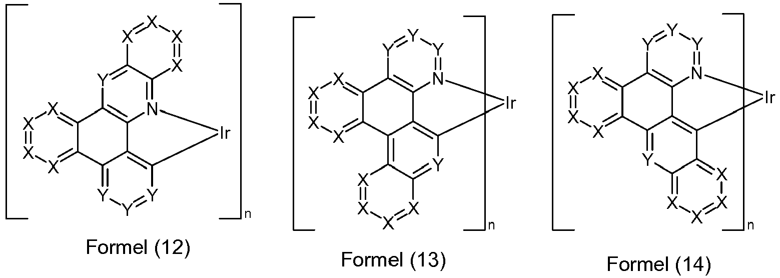 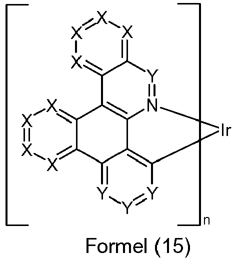 wobei Y bei jedem Auftreten gleich oder verschieden für CR oder N steht und die weiteren Symbole und Indizes die oben genannten Bedeutungen aufweisen.

In einer bevorzugten Ausführungsform der Erfindung stehen im Liganden L insgesamt 0, 1 oder 2 der Symbole Y und, falls vorhanden, X für N. Besonders bevorzugt stehen im Liganden L insgesamt 0 oder 1 der Symbole Y und, falls vorhanden, X für N. Ganz besonders bevorzugt stehen alle Symbole X und Y für CR.

In einer bevorzugten Ausführungsform der Erfindung steht die Gruppe Y, die in ortho-Position zur Koordination an das Iridium vorliegt, für CR. Dabei ist in dieser Rest R, der in ortho-Position zur Koordination an das Iridium gebunden ist, bevorzugt ausgewählt aus der Gruppe bestehend aus H, D, CN und Methyl.

Bevorzugte Ausführungsformen der Formel (6) bis (12) sind die Strukturen der folgenden Formeln (6-1) bis (6-7), (7-1) bis (7-6), (8-1) bis (8-5), (9-1) bis (9-5), (10-1) bis (10-5), (11-1) bis (11-6), (12-1) bis (12-4), (13-1) bis (13-3), (14-1) bis (14-4) und (15-1) bis (15-3), 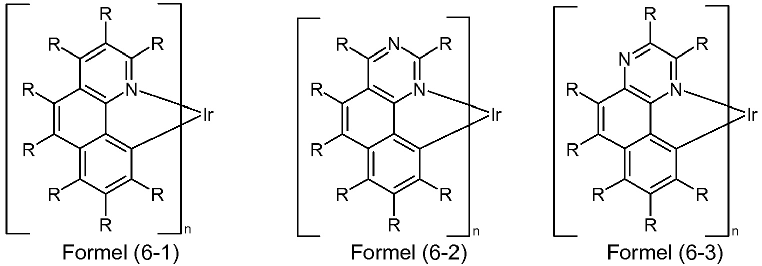 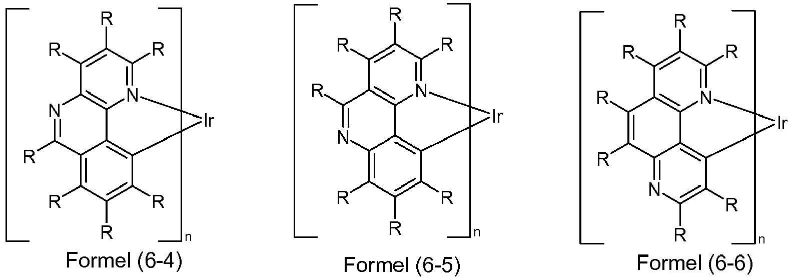 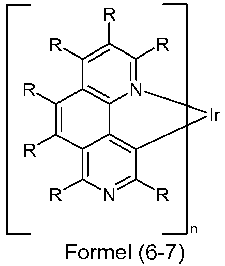 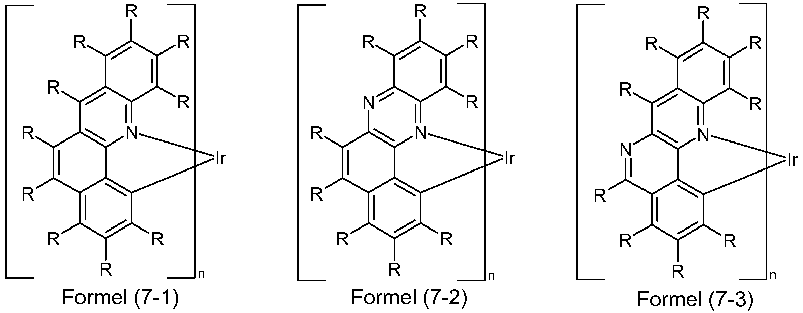 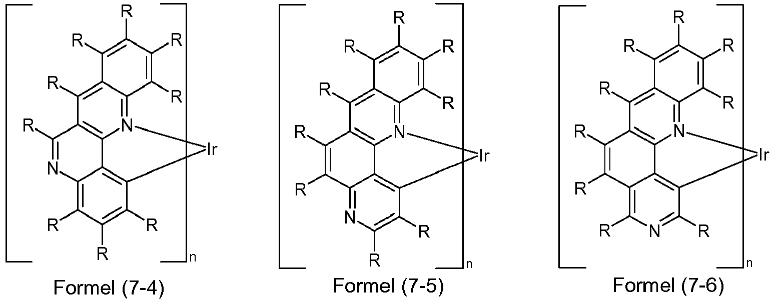 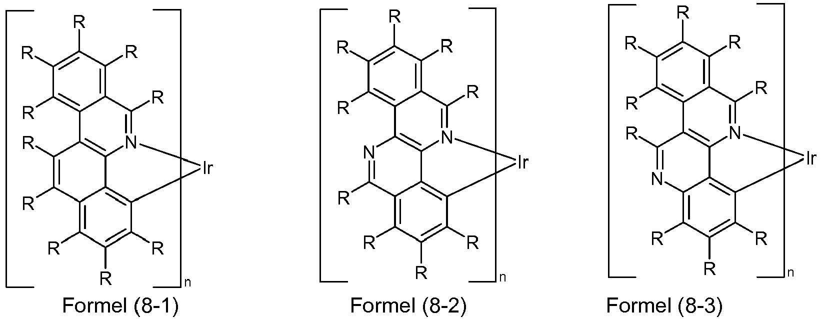 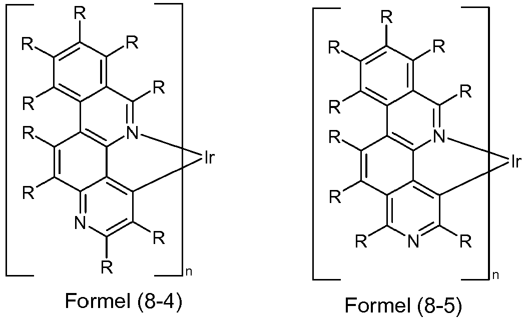 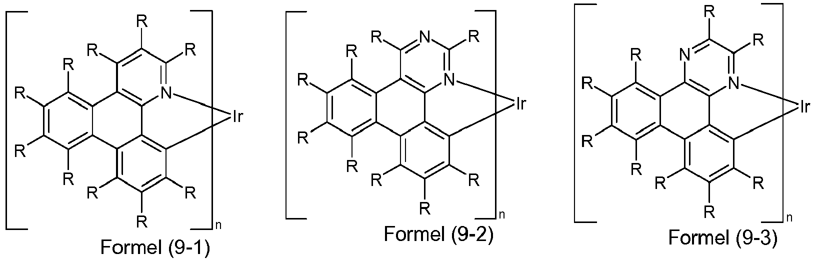 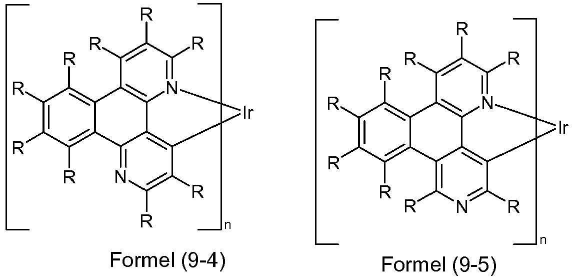 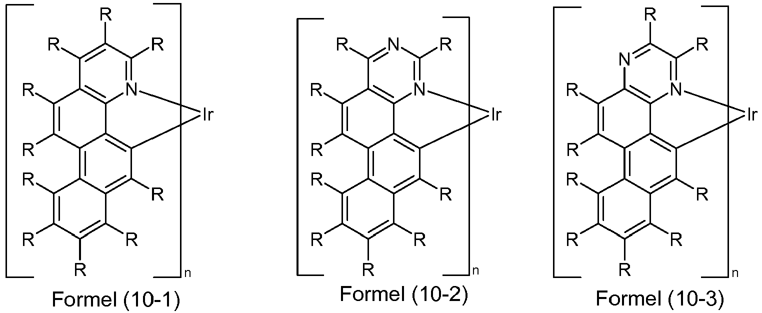 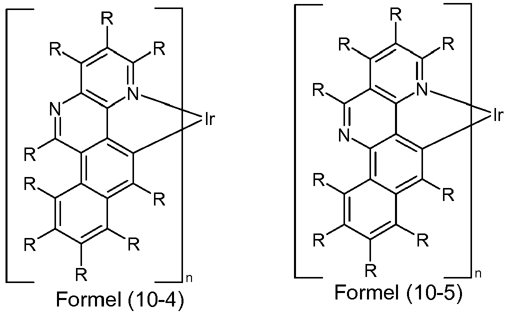 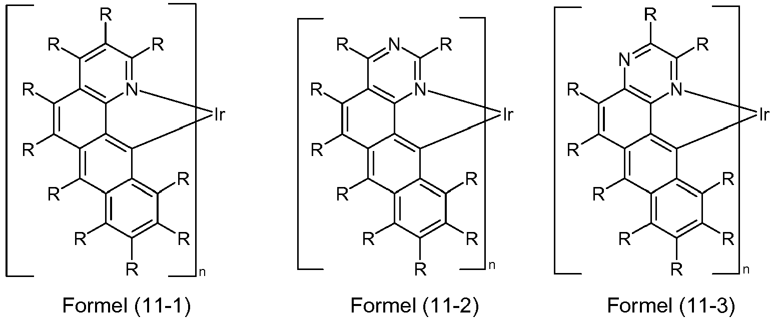 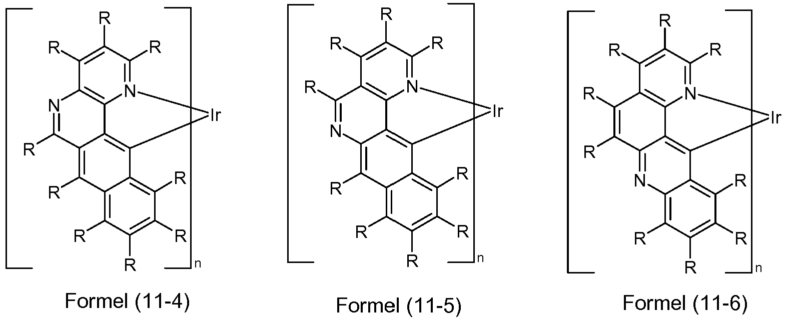 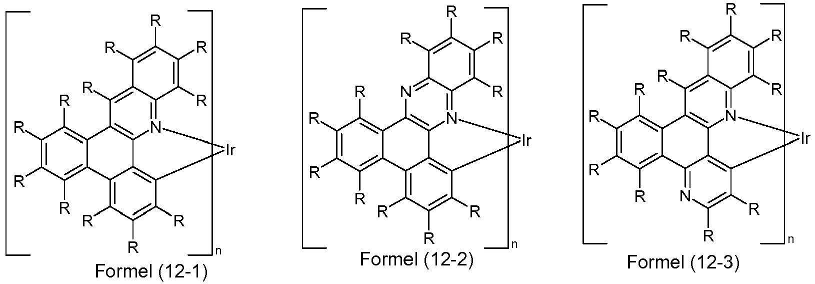 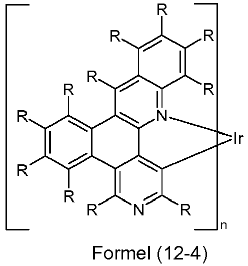 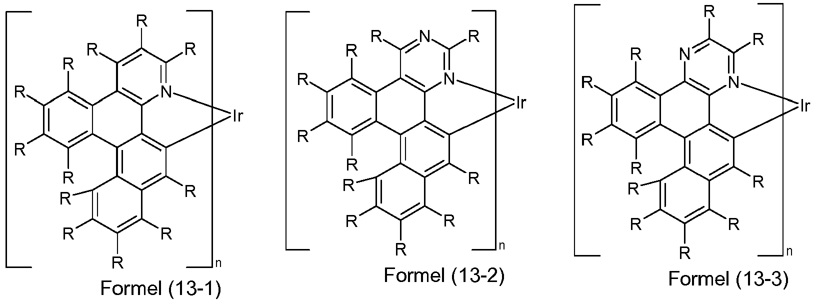 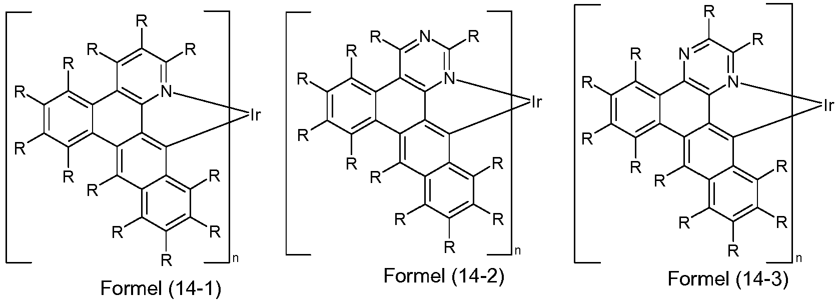 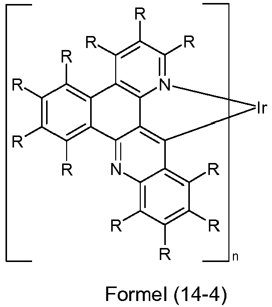 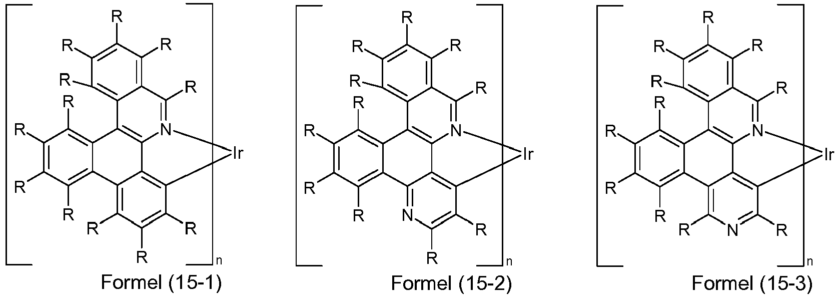 wobei die verwendeten Symbole und Indizes die oben genannten Bedeutungen aufweisen.

In einer Ausführungsform der Erfindung ist es bevorzugt, falls eines der Atome Y oder, wenn vorhanden, X für N steht, wenn benachbart zu diesem Stickstoffatom eine Gruppe R als Substituent gebunden ist, welche ungleich Wasserstoff oder Deuterium ist.

Dabei ist dieser Substituent R bevorzugt eine Gruppe, ausgewählt aus Alkyl- oder Alkoxygruppen mit 1 bis 10 C-Atomen, insbesondere verzweigten oder cyclischen Alkyl- oder Alkoxygruppen mit 3 bis 10 C-Atomen oder aromatischen bzw. heteroaromatischen Ringsystemen, die durch einen oder mehrere Substituenten R¹ substituiert sein können. Es handelt sich bei diesen Gruppen um sterisch anspruchsvolle Gruppen. Weiterhin bevorzugt kann dieser Rest R auch mit einem benachbarten Rest R einen Cyclus bilden. Dabei handelt es sich dann bevorzugt um Strukturen der Formel (4) oder (5), wie sie erfindungsgemäß in den Verbindungen der vorliegenden Erfindung vorliegen.

Wenn der Rest R, der einem Stickstoffatom benachbart ist, für eine Alkylgruppe steht, dann weist diese Alkylgruppe bevorzugt 3 bis 10 C-Atome auf. Bevorzugt handelt es sich weiterhin um eine sekundäre oder tertiäre Alkylgruppe, bei der das sekundäre oder tertiäre C-Atom entweder direkt an den Liganden gebunden ist oder über eine CH₂-Gruppe an den Liganden gebunden ist. Besonders bevorzugt ist diese Alkylgruppe ausgewählt aus den Strukturen der folgenden Formeln (R-1) bis (R-33), wobei jeweils auch die Anknüpfung dieser Gruppen an den Liganden mit eingezeichnet ist: 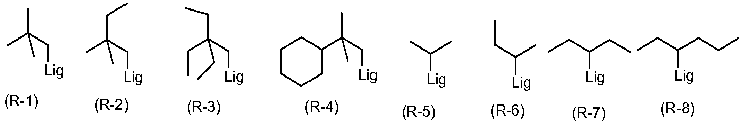 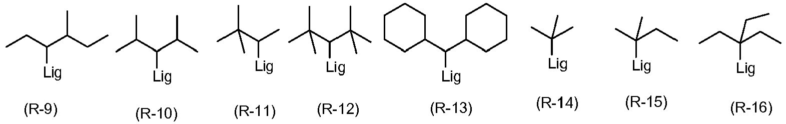 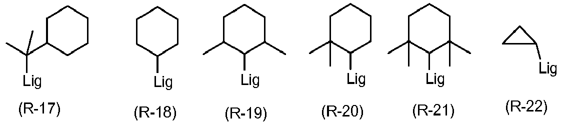 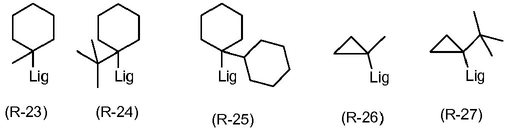 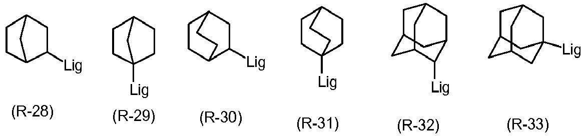 wobei Lig die Anknüpfung der Alkylgruppe an den Liganden kennzeichnet.

Wenn der Rest R, der einem Stickstoffatom benachbart ist, für eine Alkoxygruppe steht, dann weist diese Alkoxygruppe bevorzugt 3 bis 10 C-Atome auf. Bevorzugt ist diese Alkoxygruppe ausgewählt aus den Strukturen der folgenden Formeln (R-34) bis (R-47), wobei jeweils auch die Anknüpfung dieser Gruppen an den Liganden mit eingezeichnet ist: 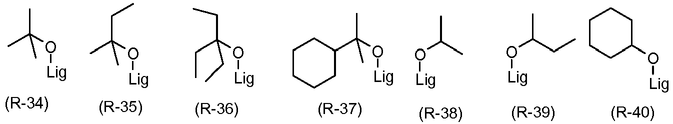 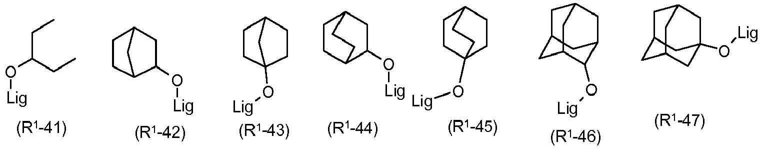 wobei Lig die Anknüpfung der Alkoxygruppe an den Liganden kennzeichnet.

Wenn der Rest R, der einem Stickstoffatom benachbart ist, für ein aromatisches bzw. heteroaromatisches Ringsystem steht, dann weist dieses aromatische bzw. heteroaromatische Ringsystem bevorzugt 5 bis 30 aromatische Ringatome auf, besonders bevorzugt 5 bis 24 aromatische Ringatome. Weiterhin enthält dieses aromatische bzw. heteroaromatische Ringsystem bevorzugt keine Aryl- bzw. Heteroarylgruppen, in denen mehr als zwei aromatische Sechsringe direkt aneinander kondensiert sind. Besonders bevorzugt enthält das aromatische bzw. heteroaromatische Ringsystem überhaupt keine kondensierten Aryl- bzw. Heteroarylgruppen, und ganz besonders bevorzugt enthält es nur Phenylgruppen. Dabei ist das aromatische Ringsystem bevorzugt ausgewählt aus den Strukturen der folgenden Formeln (R-70) bis (R-84), wobei jeweils auch die Anknüpfung dieser Gruppen an den Liganden eingezeichnet ist: 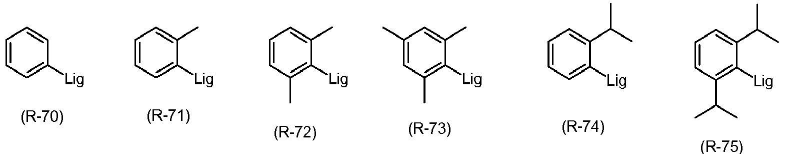 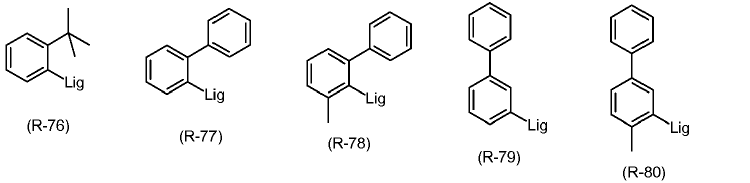 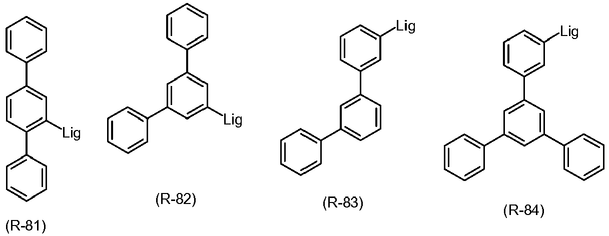 wobei Lig die Anknüpfung des aromatischen Ringsystems an den Liganden kennzeichnet und die Phenylgruppen jeweils durch einen oder mehrere Reste R¹ substituiert sein können.

Weiterhin ist das heteroaromatische Ringsystem bevorzugt ausgewählt aus den Strukturen der folgenden Formeln (R-85) bis (R-123), wobei jeweils auch die Anknüpfung dieser Gruppen an den Liganden eingezeichnet ist: 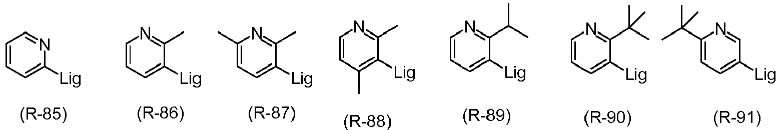 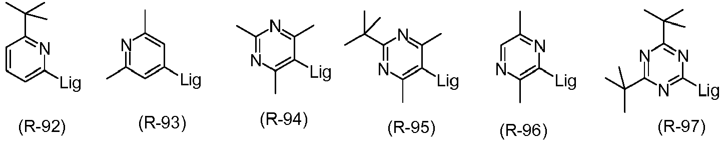 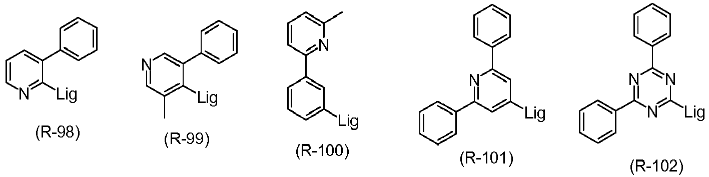 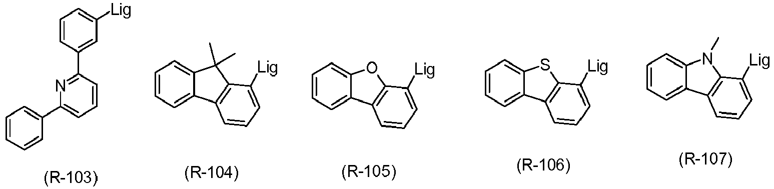 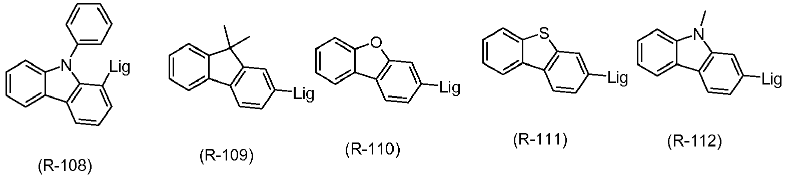 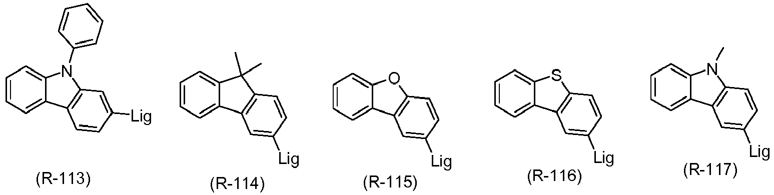 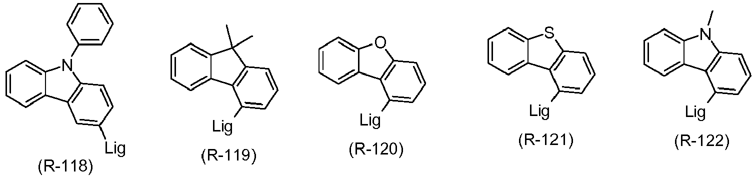 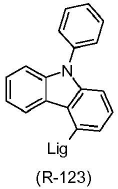 wobei Lig die Anknüpfung des heteroaromatischen Ringsystems an den Liganden kennzeichnet und die aromatischen und heteroaromatischen Gruppen jeweils durch einen oder mehrere Reste R¹ substituiert sein können.

Das charakterisierende Merkmal der vorliegenden Erfindung ist, wie oben beschrieben, dass in der Teilstruktur der Formel (2) zwei benachbarte Gruppen Y und/oder, falls vorhanden, zwei benachbarte Gruppen X für CR stehen und die jeweiligen Reste R zusammen mit den C-Atomen einen Ring der Formel (4) oder Formel (5) aufspannen.

Die Gruppen der Formel (4) bzw. (5) können in jeder Position der Teilstruktur der Formel (2) vorliegen, in der zwei Gruppen Y bzw., falls vorhanden, zwei Gruppen X direkt aneinander gebunden sind. Bevorzugte Positionen, in denen eine Gruppe der Formel (4) bzw. (5) vorliegt, sind die Teilstrukturen der folgenden Formeln (6a) bis (15h), 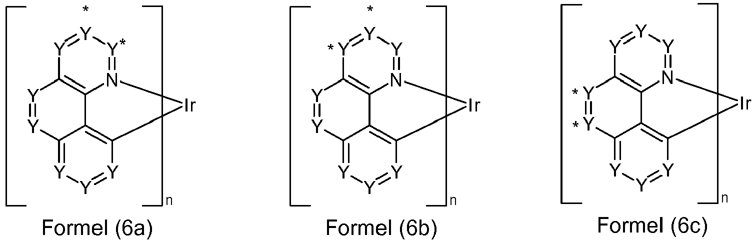 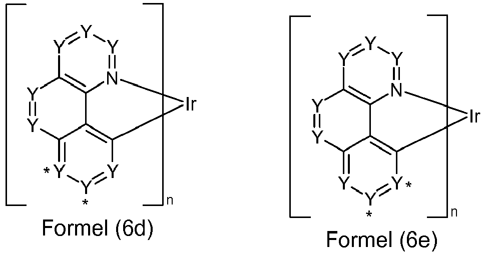 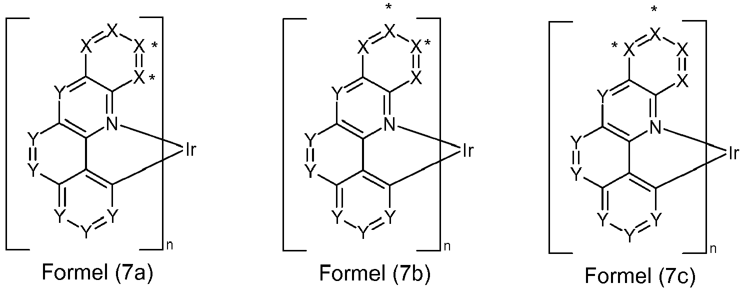 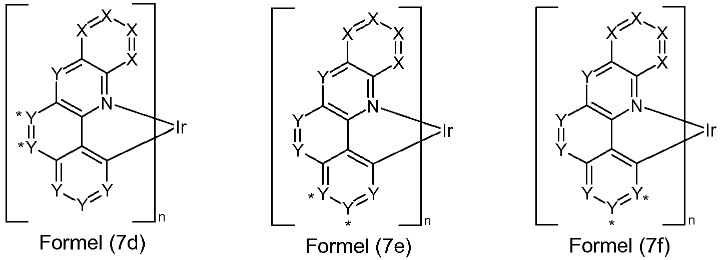 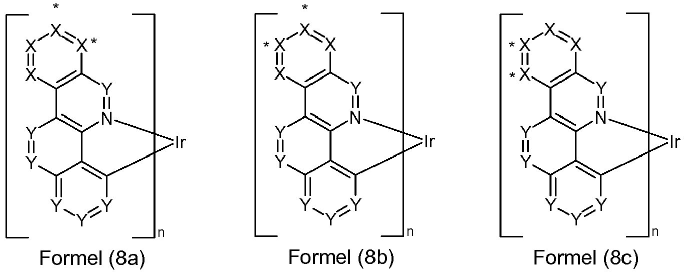 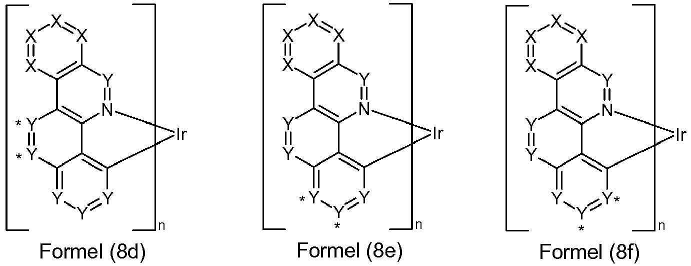 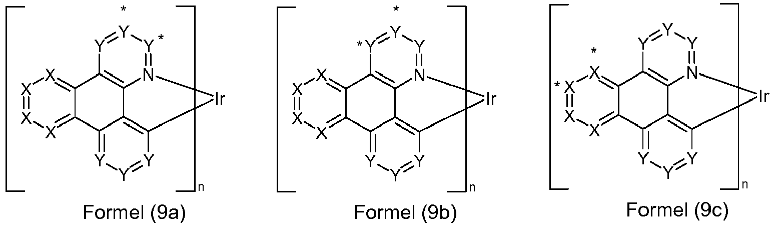 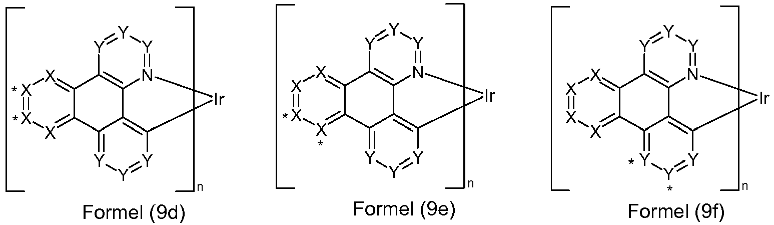 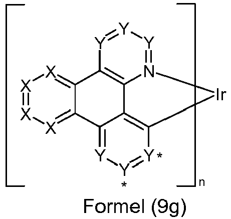 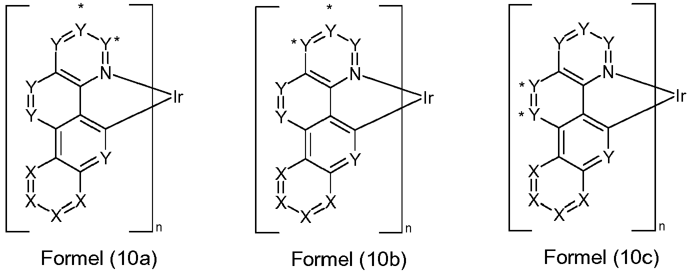 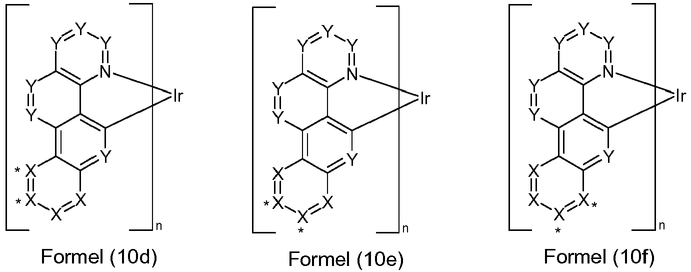 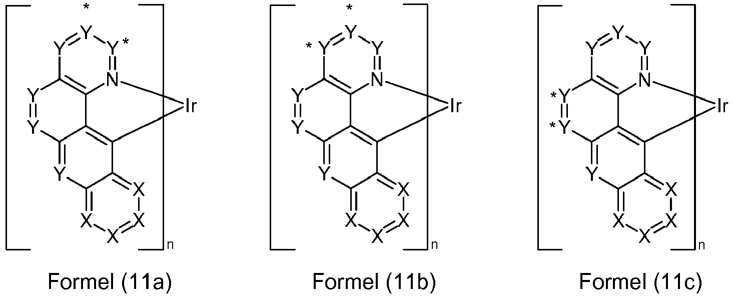 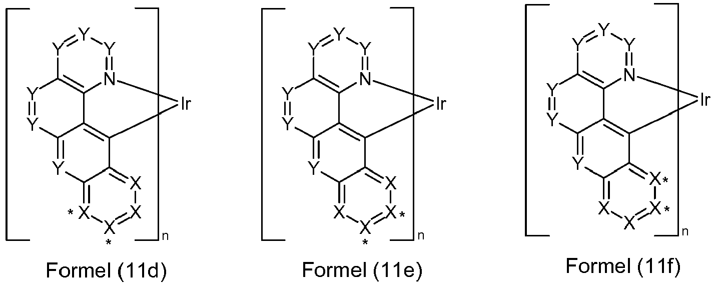 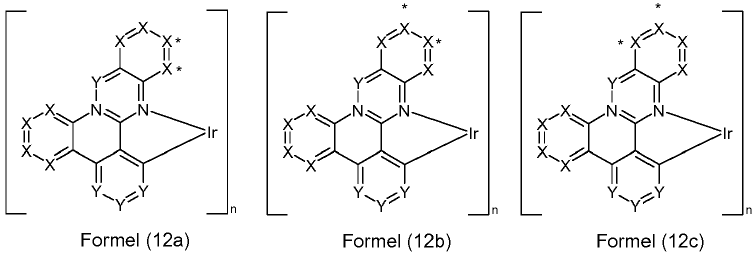 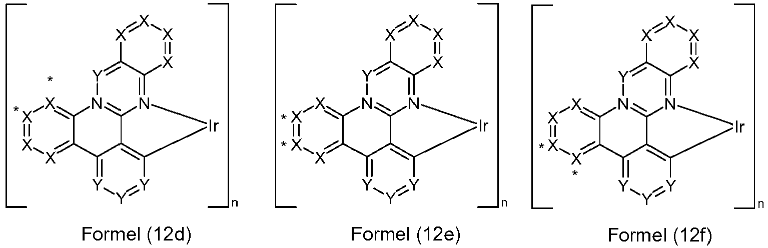 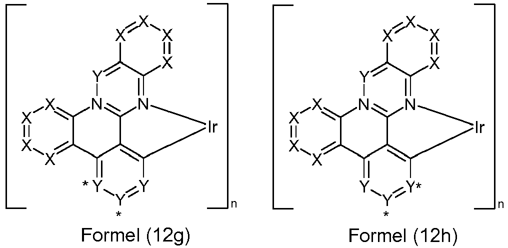 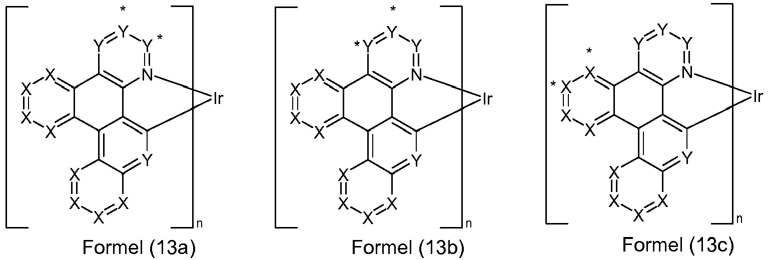 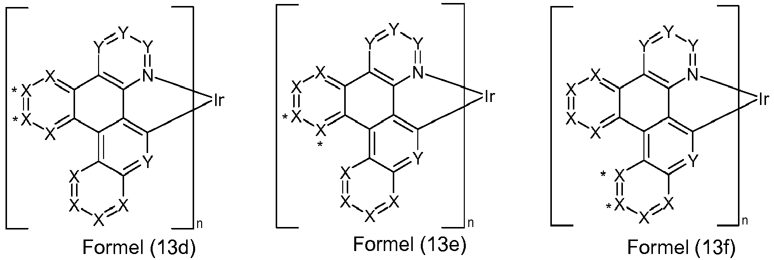 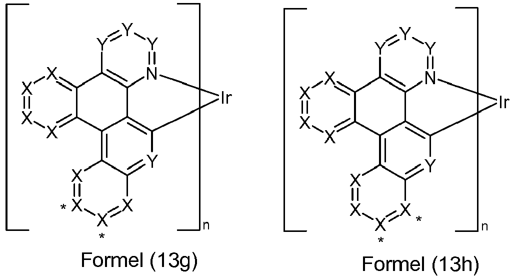 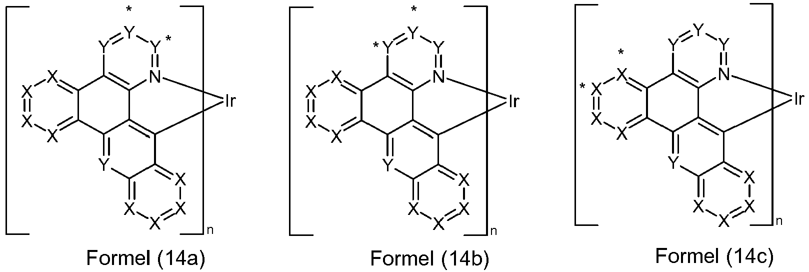 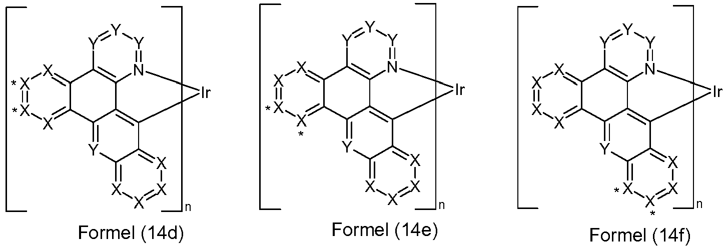 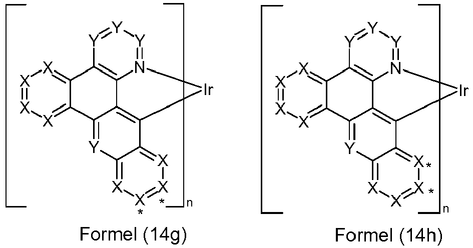 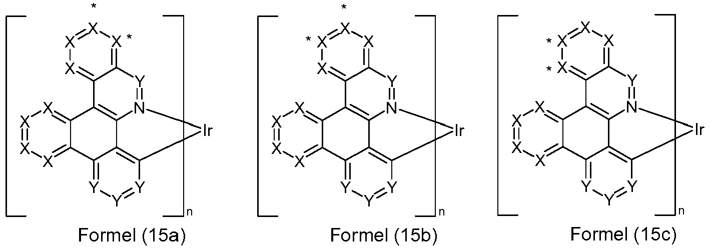 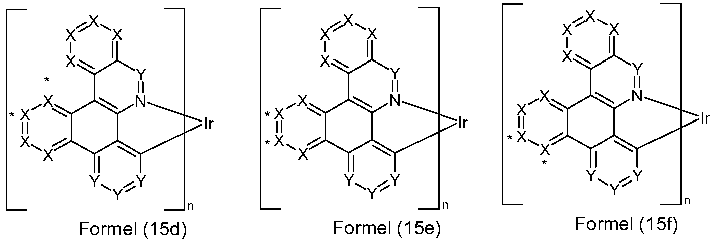 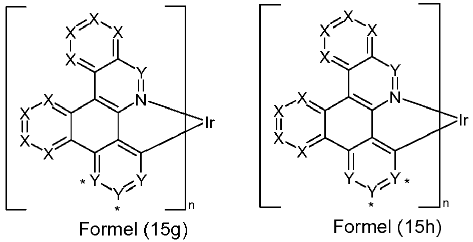 wobei die verwendeten Symbole und Indizes die oben genannten Bedeutungen aufweisen und * jeweils die Position anzeigt, an der die beiden benachbarten Gruppen Y bzw. X für CR stehen und die jeweiligen Reste R zusammen mit den C-Atomen einen Ring der Formel (4) oder Formel (5) aufspannen.

In den oben abgebildeten Strukturen der Formeln (4) und (5) sowie den weiteren als bevorzugt genannten Ausführungsformen dieser Strukturen wird formal eine Doppelbindung zwischen den zwei Kohlenstoffatomen abgebildet. Dies stellt eine Vereinfachung der chemischen Struktur dar, da diese beiden Kohlenstoffatome in ein aromatisches oder heteroaromatisches System eingebunden sind und somit die Bindung zwischen diesen beiden Kohlenstoffatomen formal zwischen dem Bindungsgrad einer Einfachbindung und dem einer Doppelbindung liegt. Das Einzeichnen der formalen Doppelbindung ist somit nicht limitierend für die Struktur auszulegen, sondern es ist für den Fachmann offensichtlich, dass es sich hier um eine aromatische Bindung handelt.

Wesentlich bei den Gruppen der Formeln (4) und (5) ist, dass diese keine aziden benzylischen Protonen aufweisen. Unter benzylischen Protonen werden Protonen verstanden, die an ein Kohlenstoffatom binden, welches direkt an den Liganden gebunden sind. Die Abwesenheit von aziden benzylischen Protonen wird in Formel (4) dadurch erreicht, dass A¹ und A³, wenn diese für C(R³)₂ stehen, so definiert sind, dass R³ ungleich Wasserstoff ist. Die Abwesenheit von aziden benzylischen Protonen ist in Formel (5) dadurch erreicht, dass es sich dabei um eine bicyclische Struktur handelt. Aufgrund der starren räumlichen Anordnung ist R¹, wenn es für H steht, deutlich weniger azide als benzylische Protonen, da das korrespondierende Anion der bicyclischen Struktur nicht mesomeriestabilisiert ist. Auch wenn R¹ in Formel (5) für H steht, handelt es sich dabei daher um ein nicht-azides Proton im Sinne der vorliegenden Anmeldung.

In einer bevorzugten Ausführungsform der Struktur gemäß Formel (4) steht maximal eine der Gruppen A¹ und A³ für für O, und die anderen beiden Gruppen stehen für C(R³)₂ bzw. C(R¹)₂, oder A¹ und A³ stehen gleich oder verschieden bei jedem Auftreten für O . In einer besonders bevorzugten Ausführungsform der Erfindung stehen A¹ und A³ gleich oder verschieden bei jedem Auftreten für C(R³)₂ und A² steht für C(R¹)₂ und besonders bevorzugt für C(R³)₂. Bevorzugte Ausführungsformen der Formel (4) sind somit die Strukturen der Formel (4-A), (4-B) und (4-D), und eine besonders bevorzugte Ausführungsform der Formel (4-A) ist die Struktur der Formel (4-E), 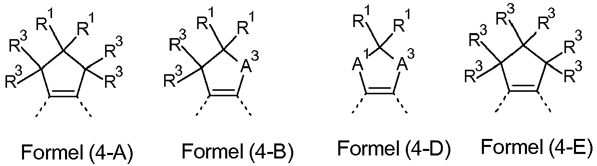 wobei R¹ und R³ die oben genannten Bedeutungen aufweisen und A¹ und A³ für O stehen.

In einer bevorzugten Ausführungsform der Struktur gemäß Formel (5) stehen die Reste R¹, die an den Brückenkopf gebunden sind, für H, D, F oder CH₃. Weiterhin bevorzugt steht A² für C(R³)₂. Bevorzugte Ausführungsformen der Formel (5) sind somit eine Strukturen der Formel (5-A), und eine besonders bevorzugte Ausführungsform der Formel (5-A) ist eine Struktur der Formel (5-C), 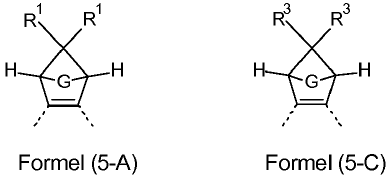 wobei die verwendeten Symbole die oben genannten Bedeutungen aufweisen.

In einer weiteren bevorzugten Ausführungsform der Erfindung steht R³ in den Gruppen der Formel (4) und (5) und in den bevorzugten Ausführungsformen gleich oder verschieden bei jedem Auftreten für eine geradkettige Alkylgruppe mit 1 bis 10 C-Atomen oder eine verzweigte oder cyclische Alkylgruppe mit 3 bis 10 C-Atomen oder ein aromatisches Ringsystem mit 5 bis 14 aromatischen Ringatomen; dabei können zwei Reste R³, welche an dasselbe Kohlenstoffatom gebunden sind, miteinander ein aliphatisches Ringsystem bilden und so ein Spirosystem aufspannen.

In einer besonders bevorzugten Ausführungsform der Erfindung steht R³ in den Gruppen der Formeln (4) und (5) und in den bevorzugten Ausführungsformen gleich oder verschieden bei jedem Auftreten für eine geradkettige Alkylgruppe mit 1 bis 3 C-Atomen, insbesondere Methyl, oder ein aromatisches Ringsystem mit 5 bis 12 aromatischen Ringatomen; dabei können zwei Reste R³, welche an dasselbe Kohlenstoffatom gebunden sind, miteinander ein aliphatisches Ringsystem bilden und so ein Spirosystem aufspannen.

Beispiele für besonders geeignete Gruppen der Formel (4) sind die im Folgenden aufgeführten Gruppen:

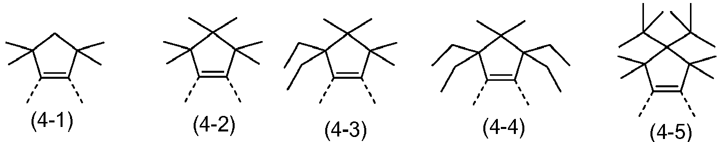

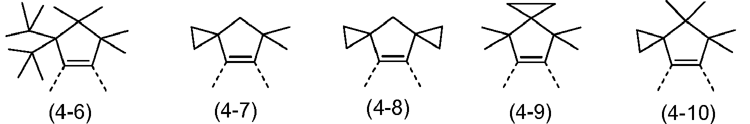

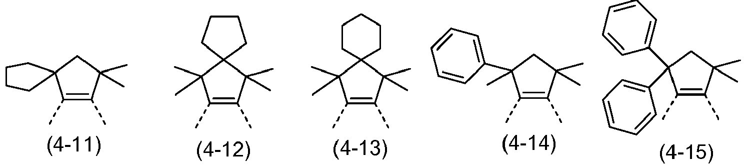

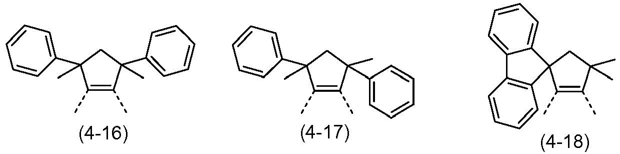

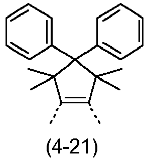

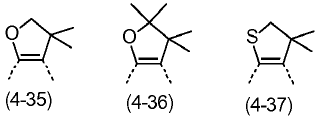

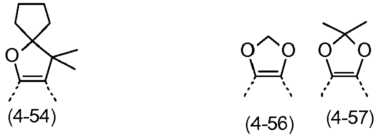

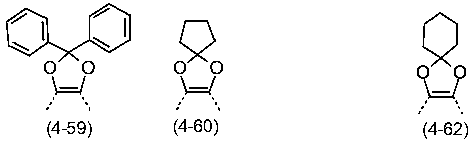

Beispiele für besonders geeignete Gruppen der Formel (5) sind die im Folgenden aufgeführten Gruppen:

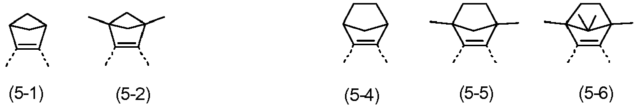

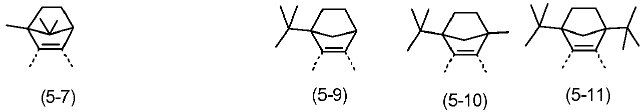

Wenn in der Teilstruktur der Formel (2) noch weitere bzw. andere Reste R gebunden sind, so sind diese Reste R bei jedem Auftreten gleich oder verschieden bevorzugt ausgewählt aus der Gruppe bestehend aus H, D, CN, einer geradkettigen Alkylgruppe mit 1 bis 10 C-Atomen oder einer verzweigten oder cyclischen Alkylgruppe mit 3 bis 10 C-Atomen oder einem aromatischen oder heteroaromatischen Ringsystem mit 5 bis 24 aromatischen Ringatomen, das jeweils durch einen oder mehrere Reste R¹ substituiert sein kann; dabei können zwei benachbarte Reste R auch miteinander ein mono- oder polycyclisches, aliphatisches Ringsystem bilden. Besonders bevorzugt sind diese Reste R bei jedem Auftreten gleich oder verschieden ausgewählt aus der Gruppe bestehend aus H, D, einer geradkettigen Alkylgruppe mit 1 bis 6 C-Atomen oder einer verzweigten oder cyclischen Alkylgruppe mit 3 bis 10 C-Atomen oder einem aromatischen oder heteroaromatischen Ringsystem mit 5 bis 18 aromatischen Ringatomen, das jeweils durch einen oder mehrere Reste R¹ substituiert sein kann; dabei können zwei benachbarte Reste R auch miteinander ein mono- oder polycyclisches, aliphatisches Ringsystem bilden. Wenn es sich um ein aromatisches oder heteroaromatisches Ringsystem handelt, ist es bevorzugt, wenn dieses nicht mehr als zwei direkt aneinander ankondensierte aromatische 6-Ringe aufweist, insbesondere überhaupt keine direkt aneinander ankondensierten aromatischen 6-Ringe.

Im Folgenden werden bevorzugte Liganden L' beschrieben, wie sie Verbindungen der Formel (1) vorkommen können. Die Liganden L' sind definitionsgemäß mono- oder bidentate Liganden. Die Liganden L' sind bevorzugt neutrale, monoanionische, dianionische oder trianionische Liganden, besonders bevorzugt neutrale oder monoanionische Liganden. Bevorzugt sind bidentate Liganden L'.

Bevorzugte neutrale, monodentate Liganden L' sind ausgewählt aus Kohlenmonoxid, Stickstoffmonoxid, Alkylcyaniden, wie z. B. Acetonitril, Arylcyaniden, wie z. B. Benzonitril, Alkylisocyaniden, wie z. B. Methylisonitril, Arylisocyaniden, wie z. B. Benzoisonitril, Aminen, wie z. B. Trimethylamin, Triethylamin, Morpholin, Phosphinen, insbesondere Halogenphosphine, Trialkylphosphine, Triarylphosphine oder Alkylarylphosphine, wie z. B. Trifluorphosphin, Trimethylphosphin, Tricyclohexylphosphin, Tri-*tert*-butylphosphin, Triphenylphosphin, Tris(pentafluorphenyl)phosphin, Phosphiten, wie z. B. Trimethylphosphit, Triethylphosphit, Arsinen, wie z. B. Trifluorarsin, Trimethylarsin, Tricyclohexylarsin, Tri-*tert-*butylarsin, Triphenylarsin, Tris(pentafluorphenyl)arsin, Stibinen, wie z. B. Trifluorstibin, Trimethylstibin, Tricyclohexylstibin, Tri-*tert*-butylstibin, Triphenylstibin, Tris(pentafluorphenyl)stibin, stickstoffhaltigen Heterocyclen, wie z. B. Pyridin, Pyridazin, Pyrazin, Pyrimidin, Triazin, und Carbenen, insbesondere Arduengo-Carbenen.

Bevorzugte monoanionische, monodentate Liganden L' sind ausgewählt aus Hydrid, Deuterid, den Halogeniden F⁻, Cl⁻, Br⁻ und I⁻, Alkylacetyliden, wie z. B. Methyl-C≡C⁻, tert-Butyl-C≡C⁻, Arylacetyliden, wie z. B. Phenyl-C≡C⁻, Cyanid, Cyanat, Isocyanat, Thiocyanat, Isothiocyanat, aliphatischen oder aromatischen Alkoholaten, wie z. B. Methanolat, Ethanolat, Propanolat, *iso*-Propanolat, *tert*-Butylat, Phenolat, aliphatischen oder aromatischen Thioalkoholaten, wie z. B. Methanthiolat, Ethanthiolat, Propanthiolat, *iso*-Propanthiolat, *tert*-Thiobutylat, Thiophenolat, Amiden, wie z. B. Dimethylamid, Diethylamid, Di-*iso*-propylamid, Morpholid, Carboxylaten, wie z. B. Acetat, Trifluoracetat, Propionat, Benzoat, Arylgruppen, wie z. B. Phenyl, Naphthyl, und anionischen, stickstoffhaltigen Heterocyclen, wie Pyrrolid, Imidazolid, Pyrazolid. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte di- bzw. trianionische Liganden sind O²⁻, S²⁻, Carbide, welche zu einer Koordination der Form R-C≡M führen, und Nitrene, welche zu einer Koordination der Form R-N=M führen, wobei R allgemein für einen Substituenten steht, und N³⁻.

Bevorzugte neutrale oder mono- oder dianionische, bidentate oder höherdentate Liganden L' sind ausgewählt aus Diaminen, wie z. B. Ethylendiamin, N,N,N',N'-Tetramethylethylendiamin, Propylendiamin, N,N,N',N'-Tetramethylpropylendiamin, cis- oder trans-Diaminocyclohexan, cis- oder trans-N,N,N',N'-Tetramethyldiaminocyclohexan, Iminen, wie z. B. 2-[1-(Phenylimino)ethyl]pyridin, 2-[1-(2-Methylphenylimino)ethyl]pyridin, 2-[1-(2,6-Di-*iso*-propylphenylimino)ethyl]pyridin, 2-[1-(Methylimino)ethyl]pyridin, 2-[1-(ethylimino)ethyl]pyridin, 2-[1-(*Iso*-Propylimino)ethyl]pyridin, 2-[1-(*Tert-*Butylimino)ethyl]pyridin, Diminen, wie z. B. 1,2-Bis(methylimino)ethan, 1,2-Bis(ethylimino)ethan, 1,2-Bis(*iso*-propylimino)ethan, 1,2-Bis(*tert*-butyl-imino)ethan, 2,3-Bis(methylimino)butan, 2,3-Bis(ethylimino)butan, 2,3-Bis-(*iso*-propylimino)butan, 2,3-Bis(*tert*-butylimino)butan, 1,2-Bis(phenylimino)-ethan, 1,2-Bis(2-methylphenylimino)ethan, 1,2-Bis(2,6-di-*iso*-propylphenyl-imino)ethan, 1,2-Bis(2,6-di-*tert*-butylphenylimino)ethan, 2,3-Bis(phenyl-imino)butan, 2,3-Bis(2-methylphenylimino)butan, 2,3-Bis(2,6-di-*iso*-propyl-phenylimino)butan, 2,3-Bis(2,6-di-*tert*-butylphenylimino)butan, Heterocyclen enthaltend zwei Stickstoffatome, wie z. B. 2,2'-Bipyridin, o-Phenanthrolin, Diphosphinen, wie z. B. Bis(diphenylphosphino)methan, Bis(diphenylphosphino)ethan, Bis(diphenylphosphino)propan, Bis(diphenylphosphino)butan, Bis(dimethylphosphino)methan, Bis(dimethylphosphino)-ethan, Bis(dimethylphosphino)propan, Bis(diethylphosphino)methan, Bis-(diethylphosphino)ethan, Bis(diethylphosphino)propan, Bis(di-*tert*-butylphosphino)methan, Bis(di-*tert*-butylphosphino)ethan, Bis(*tert*-butylphosphino)propan, 1,3-Diketonaten abgeleitet von 1,3-Diketonen, wie z. B. Acetylaceton, Benzoylaceton, 1,5-Diphenylacetylaceton, Dibenzoylmethan, Bis(1,1,1-trifluoracetyl)methan, 2,2,6,6-Tetramethyl-3,5-heptandion, 3-Ketonaten abgeleitet von 3-Ketoestern, wie z. B. Acetessigsäureethylester, Carboxylate, abgeleitet von Aminocarbonsäuren, wie z. B. Pyridin-2-carbonsäure, Chinolin-2-carbonsäure, Glycin, N,N-Dimethylglycin, Alanin, N,N-Dimethylaminoalanin, Salicyliminaten abgeleitet von Salicyliminen, wie z. B. Methylsalicylimin, Ethylsalicylimin, Phenylsalicylimin, Dialkoholaten abgeleitet von Dialkoholen, wie z. B. Ethylenglykol, 1,3-Propylenglykol und Dithiolaten abgeleitet von Dithiolen, wie z. B. 1,2-Ethylendithiol, 1,3-Propylendithiol.

In einer weiteren bevorzugten Ausführungsform der Erfindung handelt es sich bei den Liganden L' um bidentate monoanionische Liganden, welche mit dem Iridium einen cyclometallierten Fünfring oder Sechsring mit mindestens einer Iridium-Kohlenstoff-Bindung aufweisen, insbesondere einen cyclometallierten Fünfring. Dies sind insbesondere Liganden, wie sie allgemein im Gebiet der phosphoreszierenden Metallkomplexe für organische Elektrolumineszenzvorrichtungen verwendet werden, also Liganden vom Typ Phenylpyridin, Naphthylpyridin, Phenylchinolin, Phenylisochinolin, etc., welche jeweils durch einen oder mehrere Reste R substituiert sein können. Dem Fachmann auf dem Gebiet der phosphoreszierenden Elektrolumineszenzvorrichtungen ist eine Vielzahl derartiger Liganden bekannt, und er kann ohne erfinderisches Zutun weitere derartige Liganden als Ligand L' für Verbindungen gemäß Formel (1) auswählen. Generell eignet sich dafür besonders die Kombination aus zwei Gruppen, wie sie durch die folgenden Formeln (16) bis (43) dargestellt sind, wobei eine Gruppe über ein neutrales Atom und die andere Gruppe über ein negativ geladenes Atom bindet. Dabei ist das neutrale Atom insbesondere ein neutrales Stickstoffatom oder ein Carben-Kohlenstoffatom und das negativ geladene Atom insbesondere ein negativ geladenes Kohlenstoffatom, ein negativ geladenes Stickstoffatom oder ein negativ geladenes Sauerstoffatom. Der Ligand L' kann dann aus den Gruppen der Formeln (16) bis (43) gebildet werden, indem diese Gruppen jeweils an der durch # gekennzeichneten Position aneinander binden. Die Position, an der die Gruppen an das Metall koordinieren, sind durch * gekennzeichnet. Weiterhin können hier auch zwei benachbarte Reste R, die jeweils an den beiden Gruppen der Formeln (16) bis (43) gebunden sind, miteinander ein aliphatisches oder aromatisches Ringsystem bilden.

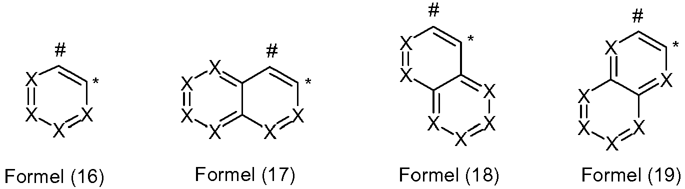

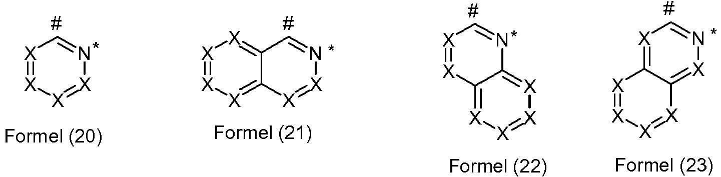

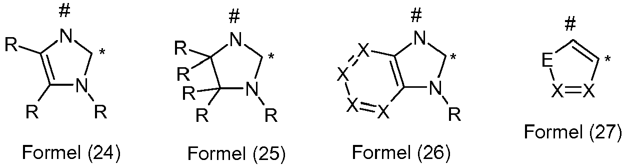

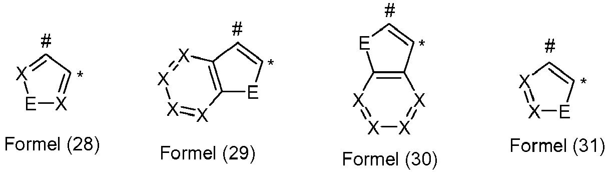

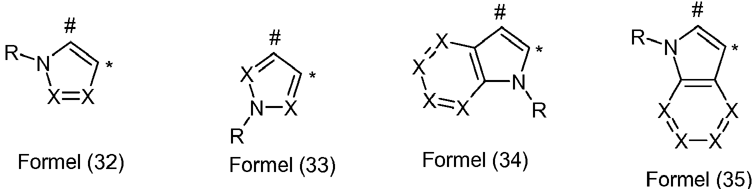

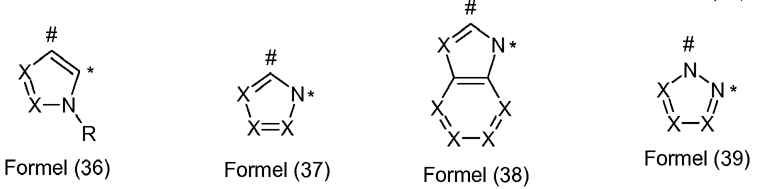

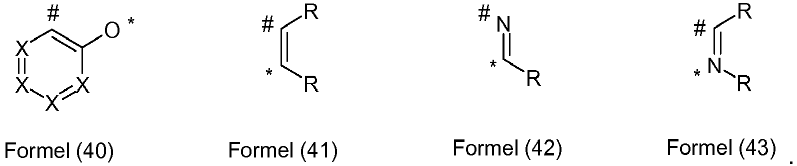

Dabei haben die verwendeten Symbole dieselbe Bedeutung wie oben beschrieben, E steht für O, S oder CR₂, und bevorzugt stehen maximal zwei Symbole X in jeder Gruppe für N, besonders bevorzugt steht maximal ein Symbol X in jeder Gruppe für N. Ganz besonders bevorzugt stehen alle Symbole X für CR.

In einer ganz besonders bevorzugten Ausführungsform der Erfindung handelt es sich bei dem Liganden L' um einen monoanionischen Liganden, der aus zwei der Gruppen der Formel (16) bis (43) gebildet wird, wobei eine dieser Gruppen über ein negativ geladenes Kohlenstoffatom und die andere dieser Gruppen über ein neutrales Stickstoffatom an das Iridium koordiniert.

Ebenfalls kann es bevorzugt sein, wenn zwei benachbarte Symbole X in diesen Liganden für eine Gruppe der oben genannten Formel (4) oder (5) stehen.

Die weiteren bevorzugten Reste R in den oben aufgeführten Strukturen sind definiert wie die Reste R des Liganden L.

Die Liganden L und L' können je nach Struktur auch chiral sein. Dies ist insbesondere dann der Fall, wenn sie eine bicyclische Gruppe der Formel (5) enthalten oder wenn sie Substituenten enthalten, beispielsweise Alkylgruppen, welche ein oder mehrere Stereozentren aufweisen. Da es sich bei der Grundstruktur des Komplexes auch um eine chirale Struktur handeln kann, ist die Bildung von Diastereomeren und mehreren Enantiomerenpaaren möglich. Die erfindungsgemäßen Komplexe umfassen dann sowohl die Mischungen der verschiedenen Diastereomere bzw. die entsprechenden Racemate wie auch die einzelnen isolierten Diastereomere bzw. Enantiomere.

Die erfindungsgemäßen Verbindungen können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, löslich gemacht werden. Solche Verbindungen sind dann in gängigen organischen Lösemitteln bei Raumtemperatur in ausreichender Konzentration löslich, um die Komplexe aus Lösung verarbeiten zu können, beispielsweise durch Druckverfahren.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Die erfindungsgemäßen Metallkomplexe sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen gemäß Formel (1) durch Umsetzung der entsprechenden freien Liganden mit Iridiumalkoholaten der Formel (44), mit Iridiumketoketonaten der Formel (45), mit Iridiumhalogeniden der Formel (46) oder mit dimeren Iridiumkomplexen der Formel (47) oder (48), 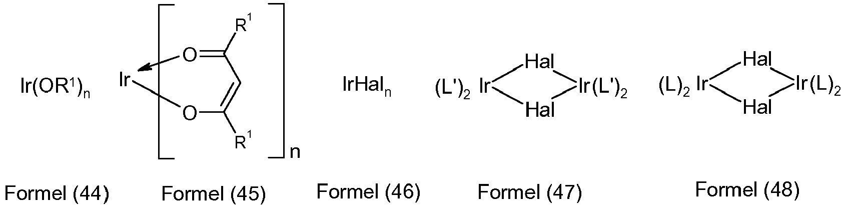 wobei die Symbole und Indizes L', m, n und R¹ die oben angegebenen Bedeutungen haben und Hal = F, Cl, Br oder I ist.

Die Synthese kann auch durch Umsetzung der Liganden L mit Iridiumkomplexen der Formel [Ir(L')₂(HOMe)₂]A oder [Ir(L')₂(NCMe)₂]A oder durch Umsetzung der Liganden L' mit Iridiumkomplexen der Formel [Ir(L)₂(HOMe)₂]A oder [Ir(L)₂(NCMe)₂]A, wobei A jeweils ein nicht koordinierendes Anion, wie z. B. Triflat, Tetrafluoroborat, Hexafluorophosphat, etc. darstellt, in dipolar-protischen Lösungsmitteln, wie z. B. Ethylenglykol, Propylenglykol, Glycerin, Diethylenglykol, Triethylenglykol, etc., durchgeführt werden.

Es können ebenfalls Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet ist [IrCl₂(acac)₂]⁻, beispielsweise Na[IrCl₂(acac)₂]. Weitere besonders geeignete Iridiumedukte sind Iridium(III)-tris(acetylacetonat) und Iridium(III)-tris(2,2,6,6-tetramethyl-3,5-heptand ionat).

Die Synthese der Komplexe wird bevorzugt durchgeführt wie in WO 2002/060910 und in WO 2004/085449 beschrieben. Heteroleptische Komplexe können beispielsweise auch gemäß WO 05/042548 synthetisiert werden. Dabei kann die Synthese beispielsweise auch thermisch, photochemisch und/oder durch Mikrowellenstrahlung aktiviert werden. Weiterhin kann die Synthese auch im Autoklaven bei erhöhtem Druck und/oder erhöhter Temperatur durchgeführt werden.

Die Reaktionen können prinzipiell auch ohne Zusatz von Lösemitteln oder Schmelzhilfen in einer Schmelze der entsprechenden zu o-metallierenden Liganden durchgeführt werden. Gegebenenfalls können Lösemittel oder Schmelzhilfen zugesetzt werden. Geeignete Lösemittel sind protische oder aprotische Lösemittel, wie aliphatische und/oder aromatische Alkohole, wie z. B. Methanol, Ethanol, iso-Propanol, t-Butanol, etc., Oligo- und Polyalkohole, wie z. B. Ethylenglykol, 1,2-Propandiol oder Glycerin, Alkoholether, wie z. B. Ethoxyethanol, Diethylenglykol, Triethylenglycol, Polyethylenglykol, etc., Ether, wie z. B. Di- und Tri-ethylenglykoldimethylether, Diphenylether, etc., aromatische, heteroaromatische und/oder aliphatische Kohlenwasserstoffe, wie z. B. Toluol, Xylol, Mesitylen, Chlorbenzol, Pyridin, Lutidin, Chinolin, iso-Chinolin, Tridecan, Hexadecan, etc., Amide, wie z. B. DMF, DMAC, etc., Lactame, wie z. B. NMP, Sulfoxide, wie z. B. DMSO, Sulfone, wie z. B. Dimethylsulfon, Sulfolan, etc.. Geeignete Schmelzhilfen sind Verbindungen, die bei Raumtemperatur fest vorliegen, jedoch beim Erwäremen der Reaktionsmischung schmelzen und die Reaktanden lösen, so dass eine homogene Schmelze entsteht. Besonders geeignet sind Biphenyl, m-Terphenyl, Triphenylen, 1,2-, 1,3-, 1,4-Bisphenoxybenzol, Triphenylphosphinoxid, 18-Krone-6, Phenol, 1-Naphthol, Hydrochinon, etc..

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Die oben beschriebenen Komplexe gemäß Formel (1) bzw. die oben aufgeführten bevorzugten Ausführungsformen können in einer organischen Elektrolumineszenzvorrichtung als aktive Komponente verwendet werden. Ein weiterer Gegenstand der vorliegenden Erfindung ist daher die Verwendung einer Verbindung nach Formel (1) bzw. nach einer der bevorzugten Ausführungsformen in einer organischen Elektrolumineszenzvorrichtung.

Nochmals ein weiterer Gegenstand der vorliegenden Erfindung ist eine organische Elektrolumineszenzvorrichtung enthaltend mindestens eine Verbindung gemäß Formel (1) bzw. nach einer der bevorzugten Ausführungsformen.

Unter einer organischen Elektrolumineszenzvorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße organische Elektrolumineszenzvorrichtung enthält also Anode, Kathode und mindestens eine Schicht, welche mindestens eine Verbindung der oben aufgeführten Formel (1) enthält. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Eine bevorzugte Ausführungsform sind Dreischichtsysteme, wobei die drei Schichten blaue, grüne und orange oder rote Emission zeigen (siehe z. B. WO 2005/011013) bzw. Systeme, welche mehr als drei emittierende Schichten aufweisen. Eine weitere bevorzugte Ausführungsform sind Zweischichtsysteme, wobei die beiden Schichten entweder blaue und gelbe oder blaugrüne und orange Emission zeigen. Zweischichtsysteme sind insbesondere für Beleuchtungsanwendungen von Interesse. Solche Ausführungsformen sind mit den erfindungsgemäßen Verbindungen besonders geeignet, da diese häufig gelbe bzw. orange Emission zeigen. Die weiß emittierenden Elektrolumineszenzvorrichtungen können für Beleuchtungsanwendungen oder als Backlight für Displays oder mit Farbfilter als Display eingesetzt werden.

In einer bevorzugten Ausführungsform der Erfindung enthält die organische Elektrolumineszenzvorrichtung die Verbindung gemäß Formel (1) bzw. die oben aufgeführten bevorzugten Ausführungsformen als emittierende Verbindung in einer oder mehreren emittierenden Schichten.

Wenn die Verbindung gemäß Formel (1) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (1) und dem Matrixmaterial enthält zwischen 1 und 99 Vol.-%, vorzugsweise zwischen 2 und 90 Vol.-%, besonders bevorzugt zwischen 3 und 40 Vol.-%, insbesondere zwischen 5 und 15 Vol.-% der Verbindung gemäß Formel (1) bezogen auf die Gesamtmischung aus Emitter und Matrixmaterial. Entsprechend enthält die Mischung zwischen 99 und 1 Vol.-%, vorzugsweise zwischen 98 und 10 Vol.-%, besonders bevorzugt zwischen 97 und 60 Vol.-%, insbesondere zwischen 95 und 85 Vol.-% des Matrixmaterials bzw. der Matrixmaterialien bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen. Hierfür eignen sich insbesondere Mischungen aus mindestens einem elektronentransportierenden Matrixmaterial und mindestens einem lochtransportierenden Matrixmaterial oder Mischungen aus mindestens zwei elektronentransportierenden Matrixmaterialien oder Mischungen aus mindestens einem loch- oder elektronentransportierenden Matrixmaterial und mindestens einem weiteren Material mit einer großen Bandlücke, welches somit weitgehend elektrisch inert ist und nicht oder nicht in wesentlichem Umfang am Ladungstransport teilnimmt, wie z. B. in WO 2010/108579 beschrieben. Eine bevorzugte Kombination ist beispielsweise die Verwendung eines aromatischen Ketons oder eines Triazinderivats mit einem Triarylamin-Derivat oder einem Carbazol-Derivat als gemischte Matrix für den erfindungsgemäßen Metallkomplex.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise blau oder grün emittierende Triplettemitter als Co-Matrix für die erfindungsgemäßen Komplexe gemäß Formel (1) eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

In den weiteren Schichten können generell alle Materialien verwendet werden, wie sie gemäß dem Stand der Technik für die Schichten verwendet werden und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch folgende überraschende Vorteile gegenüber dem Stand der Technik aus:
1. Organische Elektrolumineszenzvorrichtungen enthaltend Verbindungen gemäß Formel (1) als emittierende Materialien weisen eine sehr gute Lebensdauer auf. Insbesondere weisen sie eine bessere Lebensdauer auf als Elektrolumineszenzvorrichtungen, welche analoge Verbindungen enthalten, die keinen ankondensierten aliphatischen Fünfring der Formel (4) oder (5) enthalten.
2. Organische Elektrolumineszenzvorrichtungen enthaltend Verbindungen gemäß Formel (1) als emittierende Materialien weisen eine sehr gute Effizienz auf. Insbesondere weisen sie eine bessere Effizienz auf als Elektrolumineszenzvorrichtungen, welche analoge Verbindungen enthalten, die keinen ankondensierten aliphatischen Fünfring der Formel (4) oder (5) enthalten.
3. Organische Elektrolumineszenzvorrichtungen enthaltend Verbindungen gemäß Formel (1) als emittierende Materialien weisen eine sehr niedrige Betriebsspannung auf.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße Verbindungen synthetisieren und diese in elektronischen Vorrichtungen verwenden und kann somit die Erfindung im gesamten offenbarten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von VWR, Sigma-ALDRICH bzw. ABCR bezogen werden. Die Nummern bei den literaturbekannten Verbindungen, die teilweise auch in eckigen Klammern angegeben sind, sind die CAS-Nummern der Verbindungen.

### 2,3-substituierte Benzo[h]chinoline - Synthese-Vorschriften

### Allgemeine Vorschrift:

Ein aromatischer o-Aminocarbaldehyd (400 mmol) wird in 1.1 L trockenem 1,4-Dioxan bei 60 °C gelöst und die gesamte Apparatur sorgfältig mit Inertgas gespült. Ein (bi)-cyclisches α-Methylenketon (600 mmol, 1.5 Eq) und Kalium-*tert*-butylat (400 mmol, 1 Eq) werden zugegeben und die Reaktionsmischung auf 90 °C erhitzt, bis das Edukt vollständig abreagiert hat. Nach dem Abkühlen wird die Reaktion mit Wasser verdünnt und mit Ethylacetat extrahiert. Die organischen Phasen werden vereinigt, mit Wasser und gesättigter NaCl-Lsg gewaschen und im Vakuum vom Lösemittel befreit. Der Rückstand wird in 1 L Dichlormethan aufgenommen und über Kieselgel filtriert. Zur weiteren Abtrennung von Nebenkomponenten wird aus Methanol umkristallisiert und säulenchromatographisch über Kieselgel mit Ethylacetat/Heptan aufgereinigt.

### Beispiel Ligand 1 (L1)

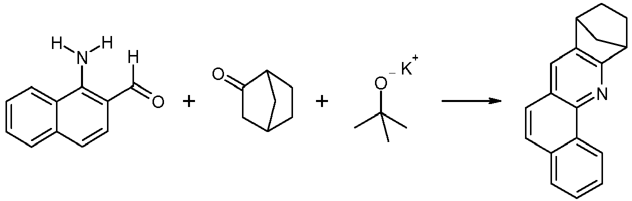

Es werden 1-Aminonaphthalin-2-carbaldehyd (407 mmol, 69.7 g), Norcampher (611 mmol, 67.3 g, 1.5 Eq) und als Base Kalium-*tert*-butylat (407 mmol, 45.7 g) nach der allgemeinen Vorschrift umgesetzt. Zur Abtrennung von Nebenkomponenten wird aus Methanol umkristallisiert und säulenchromatographisch über Kieselgel mit Ethylacetat/Heptan (1:5) aufgereinigt. Es werden 63.7 g (259 mmol, 64%) eines farblosen Feststoffs erhalten.

Als Edukte können die in den folgenden Tabellen angegebenen o-Aminocarbaldehyde und (bi)-cyclische α-Methylenketone nach der allgemeinen Vorschrift miteinander umgesetzt werden.

| **Bsp.** | ***o-A*mino-carb aldehyd** | **(bi)-cyclisches α- Methylenketon** | **Ligand** | **Aus beute** |
|---|---|---|---|---|
| L1 | 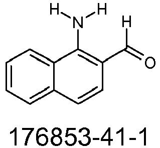 | 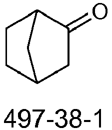 | 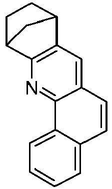 | 64% |
| L2 | 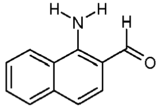 | 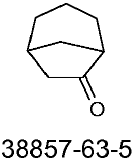 | 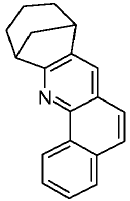 | 53% |
| L3 | 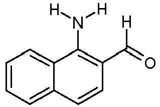 | 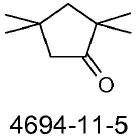 | 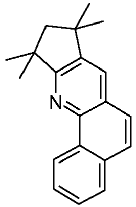 | 33% |
| L4 | 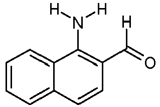 | 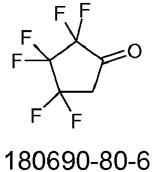 | 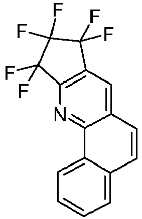 | 40% |
| L5 | 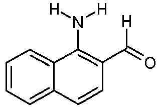 | 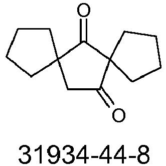 | 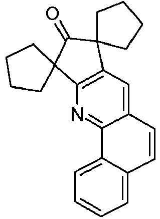 | 11% |
| L6 | 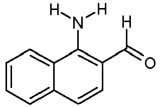 | 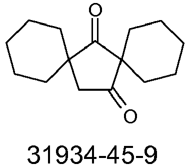 | 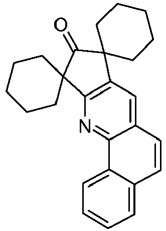 | 9% |
| L7 | 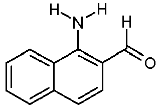 | 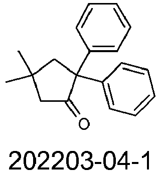 | 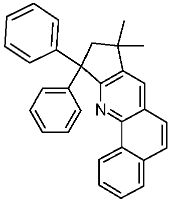 | 43% |
| L8 | 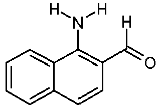 | 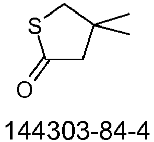 | 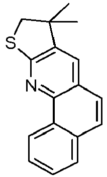 | 53% |
| L9 | 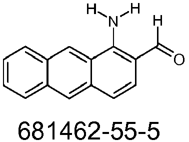 | 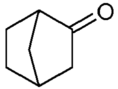 | 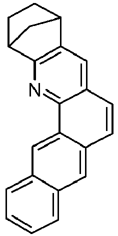 | 30% |
| L10 | 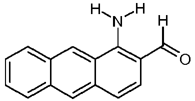 | 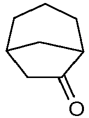 | 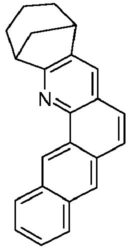 | 36% |
| L11 | 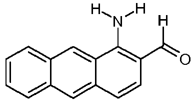 | 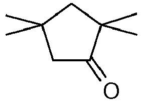 | 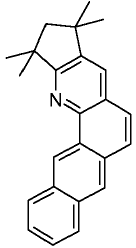 | 24% |
| L12 | 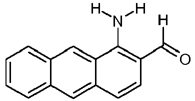 | 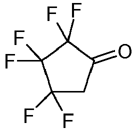 | 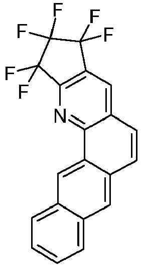 | 29% |
| L13 | 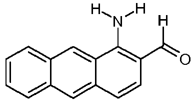 | 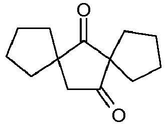 | 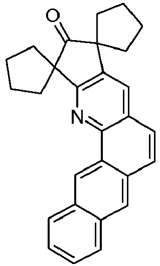 | 30% |
| L14 | 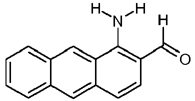 | 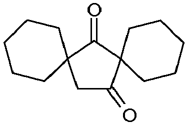 | 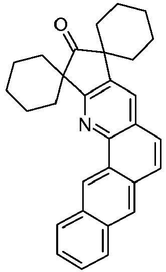 | 32% |
| L15 | 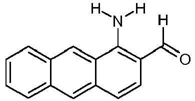 | 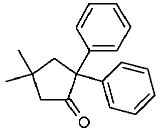 | 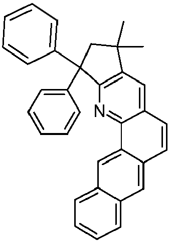 | 30% |
| L16 | 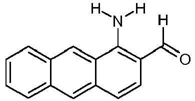 | 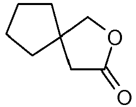 | 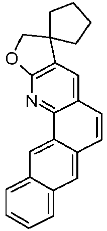 | 26% |
| L17 | 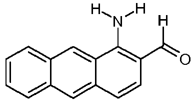 | 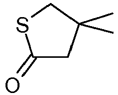 | 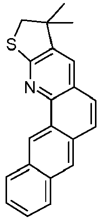 | 39% |
| L18 | 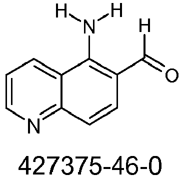 | 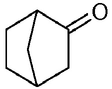 | 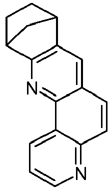 | 36% |
| L19 | 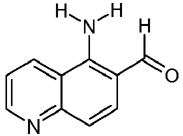 | 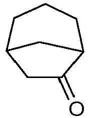 | 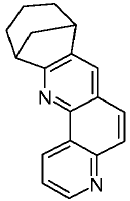 | 23% |
| L20 | 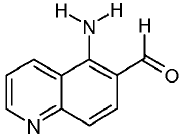 | 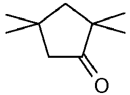 | 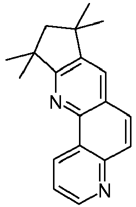 | 38% |
| L21 | 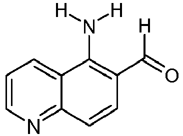 | 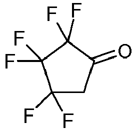 | 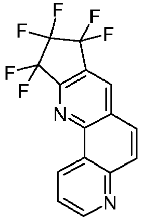 | 31% |
| L22 | 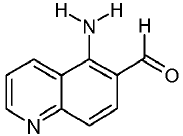 | 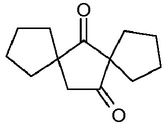 | 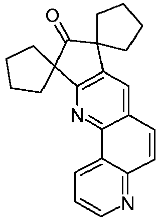 | 22% |
| L23 | 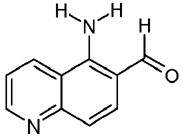 | 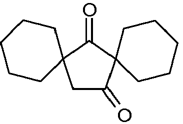 | 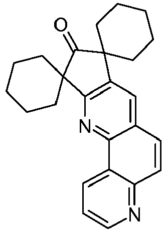 | 24% |
| L24 | 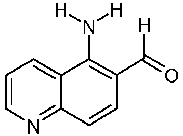 | 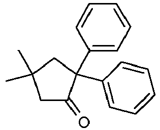 | 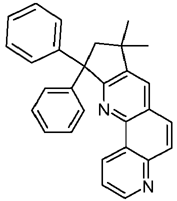 | 27% |
| L25 | 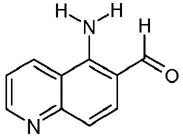 | 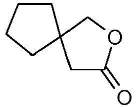 | 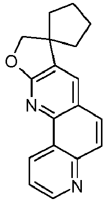 | 32% |
| L26 | 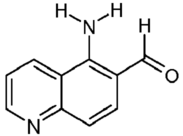 | 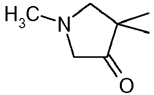 | 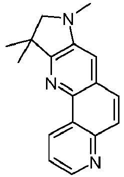 | 37% |
| L27 | 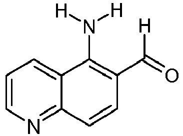 | 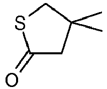 | 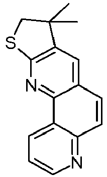 | 29% |
| L28 | 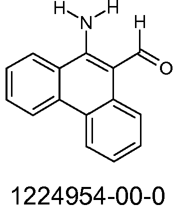 | 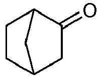 | 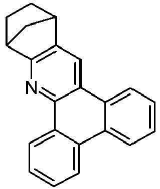 | 58% |
| L29 | 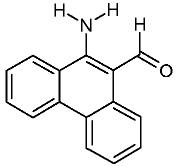 | 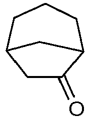 | 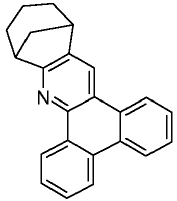 | 48% |
| L30 | 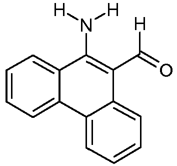 | 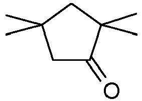 | 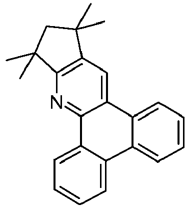 | 37% |
| L31 | 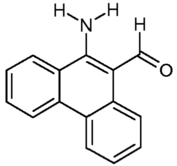 | 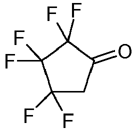 | 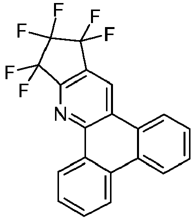 | 32% |
| L32 | 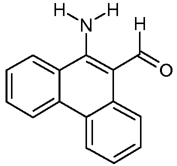 | 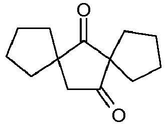 | 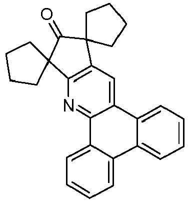 | 34% |
| L33 | 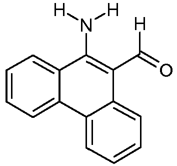 | 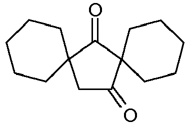 | 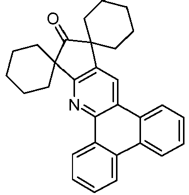 | 35% |
| L34 | 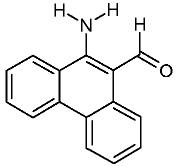 | 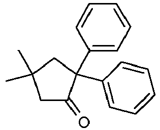 | 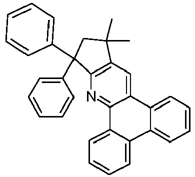 | 36% |
| L35 | 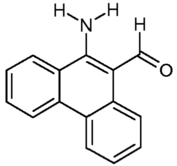 | 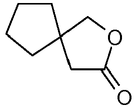 | 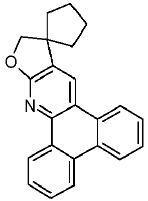 | 31% |
| L36 | 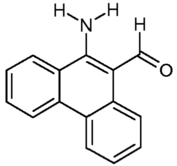 | 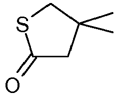 | 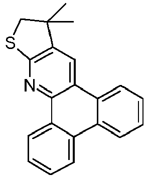 | 71% |

### 3,4-substituierte Benzo[h]chinoline

### Reaktionsschema:

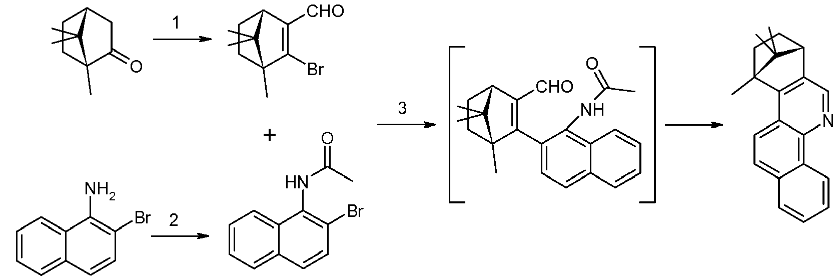

### Synthese-Vorschriften

### Allgemeine Vorschrift für Schritt 1: Umsetzung eines α-Methylenketons zu einem Bromalkencarbaldehyd

Eine Mischung aus DMF (25 ml) und Chloroform (80 ml) wird auf 0 °C abgekühlt und inertisiert. Es werden langsam 5.8 ml (60 mmol) PBr₃ in 10 ml Chloroform getropft und die Reaktionsmischung 2 h bei Raumtemperatur gerührt. Man erhitzt die Suspension auf Rückfluss und tropft das Keton (50 mmol) in 15 ml Chloroform langsam zu. Die Reaktion wird weitere 12 h unter Rückfluss gekocht, abgekühlt und langsam in eine 1M NaOH-Lösung gegeben. Man extrahiert mehrfach mit Diethylether, trocknet über Natriumsulfat und entfernt das Lösemittel im Vakuum.

Beispiel für S2:

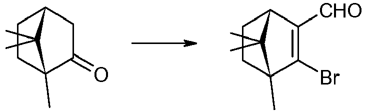

Eine Mischung aus DMF (25 ml) und Chloroform (80 ml) wird auf 0 °C abgekühlt und inertisiert. Es werden langsam 5.8 ml (60 mmol) PBr₃ in 10 ml Chloroform getropft und die Reaktionsmischung 2 h bei Raumtemperatur gerührt. Man erhitzt die Suspension auf Rückfluss und tropft den (+)-Campher (50 mmol, 7.6 g) in 15ml Chloroform langsam dazu. Die Mischung wird weitere 12 h unter Rückfluss gekocht, abgekühlt und langsam in eine 1M NaOH-Lösung gegeben. Man extrahiert mehrfach mit Diethylether, trocknet über Natriumsulfat und entfernt das Lösemittel im Vakuum. Das erhaltene Öl wird über eine Vakuumdestillation weiter aufgereinigt. Man erhält ein hellgelbes Öl in einer Ausbeute von 24 % (2.92 g, 12 mmol).

Folgende (bi)-cyclische α-Methylenketone können nach der allgemeinen Vorschrift in der angegebenen Ausbeute zu Bromalkencarbaldehyden umgesetzt werden:

| **Bsp.** | **Methylenketon** | **Bromalkencarbaldehyd** | **Ausbeute** |
|---|---|---|---|
| S1 | 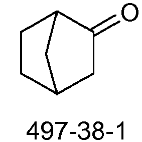 | 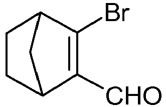 | 27% |
| S2 | 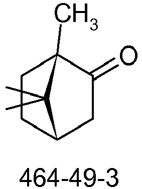 | 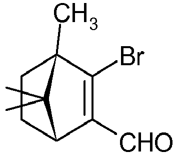 | 24% |
| S3 | 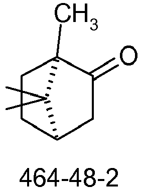 | 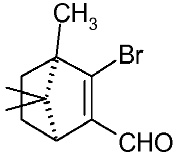 | 15% |
| S4 | 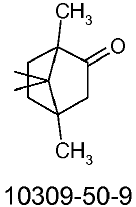 | 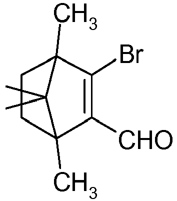 | 8% |
| S5 | 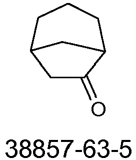 | 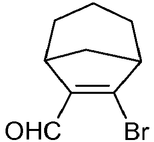 | 21% |
| S6 | 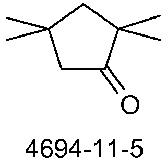 | 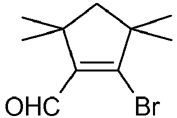 | 14% |
| S7 | 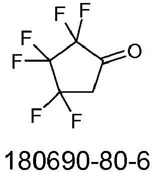 | 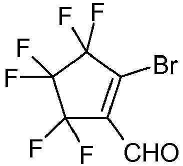 | 44% |
| S8 | 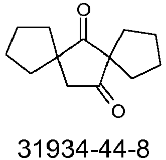 | 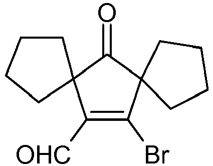 | 13% |
| S9 | 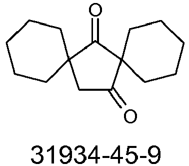 | 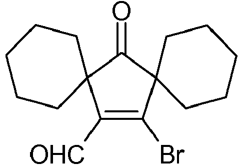 | 10% |
| S10 | 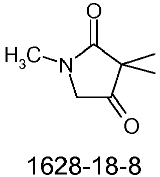 | 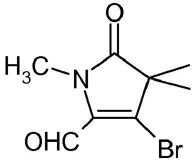 | 22% |
| S11 | 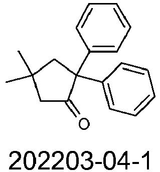 | 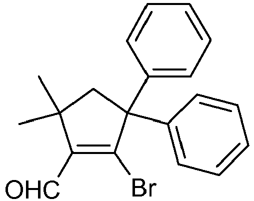 | 15% |
| S12 | 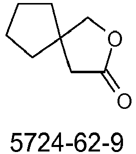 | 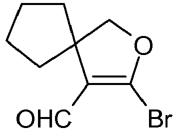 | 12% |
| S13 | 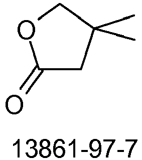 | 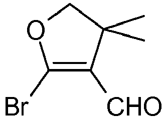 | 16% |
| S14 | 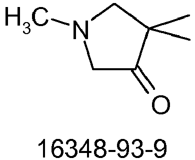 | 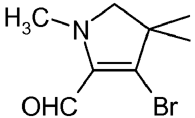 | 15% |
| S15 | 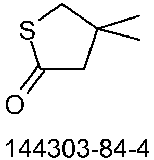 | 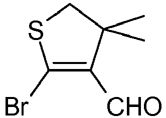 | 17% |

### Allgemeine Vorschrift zu Schritt 2: N-Acetylierung eines o-Amino-bromnaphthalins

Ein 1-Amino-2-bromaromat (100 mmol) wird portionsweise und unter gutem Rühren in eine auf 0 °C gekühlte Vorlage mit Essigsäureanhydrid (2.1 ml, 20 mmol) gegeben. Das Eisbad wird entfernt und die Reaktionsmischung wird für 30 Minuten auf Rückfluss erhitzt. Nach Abkühlen wird die Essigsäure und das überschüssige Essigsäureanhydrid unter Vakuum abdestilliert. Der Rückstand wird in Dichlormethan oder Ethylacetat gelöst und über eine Kieselgelsäule filtriert, so dass man den 1-(N-acetylamin)-2-bromaromaten in 80-95% Ausbeute erhält.

### Beispiel für S100:

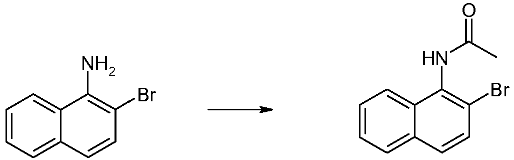

8.5 ml Essigsäureanhydrid (90 mmol, 2 Eq) werden vorgelegt und auf 0 °C gekühlt. In kleinen Portionen werden 10 g (45 mmol) 2-Brom-aminonaphthalin zugegeben. Die Reaktionsmischung wird langsam auf Raumtemperatur erwärmen lassen, danach für 1 h unter Rückfluss erhitzt. Es wird abgekühlt und die Essigsäure bzw. das überschüssige Essigsäureanhydrid im Vakuum abdestilliert. Der Rückstand wird in Ethylacetat gelöst und über eine Kieselgelsäule filtriert. Es werden 10.5 g (88%, 39.8 mmol) eines weißen Feststoffs erhalten.

Folgende N-Acetyl-bromaromaten können nach der allgemeinen Vorschrift in der angegebenen Ausbeute erhalten werden:

| **Bsp.** | **Amino-2- bromaromat** | **N-Acetyl-amino- 2-bromaromat** | **Ausbeute** |
|---|---|---|---|
| S100 | 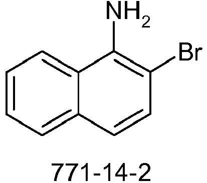 | 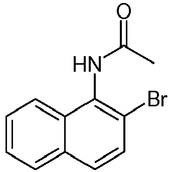 | 88% |
| S101 | 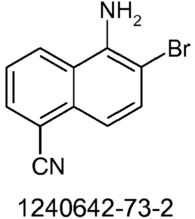 | 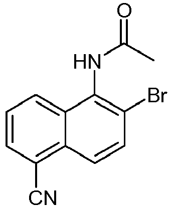 | 85% |
| S102 | 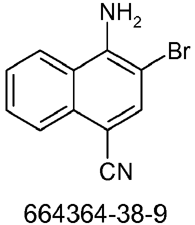 | 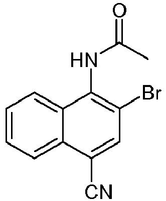 | 92% |
| S103 | 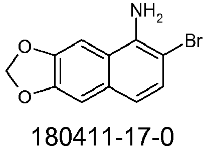 | 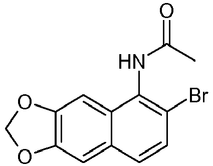 | 89% |
| S104 | 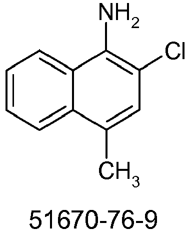 | 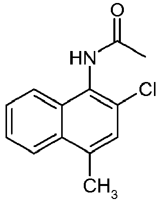 | 95% |
| S105 | 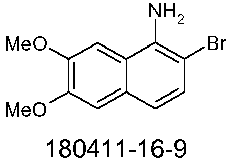 | 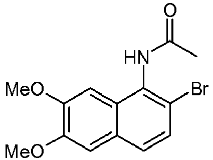 | 80% |

### Allgemeine Vorschrift zu Schritt 3: Kupplung des Bromalkencarbaldehyds mit 1-(N-acetylamino)-2-bromnaphthalin und anschließender Cyclisierung

Eine Mischung aus Bromalkencarbaldehyd (5 mmol), dem 1-(N-acetyl-amino)-2-bromaromaten (5 mmol) aus Schritt 2, Kupferpulver (2.6 g, 41 mmol) und Pd(PPh₃)₄ (577 mg, 0.5 mmol) wird in 15 ml trockenem DMSO gelöst und sorgfältig inertisiert. Die Reaktionsmischung wird für 12 h auf 85 °C erhitzt, dann wasserfreies K₂CO₃ zugegeben und weitere 6 h erhitzt. Die Reaktionsmischung wird abgekühlt und mit 100 ml Ethylacetat verdünnt. Die Mischung wird filtriert, das Filtrat mit Wasser gewaschen, über Natriumsulfat getrocknet und vom Lösemittel unter Vakuum befreit. Der Rückstand wird säulenchromatographisch aufgereinigt und ein farbloses Pulver wird in 35-65% Ausbeute erhalten.

### Beispiel für L501

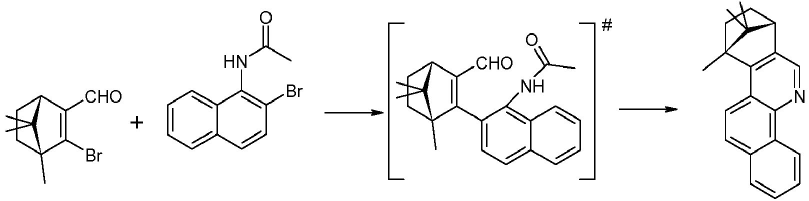

Eine Mischung aus Bromalkencarbaldehyd (S2, 1.22 g, 5 mmol), dem 1-(N-acetylamino)-2-bromnaphthalin (S100, 1.32 g, 5 mmol), Kupferpulver (2.6 g, 41 mmol) und Pd(PPh₃)₄ (577 mg, 0.5 mmol) wird in 15 ml trockenem DMSO gelöst und sorgfältig inertisiert. Die Reaktionsmischung wird für 12 h auf 85 °C erhitzt, dann wasserfreies K₂CO₃ zugegeben und weitere 6 h erhitzt. Die Reaktionsmischung wird abgekühlt und mit 100 ml Ethylacetat verdünnt. Die Mischung wird filtriert, das Filtrat mit Wasser gewaschen, über Natriumsulfat getrocknet und vom Lösemittel unter Vakuum befreit. Der Rückstand wird säulenchromatographisch aufgereinigt (Kieselgel, Eluent Heptan/EE 3:1), und ein farbloses Pulver wird in 65% Ausbeute (1.13 g, 3.2 mmol) erhalten.

Aus den Synthonen S1 - S15 können nach der allgemeinen Vorschrift die folgenden Liganden hergestellt werden:

| **Bsp.** | **Methylenketon** | **Edukt 2** | **Ligand** | **Aus beute** |
|---|---|---|---|---|
| L500 | S1 | S100 | 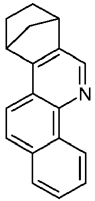 | 59% |
| L501 | S2 | S100 | 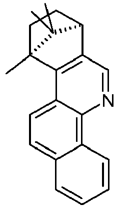 | 65% |
| L502 | S3 | S100 | 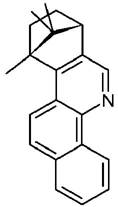 | 66% |
| L503 | S4 | S100 | 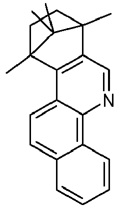 | 49% |
| L504 | S5 | S100 | 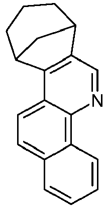 | 68% |
| L505 | S6 | S100 | 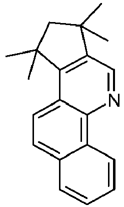 | 68% |
| L506 | S7 | S100 | 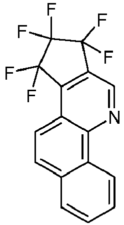 | 62% |
| L507 | S8 | S100 | 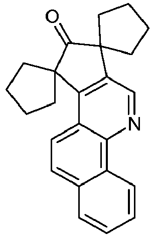 | 60% |
| L508 | S9 | S100 | 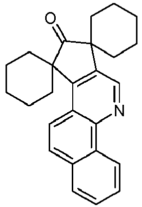 | 37% |
| L509 | S10 | S100 | 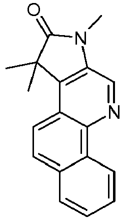 | 62% |
| L510 | S11 | S100 | 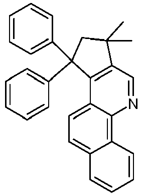 | 61% |
| L511 | S12 | S100 | 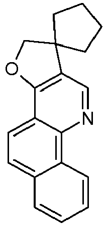 | 37% |
| L512 | S13 | S100 | 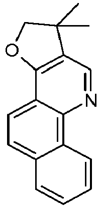 | 38% |
| L513 | S14 | S100 | 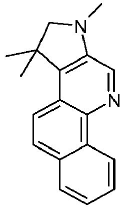 | 43% |
| L514 | S15 | S100 | 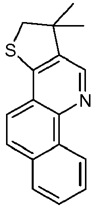 | 47% |
| L515 | S1 | S101 | 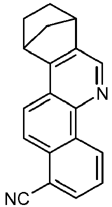 | 52% |
| L516 | S2 | S101 | 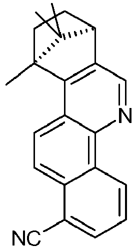 | 58% |
| L517 | S3 | S101 | 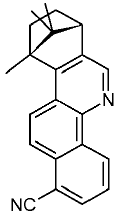 | 37% |
| L518 | S4 | S101 | 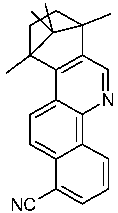 | 48% |
| L519 | S5 | S101 | 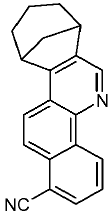 | 71% |
| L520 | S6 | S101 | 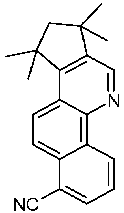 | 54% |
| L521 | S7 | S101 | 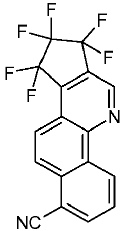 | 67% |
| L522 | S8 | S101 | 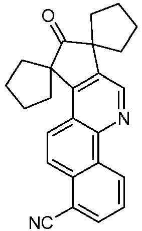 | 51% |
| L523 | S9 | S101 | 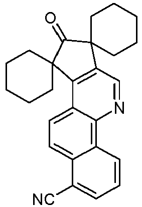 | 53% |
| L524 | S10 | S101 | 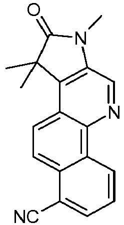 | 41% |
| L525 | S11 | S101 | 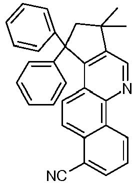 | 51% |
| L526 | S12 | S101 | 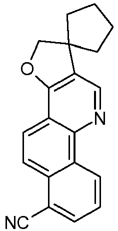 | 56% |
| L527 | S13 | S101 | 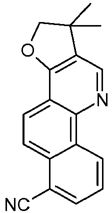 | 61% |
| L528 | S14 | S101 | 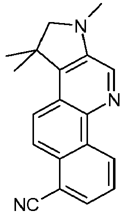 | 41% |
| L529 | S15 | S101 | 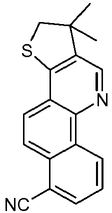 | 59% |
| L530 | S1 | S102 | 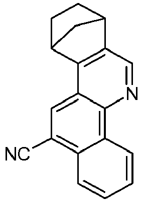 | 65% |
| L531 | S2 | S102 | 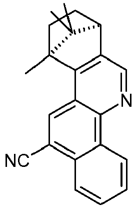 | 45% |
| L532 | S3 | S102 | 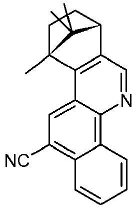 | 47% |
| L533 | S4 | S102 | 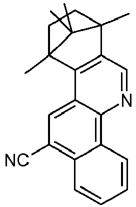 | 56% |
| L534 | S5 | S102 | 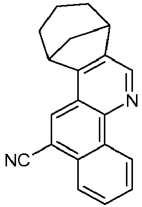 | 68% |
| L535 | S6 | S102 | 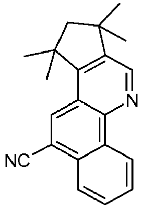 | 72% |
| L536 | S7 | S102 | 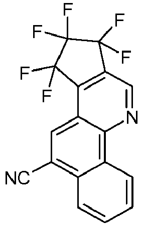 | 55% |
| L537 | S8 | S102 | 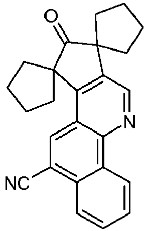 | 56% |
| L538 | S9 | S102 | 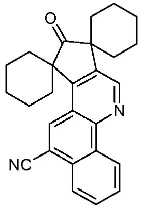 | 57% |
| L539 | S10 | S102 | 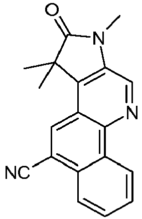 | 44% |
| L540 | S11 | S102 | 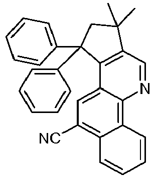 | 36% |
| L541 | S12 | S102 | 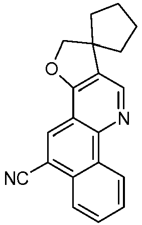 | 42% |
| L542 | S13 | S102 | 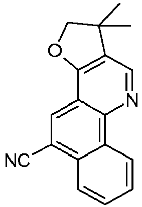 | 53% |
| L543 | S14 | S102 | 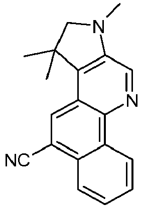 | 67% |
| L544 | S15 | S102 | 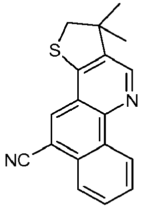 | 67% |
| L545 | S1 | S103 | 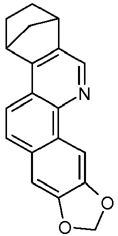 | 35% |
| L546 | S2 | S103 | 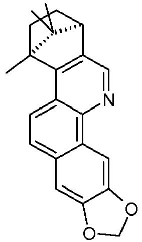 | 63% |
| L547 | S3 | S103 | 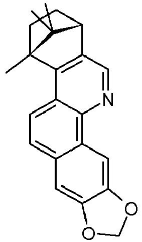 | 68% |
| L548 | S4 | S103 | 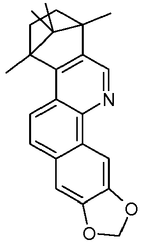 | 64% |
| L549 | S5 | S103 | 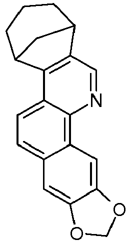 | 38% |
| L550 | S6 | S103 | 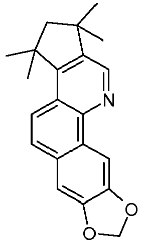 | 36% |
| L551 | S7 | S103 | 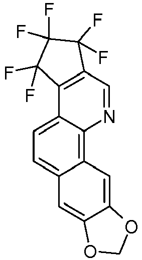 | 56% |
| L552 | S8 | S103 | 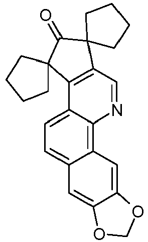 | 43% |
| L553 | S9 | S103 | 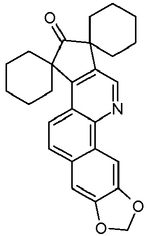 | 60% |
| L554 | S10 | S103 | 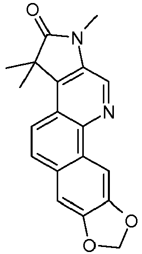 | 41% |
| L555 | S11 | S103 | 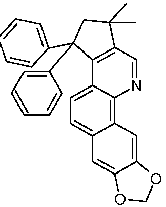 | 58% |
| L556 | S12 | S103 | 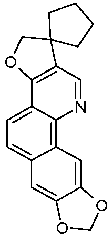 | 65% |
| L557 | S13 | S103 | 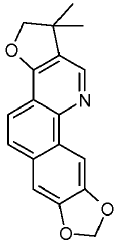 | 35% |
| L558 | S14 | S103 | 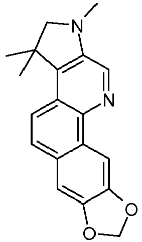 | 38% |
| L559 | S15 | S103 | 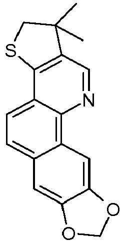 | 53% |
| L560 | S1 | S104 | 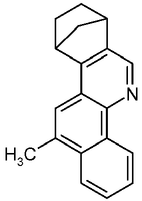 | 56% |
| L561 | S2 | S104 | 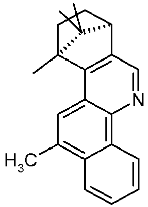 | 36% |
| L562 | S3 | S104 | 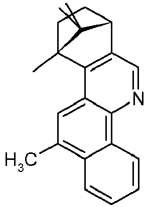 | 63% |
| L563 | S4 | S104 | 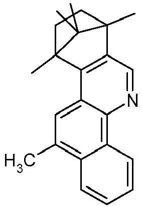 | 36% |
| L564 | S5 | S104 | 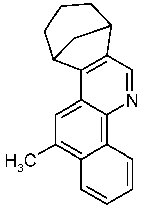 | 64% |
| L565 | S6 | S104 | 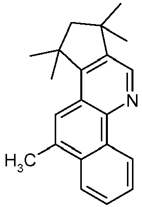 | 65% |
| L566 | S7 | S104 | 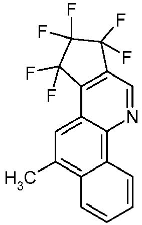 | 66% |
| L567 | S8 | S104 | 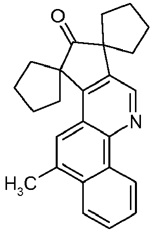 | 58% |
| L568 | S9 | S104 | 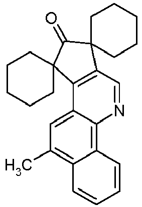 | 51% |
| L569 | S10 | S104 | 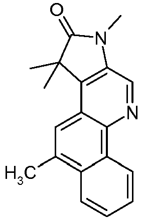 | 60% |
| L570 | S11 | S104 | 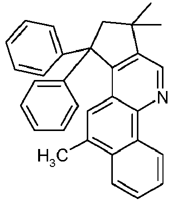 | 65% |
| L571 | S12 | S104 | 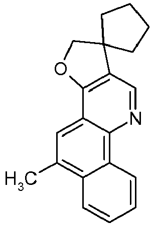 | 55% |
| L572 | S13 | S104 | 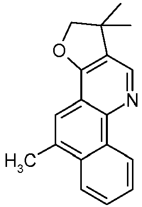 | 44% |
| L573 | S14 | S104 | 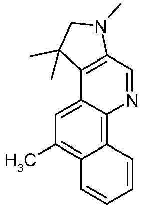 | 40% |
| L574 | S15 | S104 | 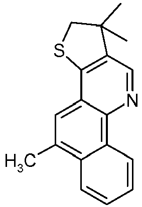 | 58% |
| L575 | S1 | S105 | 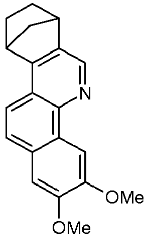 | 38% |
| L576 | S2 | S105 | 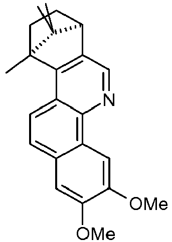 | 56% |
| L577 | S3 | S105 | 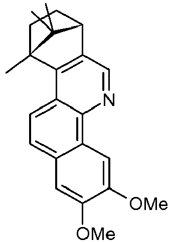 | 70% |
| L578 | S4 | S105 | 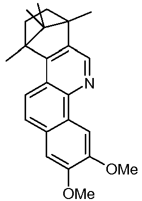 | 62% |
| L579 | S5 | S105 | 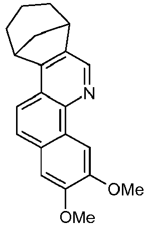 | 34% |
| L580 | S6 | S105 | 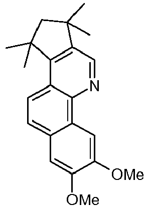 | 35% |
| L581 | S7 | S105 | 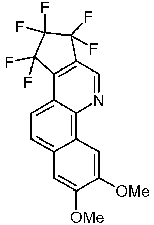 | 69% |
| L582 | S8 | S105 | 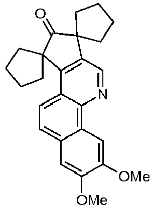 | 66% |
| L583 | S9 | S105 | 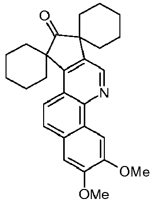 | 50% |
| L584 | S10 | S105 | 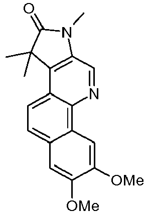 | 42% |
| L585 | S11 | S105 | 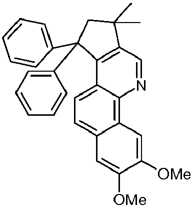 | 60% |
| L586 | S12 | S105 | 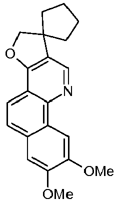 | 39% |
| L587 | S13 | S105 | 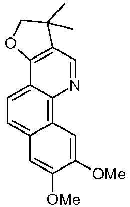 | 48% |
| L588 | S14 | S105 | 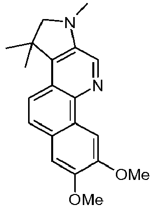 | 64% |
| L589 | S15 | S105 | 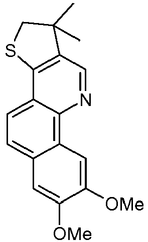 | 49% |

### 5,6-substituierte Benzo[h]chinoline

### Reaktionsschema:

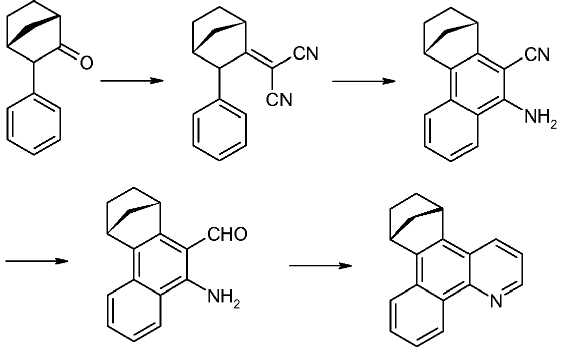

### Allgemeine Vorschriften:

### Schritt 1: Kupplung eines aliphatischen Ketons mit Malodinitril

Das Keton (50 mmol), Malodinitril (50 mmol, 1 eq), Ammoniumacetat (0.75 g, 10 mmol, 0.2 eq) und Essigsäure (2.3 ml, 40 mmol, 0.8 eq) werden vorgelegt und 45 ml wasserfreies Benzol zugegeben. Die Reaktionsmischung wird am Wasserabscheider unter Rückfluss gekocht, bis keine Edukte mehr nachweisbar sind. Nach dem Abkühlen wird die Mischung mit Wasser und gesättigter NaHCO₃-Lösung gewaschen und über Magnesiumsulfat getrocknet. Das Benzol wird im Vakuum abgezogen und der Rückstand wird aus Heptan umkristallisiert.

### Beispiel: Kupplung des Phenylketons mit Malodinitril

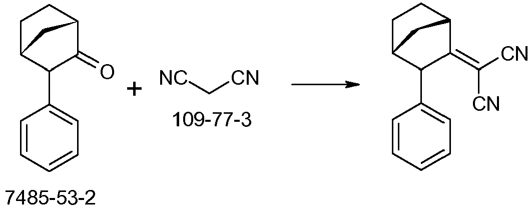

(1S,4R)-3-Phenyl-bicyclo[2.2.1]heptan-2-on (9.3 g, 50 mmol), Malodinitril (3.3 g, 50 mmol), Ammoniumacetat (0.75 g, 10 mmol) und Essigsäure (2.3 ml, 40 mmol) werden vorgelegt und 45 ml wasserfreies Benzol zugegeben. Die Reaktionsmischung wird für 4 h am Wasserabscheider Rückfluss gekocht. Nach dem Abkühlen wird die Mischung mit Wasser und gesättigter NaHCO₃-Lösung gewaschen und über Magnesiumsulfat getrocknet. Das Benzol wird im Vakuum abgezogen und der Rückstand wird aus Heptan umkristallisiert. Man erhält 5.8 g (40 mmol, 81%) eines farblosen Feststoffs.

### Schritt 2: Cyclisierung

Das Edukt (35 mmol) wird langsam in 5 °C kalter konzentrierter Schwefelsäure (25 ml) gelöst und über Nacht bei Raumtemperatur gerührt. Die Reaktionsmischung wird auf Eis gegeben und der ausgefallene Feststoff wird abfiltriert, mit Wasser gewaschen und getrocknet. Das Produkt wird mehrfach aus Methanol umkristallisiert.

### Beispiel: Cyclisierung

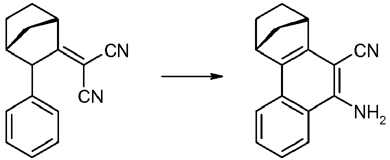

Das Edukt (5 g, 35 mmol) wird langsam in 5 °C kalter konzentrierter Schwefelsäure (25 ml) gelöst und über Nacht bei Raumtemperatur gerührt. Die Reaktionsmischung wird auf Eis gegeben, der ausgefallene Feststoff wird abfiltriert, mit Wasser gewaschen und getrocknet. Das Produkt wird mehrfach aus Methanol umkristallisiert. Es werden 3.5 g (14 mmol, 40%) des Produkts erhalten.

### Schritt 3: Reduktion des o-Aminonitrils zu o-Aminocarbaldehvd

Das ortho-Aminonitril (10 mmol) wird in 25 ml wasserfreiem Dichlormethan gelöst und auf 0 °C abgekühlt. Es wird langsam eine 1M Lösung DIBAL-H in Toluol (15 mmol, 1.5 eq) zugetropft und die Lösung bei Raumtemperatur für 24 h gerührt. Die Reaktionsmischung wird mit wasserfreiem Diethylether verdünnt und auf 0 °C abgekühlt. Es wird langsam 0.6 ml Wasser, dann 0.6 ml einer 15%igen wässrigen NaOH-Lösung, dann erneut 1.5 ml Wasser vorsichtig zugetropft und die Lösung für 15 Minuten gerührt. Wasserfreies Magnesiumsulfat wird zugegeben, für weitere 15 Minuten gerührt und filtriert. Die Lösemittel werden im Vakuum entfernt und das Rohprodukt per Säulenchromatographie mit einer Mischung aus Dichlormethan/Heptan gereinigt.

### Beispiel: Hydrolyse

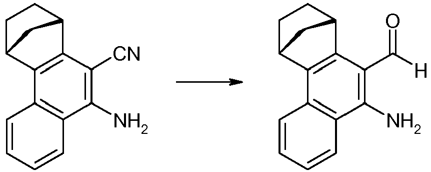

Das ortho-Aminonitril (2.34 g, 10 mmol) wird in 25 ml wasserfreiem Dichlormethan gelöst und auf 0 °C abgekühlt. Es wird langsam eine 1M Lösung DIBAL-H in Toluol (15 ml, 15 mmol, 1.5 eq) zugetropft und die Lösung bei Raumtemperatur für 24 h gerührt. Die Reaktionsmischung wird mit wasserfreiem Diethylether verdünnt und auf 0 °C abgekühlt. Es wird langsam 0.6 ml Wasser, dann 0.6 ml einer 15%igen wässrigen NaOH-Lösung, dann erneut 1.5 ml Wasser vorsichtig zugetropft und die Lösung für 15 Minuten gerührt. Wasserfreies Magnesiumsulfat wird zugegeben, für weitere 15 Minuten gerührt und abfiltriert. Die Lösemittel werden im Vakuum entfernt und das Rohprodukt per Säulenchromatographie mit einer Mischung aus Dichlormethan/Heptan (1:1) gereinigt. Man erhält einen farblosen Feststoff in 67% Ausbeute (1.69 g, 6.7 mmol).

### Schritt 4: Überführung des ortho-Aminocarbaldehvds in das 5,6-substituierte Benzo[h]chinolin

Der o-Aminocarbaldehyd (4 mmol) und Acetaldehyd (4 mmol, 1 eq) werden vorgelegt und mit 15 ml trockenem Ethanol versetzt. Es wird langsam pulvriges Kaliumhydroxid (4.8 mmol, 1.2 Eq) zugegeben und die Reaktionsmischung unter Rückfluss für 24 h gerührt. Nach vollständigem Umsatz wird die Lösung abgekühlt, mit Dichlormethan versetzt und über Celite filtriert. Die organische Phase wird mit Wasser gewaschen, über Magnesiumsulfat getrocknet und vom Lösemittel im Vakuum befreit. Das Produkt wird per Säulenchromatographie gereinigt.

### Beispiel: Cyclisierung zu L1000

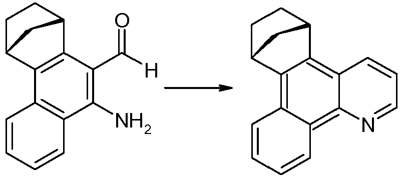

Der o-Aminocarbaldehyd (1 g, 4 mmol) und Acetaldehyd (177 mg, 0.225 ml, 4 mmol) werden vorgelegt und mit 15 ml trockenem Ethanol versetzt. Es wird langsam pulvriges Kaliumhydroxid (0.27 g, 4.8 mmol, 1.2 Eq) zugegeben und die Reaktionsmischung unter Rückfluss für 24 h gerührt. Nach vollständigem Umsatz wird die Lösung abgekühlt und mit Dichlormethan versetzt und über Celite filtriert. Die organische Phase wird mit Wasser gewaschen, über Magnesiumsulfat getrocknet und vom Lösemittel im Vakuum befreit. Das Produkt wird per Säulenchromatographie gereinigt und ergibt 0.71 g (2.9 mmol, 74%) eines farblosen Feststoffs.

Unter Durchführung der allgemeinen Vorschriften können folgende Liganden hergestellt werden:

| **Bsp.** | **Keton** | **Ligand** | **Ausbeute über 4 Stufen** |
|---|---|---|---|
| L1000 | 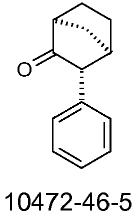 | 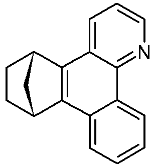 | 8,4% |
| L1001 | 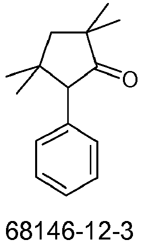 | 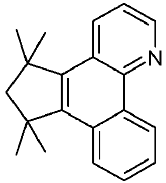 | 9,3% |
| L1002 | 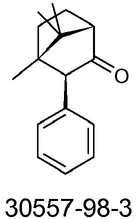 | 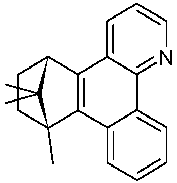 | 7,9% |

### 7,8-substituierte Benzo[h]chinoline

### Reaktionsschema:

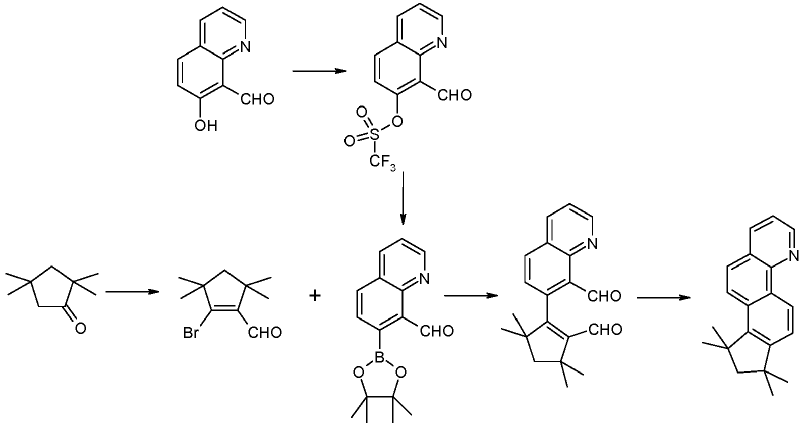

### Synthese-Vorschriften:

### Allgemeine Vorschrift: Überführung eines o-Phenol-Carbaldehvds in einen o-Trifluormethansulfonsäure-Carbaldehyd

Der Hydroxychinolincarbaldehyd (50 mmol) wird in 25 ml Dichlormethan gelöst und auf 0 °C abgekühlt. Eine auf 0 °C gekühlte Lösung aus Pyridin (12.6 ml, 75 mmol, 1.5 eq) und Trifluormethansulfonsäureanhydrid (8.1 ml, 100 mmol, 2 eq) wird langsam innerhalb von 15 Minuten zugetropft und die Reaktionsmischung für 12-24 h bei Raumtemperatur gerührt. Die Reaktion wird durch Zugabe von 60 ml Wasser abgebrochen und die organische Phase abgetrennt. Die wässrige Phase wird mehrfach mit Diethylether extrahiert und die vereinigten organischen Phasen über Magnesiumsulfat getrocknet. Das Lösemittel wird im Vakuum entfernt und der Rückstand per Säulenchromatographie aufgereinigt.

### Beispiel:

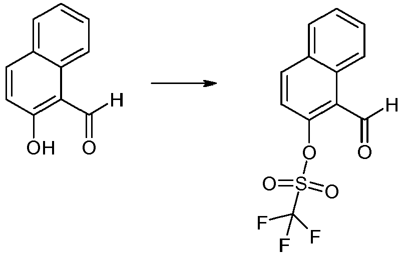

Der 2-Hydroxynaphthalincarbaldehyd (8.61 g, 50 mmol) wird in 25 ml Dichlormethan gelöst und auf 0 °C abgekühlt. Eine auf 0 °C gekühlte Lösung aus Pyridin (12.6 ml, 75 mmol, 1.5 eq) und Trifluormethansulfonsäureanhydrid (8.1 ml, 100 mmol, 2 eq) wird langsam innerhalb von 15 Minuten zugetropft und die Reaktionsmischung für 24 h bei Raumtemperatur gerührt. Die Reaktion wird durch Zugabe von 60 ml Wasser abgebrochen und die organische Phase abgetrennt. Die wässrige Phase wird mehrfach mit Diethylether extrahiert und die vereinigten organischen Phasen über Magnesiumsulfat getrocknet. Das Lösemittel wird im Vakuum entfernt und der Rückstand per Säulenchromatographie aufgereinigt. Man erhält (75%, 11.4 g, 37.5 mmol) eines farbloses Öls.

### Schritt 2: Überführung des o-Trifluormethansulfonsäurecarbaldehvd in ein Pinacolborancarbaldehyd

Eine Mischung aus PdCl₂(dppf)₂ (220 mg, 0.3 mmol, 0.03 eq), dem Trifluormethansulfonsäurecarbaldehyd (10 mmol), Triethylamin (4.2 ml, 30 mmol) und Pinacolboran (2.2 ml, 15 mmol) wird sorgfältig inertisiert und in 40 ml Dioxan gelöst. Die Reaktionsmischung wird für 3-12 h auf 80 °C erhitzt, abgekühlt, mit Wasser verdünnt und mit Toluol extrahiert. Die organischen Phasen werden mit Wasser und gesättigter NaCl-Lösung gewaschen, über Magnesiumsulfat getrocknet und vom Lösemittel im Vakuum befreit. Der Rückstand wird per Säulenchromatographie aufgereinigt.

### Beispiel:

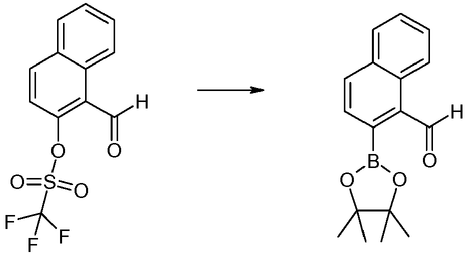

Eine Mischung aus PdCl₂(dppf)₂ (220 mg, 0.3 mmol, 0.03 eq), 2-Trifluormethansulfonyl-naphthalincarbaldehyd (2.48 g, 10 mmol), Triethylamin (4.2 ml, 30 mmol) und Pinacolboran (2.2 ml, 15 mmol) wird sorgfältig inertisiert und in 40 ml Dioxan gelöst. Die Reaktionsmischung wird für 12 h auf 80 °C erhitzt, abgekühlt, mit Wasser verdünnt und mit Toluol extrahiert. Die organischen Phasen werden mit Wasser und gesättigter NaCl-Lösung gewaschen, über Magnesiumsulfat getrocknet und vom Lösemittel im Vakuum befreit. Der Rückstand wird per Säulenchromatographie (EE/Heptan 1:10) aufgereinigt, und man erhält (65%, 1.84 g, 6.5 mmol) eines farblosen Öls.

### Schritt 3: Kupplung des Pinacolborancarbaldehvds mit dem Bromalkencarbaldehyd

5.5 mmol des Bromalkencarbaldehyd, 5.5 mmol (1 Eq) Pinacolborancarbaldehyd und 10.5mmol (1.9 Eq) Kaliumphosphat werden vorgelegt, in 25 ml Toluol, 25 ml Dioxan und 25 ml Wasser suspendiert und inertisiert. Zu dieser Suspension werden 45 mg Tri-o-tolylphosphin (0.15 mmol) und anschließend 6 mg Palladium(II)acetat (0.025 mmol) gegeben und die Reaktionsmischung für 24 h unter Rückfluss erhitzt. Nach Erkalten wird die Reaktionsmischung mit Toluol verdünnt, die organischen Phasen abgetrennt, mit Wasser gewaschen, über Kieselgel filtriert und über Magnesiumsulfat getrocknet. Das Lösemittel wird im Vakuum entfernt. Der Rückstand wird säulenchromatographisch gereinigt.

### Beispiel:

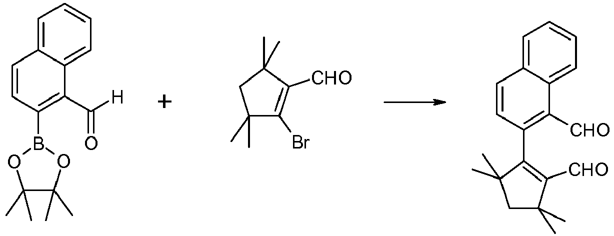

1.27 g (5.5 mmol) des Bromalkencarbaldehyds S6, 1.55 g (5.5 mmol, 1 eq) Pinacolborancarbaldehyd und 2.23 g (10.5 mmol, 1.9 eq) Kaliumphosphat werden vorgelegt, in 25 ml Toluol, 25 ml Dioxan und 25 ml Wasser suspendiert und inertisiert. Zu dieser Suspension werden 45 mg (0.15 mmol) Tri-o-tolylphosphin und anschließend 6 mg (0.025 mmol) Palladium(II)acetat gegeben und die Reaktionsmischung für 24 h unter Rückfluss erhitzt. Nach Erkalten wird die Reaktionsmischung mit Toluol verdünnt, die organischen Phasen abgetrennt, mit Wasser gewaschen, über Kieselgel filtriert und über Magnesiumsulfat getrocknet. Das Toluol wird im Vakuum entfernt. Der Rückstand wird säulenchromatographisch getrennt (Ethylacetat/Heptan 1:9), und man erhält 1.52 g (4.95 mmol, 95%) eines farblosen Feststoffs.

### Schritt 4: McMurry-Kupplung des Biscarbaldehyds

Zu einer auf 0 °C abgekühlten und inertisierten Suspension aus aktiviertem Zinkstaub (3.2 g, 49 mmol) in 60 ml THF werden langsam 3 ml (27.4 mmol) TiCl₄ zugetropft. Die Suspension wird für 2 h unter Rückfluss erhitzt, auf 0 °C gekühlt und eine Lösung des Bisaldehyds (5.3 mmol) in 20 ml THF innerhalb von 30 Minuten zugetropft. Die Reaktionsmischung wird langsam auf Raumtemperatur aufgewärmt, dann für zwei h unter Rückfluss erhitzt. Nach Abkühlen wird die Lösung in eine auf 0 °C gekühlte gesättigte Kaliumcarbonat-Lösung gegossen und mit Dichlormethan extrahiert. Die organischen Phasen werden vereinigt und mit Wasser und gesättigter NaCl-Lösung gewaschen. Man trocknet über Natriumsulfat, entfernt das Lösemittel im Vakuum und reinigt per Säulenchromatographie auf.

### Beispiel L1500:

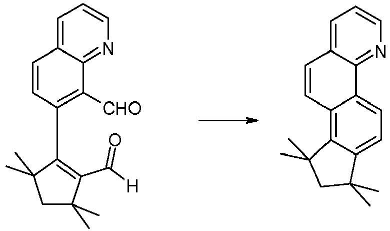

Zu einer auf 0 °C abgekühlten und inertisierten Suspension aus aktiviertem Zinkstaub (3.2 g, 49 mmol) in 60 ml THF werden langsam 3 ml (27.4 mmol) TiCl₄ zugetropft. Die Suspension wird für 2 h unter Rückfluss erhitzt, auf 0 °C gekühlt und eine Lösung des Bisaldehyds (1.63 g, 5.3mmol) in 20 ml THF innerhalb von 30 Minuten zugetropft. Die Reaktionsmischung wird langsam auf Raumtemperatur aufgewärmt, dann für zwei h unter Rückfluss gekocht. Nach Abkühlen wird die Lösung in eine auf 0 °C gekühlte gesättigte Kaliumcarbonat-Lösung gegossen und mit Dichlormethan extrahiert. Die organischen Phasen werden vereinigt und mit Wasser und gesättigter NaCl-Lösung gewaschen. Man trocknet über Natriumsulfat, entfernt das Lösemittel im Vakuum, reinigt per Säulenchromatographie (Kieselgel, Ethylacetat/Heptan 1:5) auf und erhält 1.13 g (4.09 mmol, 73%) eines farblosen Feststoffs.

Durch Kupplung der Synthone S1 - S15 mit 7-(4,4,5,5,-Tetramethyl-[1,3,2]-dioxaborolan-2yl)-chinolin-8-carbaldehyd können folgende Liganden erhalten werden:

| **Bsp.** | **Bromalken-carbaldehyd** | **Ligand** | **Ausbeute über 4 Stufen** |
|---|---|---|---|
| L1500 | S6 | 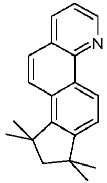 | 55% |
| L1501 | S1 | 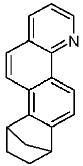 | 47% |
| L1502 | S2 | 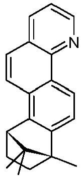 | 48% |
| L1503 | S3 | 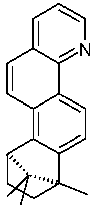 | 40% |
| L1504 | S4 | 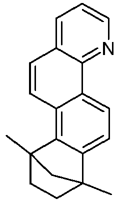 | 54% |
| L1505 | S5 | 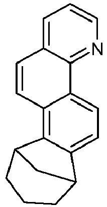 | 40% |
| L1506 | S7 | 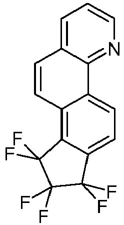 | 55% |
| L1507 | S11 | 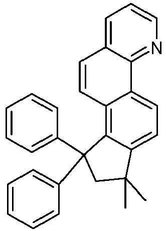 | 50% |
| L1508 | S12 | 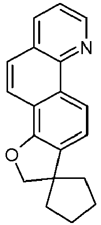 | 57% |
| L1509 | S13 | 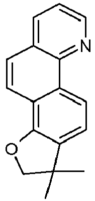 | 40% |
| L1510 | S14 | 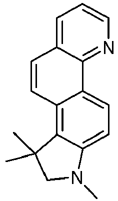 | 51% |
| L1511 | S15 | 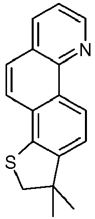 | 45% |

### Synthese der Metallkomplexe

### 1) Homoleptische tris-faciale Iridium-Komplexe:

### Variante A: Trisacetylacetonato-iridium(III) als Iridium-Edukt

Ein Gemisch aus 10 mmol Tris-acetylacetonato-iridium(III) [15635-87-7], 40-60 mmol des Liganden L, gegebenenfalls 1 g eines inerten hochsiedenden Zusatzes als Schmelzhilfe bzw. Lösungsmittel, z. B. Hexadecan, m-Terphenyl, Triphenylen, Diphenylether, 3-Phenoxytoluol, 1,2-, 1,3-, 1,4-Bisphenoxybenzol, Triphenylphosphinoxid, Sulfolan, 18-Krone-6, Triethylenglykol, Glycerin, Polyethylenglykole, Phenol, 1-Naphthol, etc., und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Um eine Sublimation der Liganden an kältere Stellen der Ampulle zu vermeiden, muss die gesamte Ampulle die angegebene Temperatur besitzen. Alternativ kann die Synthese in einem Rührautoklaven mit Glaseinsatz erfolgen. Nach Erkalten (ACHTUNG: die Ampullen stehen meist unter Druck!) wird die Ampulle geöffnet, der Sinterkuchen wird mit 100 g Glaskugeln (3 mm Durchmessser) in 100 ml eines Suspensionsmittels (das Suspensionsmittel wird so gewählt, dass der Ligand gut, der Metallkomplex jedoch schlecht darin löslich ist, typische Suspensionsmittel sind Methanol, Ethanol, Dichlormethan, Aceton, THF, Ethylacetat, Toluol, etc.) 3 h gerührt und dabei mechanisch aufgeschlossen. Man dekantiert die feine Suspension von den Glaskugeln ab, saugt den Feststoff ab, wäscht mit 50 ml des Suspensionsmittels nach, und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit einem Extraktionsmittel (Vorlagemenge ca. 500 ml, das Extraktionsmittel wird so gewählt, dass der Komplex darin in der Hitze gut und in der Kälte schlecht löslich ist; besonders geeignete Extraktionsmittel sind Kohlenwasserstoffe wie Toluol, Xylole, Mesitylen, Naphthalin, o-Dichlorbenzol, Tetrahydrofuran, Dichlormethan, 1,2-Dichlorethan, 1,1,2,2-Tetrachlorethan, Chloroform, Tetrachlorkohlenstoff) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspension wird abgesaugt, einmal mit ca. 50 ml Methanol gewaschen und getrocknet. Nach Trocknen wird die Reinheit des Metallkomplexes mittels NMR und/oder HPLC bestimmt. Liegt die Reinheit unter 99.5 % wird der Heißextraktionsschritt wiederholt, wobei ab der 2. Extraktion das Alox-Bett weggelassen wird. Ist eine Reinheit von 99.5 - 99.9 % erreicht, wird der Metallkomplex getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 230 - 400 °C, wobei die Sublimation bevorzugt in Form einer fraktionierten Sublimation durchgeführt wird. Gut in organischen Lösungsmitteln lösliche Komplexe können alternativ auch an Kieselgel chromatographiert werden.

Werden Liganden der Punktgruppe C1 racemisch eingesetzt, fallen die abgeleiteten fac-Metallkomplexe als Diastereomerenmischung an. Das Enantiomerenpaar der Punktgruppe C3 weist in der Regel eine deutlich geringere Löslichkeit im Extraktionsmittel auf als das der Punktgruppe C1, das sich folglich in der Mutterlauge anreichert. Eine Trennung der Diasteromeren auf diesem Wege ist häufig möglich. Daneben können die Diastereomeren auch chromatographisch getrennt werden. Werden Liganden der Punktgruppe C1 enantiomerenrein eingesetzt, entsteht das Δ-, Λ-Diastereomerenpaar der Punktgruppe C3.

### Variante B: Tris-(2,2,6,6-tetramethyl-3,5-heptandionato)iridium(III) als Iridium-Edukt

Durchführung analog zu Variante A, wobei anstelle von 10 mmol Tris-acetylacetonato-iridium(III) [15635-87-7] 10 mmol Tris-(2,2,6,6-tetramethyl-3,5-heptandionato)iridium [99581-86-9] eingesetzt werden. Die Verwendung dieses Edukts ist vorteilhaft, da die Reinheit der erhaltenen Rohprodukte häufig besser ist als bei Variante A. Außerdem ist der Druckaufbau in der Ampulle häufig nicht so ausgeprägt.

### Vaiante C: Natrium[cis-,trans-dichloro-(bisacetylacetonato]iridat(III) als Iridium-Edukt

Ein Gemisch aus 10 mmol Natrium[cis-,trans-dichloro-(bisacetyl-acetonato]-iridat(III) [876296-21-8] und 60 mmol das Liganden in 50 ml Ethylen-, Propylen- oder Diethylenglykol wird unter einem leichten Argonstrom für die angegebene Zeit unter schwachem Rückfluss erhitzt. Nach Erkalten auf 60 °C verdünnt man unter Rühren mit einem Gemisch aus 50 ml Ethanol und 50 ml 2 N Salzsäure, rührt 1 h nach, saugt vom ausgefallenen Feststoff ab, wäscht diesen dreimal mit je 30 ml Ethanol und trocknet dann im Vakuum. Reinigung durch Heißextraktion oder Chromatographie und fraktionierte Sublimation, wie unter A beschrieben.

Die mit * markierten Komplexe sind nicht Gegenstand der Ansprüche.

| **Bsp.** | **Ligand** | **Ir-Komplex Diastereomer** | **Variante** | **Ausbeute %** |
|---|---|---|---|---|
| | | | **Reaktions-medium** | |
| | | | **Reaktions-temperatur** | |
| | | | **Reaktions-zeit** | |
| | | | **Susp.mittel** | |
| | | | **Extraktions-mittel** | |
| Ir(L1)₃ | L1 | 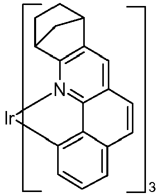 | A | 35 |
| | | | --- | |
| | | | 270°C | |
| | | | 48 h | |
| | | | Aceton | |
| | | | o-Xylol | |
| Ir(L2)₃ * | L2 | Ir(L2)₃ | wie Ir(L1)₃ | 12 |
| Ir(L8)₃ * | L8 | 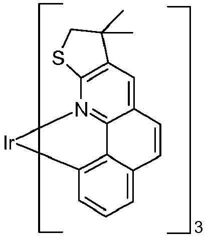 | A | 34 |
| | | | --- | |
| | | | 260°C | |
| | | | 48 h | |
| | | | Ethanol | |
| | | | o-Xylol | |
| Ir(L9)₃ | L9 | 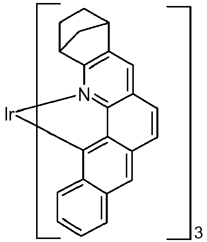 | A | 14 |
| | | | --- | |
| | | | 290°C | |
| | | | 48 h | |
| | | | Ethylacetat | |
| | | | o-Xylol | |
| Ir(L10)₃ * | L10 | Ir(L10)₃ | wie Ir(L9)₃ | 32 |
| Ir(L16)₃ | L16 | 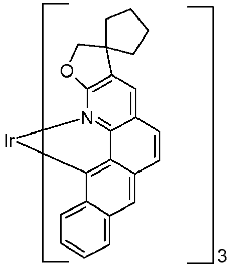 | C | 27 |
| | | | --- | |
| | | | 320°C | |
| | | | 48 h | |
| | | | Ethanol | |
| | | | o-Xylol | |
| Ir(L17)₃ * | L17 | 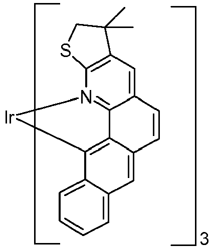 | A | 33 |
| | | | --- | |
| | | | 300°C 36 h | |
| | | | Ethanol | |
| | | | o-Xylol | |
| Ir(L18)₃ | L18 | 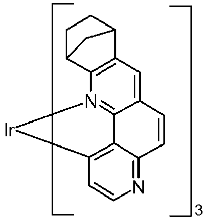 | A | 24 |
| | | | --- | |
| | | | 280°C | |
| | | | 48 h | |
| | | | Ethylacetat | |
| | | | o-Xylol | |
| Ir(L19)₃ * | L19 | 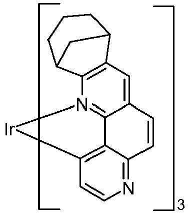 | A | 27 |
| | | | --- | |
| | | | 290°C | |
| | | | 60 h | |
| | | | Ethylacetat | |
| | | | o-Xylol | |
| Ir(L25)₃ | L25 | 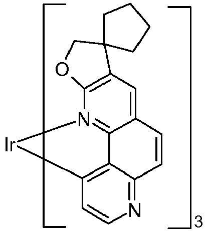 | A | 32 |
| | | | --- | |
| | | | 260°C | |
| | | | 24 h | |
| | | | Ethylacetat | |
| | | | o-Xylol | |
| Ir(L26)₃ * | L26 | 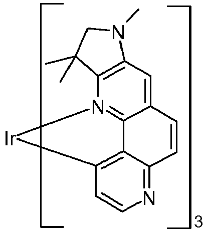 | A | 27 |
| | | | --- | |
| | | | 290°C | |
| | | | 48 h | |
| | | | Ethylacetat | |
| | | | o-Xylol | |
| Ir(L27)₃ * | L27 | 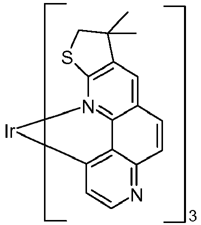 | A | 25 |
| | | | --- | |
| | | | 290°C | |
| | | | 24 h | |
| | | | Methanol | |
| | | | o-Xylol | |
| Ir(L28)₃ | L28 | 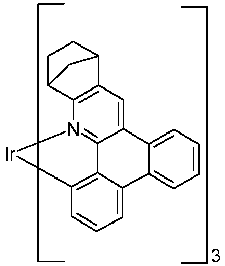 | A | 27 |
| | | | --- | |
| | | | 260°C | |
| | | | 60 h | |
| | | | Ethanol | |
| | | | o-Xylol | |
| Ir(L29)₃ * | L29 | 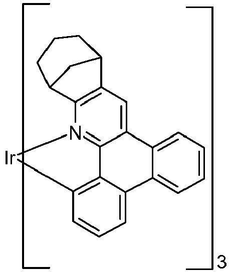 | C | 34 |
| | | | --- | |
| | | | 280°C | |
| | | | 60 h | |
| | | | Ethanol | |
| | | | o-Xylol | |
| Ir(L35)₃ | L35 | 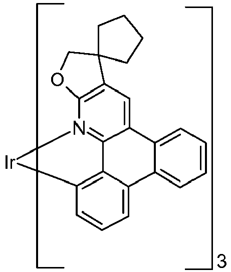 | C | 20 |
| | | | --- | |
| | | | 260°C | |
| | | | 48 h | |
| | | | Ethanol | |
| | | | o-Xylol | |
| Ir(L36)₃ * | L36 | 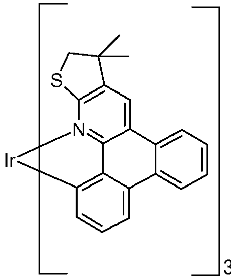 | C | 23 |
| | | | --- | |
| | | | 260°C | |
| | | | 48 h | |
| | | | Ethanol | |
| | | | o-Xylol | |
| Ir(L500)₃ | L500 | 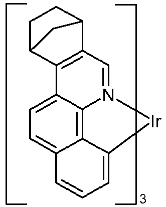 | A | 24 |
| | | | --- | |
| | | | 260°C | |
| | | | 24 h | |
| | | | Ethylacetat | |
| | | | o-Xylol | |
| Ir(L501)₃ | L501 | 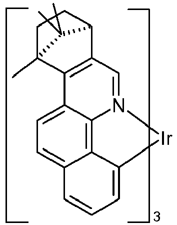 | A | 33 |
| | | | --- | |
| | | | 280°C | |
| | | | 24 h | |
| | | | Ethylacetat | |
| | | | o-Xylol | |
| Ir(L502)₃ | L502 | 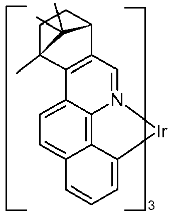 | A | 22 |
| | | | --- | |
| | | | 280°C | |
| | | | 24 h | |
| | | | Ethylacetat | |
| | | | o-Xylol | |
| Ir(L503)₃ | L503 | 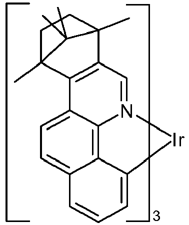 | A | 27 |
| | | | --- | |
| | | | 280°C | |
| | | | 24 h | |
| | | | Ethanol | |
| | | | o-Xylol | |
| Ir(L504)₃ * | L504 | 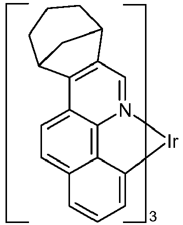 | A | 28 |
| | | | --- | |
| | | | 280°C | |
| | | | 24 h | |
| | | | Ethanol | |
| | | | o-Xylol | |
| Ir(L505)₃ | L505 | 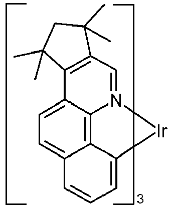 | A | 28 |
| | | | --- | |
| | | | 260°C | |
| | | | 24 h | |
| | | | Ethanol | |
| | | | o-Xylol | |
| Ir(L506)₃ * | L506 | 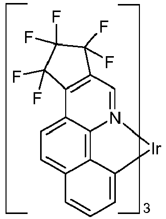 | A | 12 |
| | | | --- | |
| | | | 300°C | |
| | | | 48 h | |
| | | | Ethylacetat | |
| | | | o-Xylol | |
| Ir(L507)₃ * | L507 | 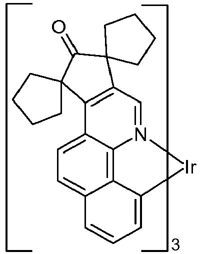 | B | 9 |
| | | | --- | |
| | | | 300°C | |
| | | | 48 h | |
| | | | Ethylacetat | |
| | | | o-Xylol | |
| Ir(L508)₃ * | L508 | 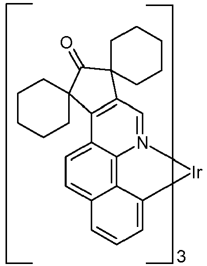 | B | 10 |
| | | | --- | |
| | | | 300°C | |
| | | | 48 h | |
| | | | Ethylacetat | |
| | | | o-Xylol | |
| Ir(L509)₃ * | L509 | 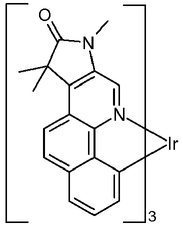 | A | 37 |
| | | | --- | |
| | | | 280°C | |
| | | | 24 h | |
| | | | Ethylacetat | |
| | | | o-Xylol | |
| Ir(L510)₃ | L510 | 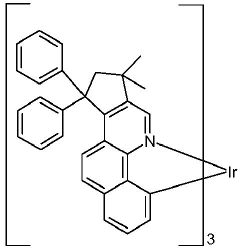 | B | 10 |
| | | | --- | |
| | | | 300°C | |
| | | | 24 h | |
| | | | Ethylacetat | |
| | | | o-Xylol | |
| Ir(L511)₃ | L511 | 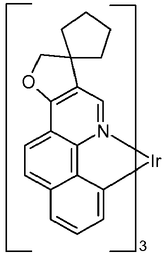 | A | 22 |
| | | | --- | |
| | | | 260°C | |
| | | | 24 h | |
| | | | Ethylacetat | |
| | | | o-Xylol | |
| Ir(L512)₃ | L512 | 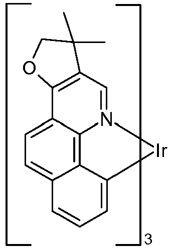 | A | 27 |
| | | | --- | |
| | | | 260°C | |
| | | | 24 h | |
| | | | Ethylacetat | |
| | | | o-Xylol | |
| Ir(L513)₃ * | L513 | 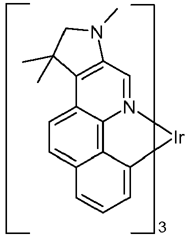 | A | 25 |
| | | | --- | |
| | | | 280°C | |
| | | | 24 h | |
| | | | Ethylacetat | |
| | | | o-Xylol | |
| Ir(L514)₃ * | L514 | 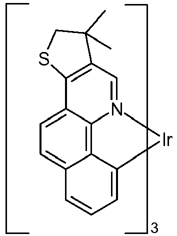 | A | 25 |
| | | | --- | |
| | | | 260°C | |
| | | | 24 h | |
| | | | Ethylacetat | |
| | | | o-Xylol | |
| Ir(L515)₃ | L515 | 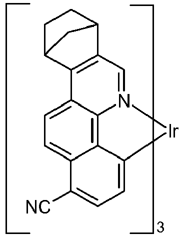 | C | 31 |
| | | | --- | |
| | | | 280°C | |
| | | | 24 h | |
| | | | Ethylacetat | |
| | | | o-Xylol | |
| Ir(L516)₃ | L516 | 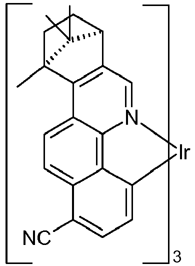 | C | 29 |
| | | | --- | |
| | | | 280°C | |
| | | | 24 h | |
| | | | Ethylacetat | |
| | | | o-Xylol | |
| Ir(L517)₃ | L517 | 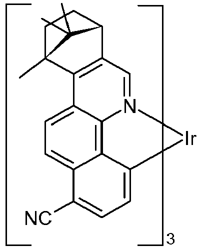 | C | 30 |
| | | | --- | |
| | | | 280°C | |
| | | | 24 h | |
| | | | Ethylacetat | |
| | | | o-Xylol | |
| Ir(L518)₃ | L518 | 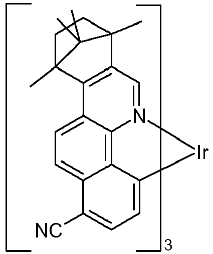 | C | 24 |
| | | | --- | |
| | | | 280°C | |
| | | | 24 h | |
| | | | Ethylacetat | |
| | | | o-Xylol | |
| Ir(L519)₃ * | L519 | 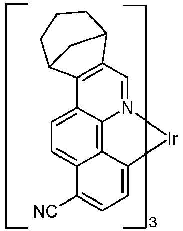 | C | 27 |
| | | | --- | |
| | | | 280°C | |
| | | | 24 h | |
| | | | Ethylacetat | |
| | | | o-Xylol | |
| Ir(L520)₃ | L520 | 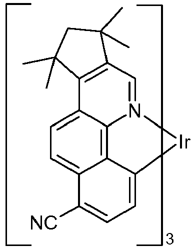 | C | 26 |
| | | | --- | |
| | | | 280°C | |
| | | | 24 h | |
| | | | Ethylacetat | |
| | | | o-Xylol | |
| Ir(L521)₃ * | L521 | 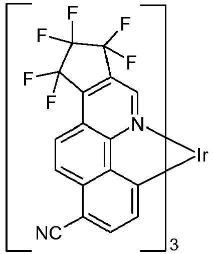 | C | 12 |
| | | | --- | |
| | | | 280°C | |
| | | | 36 h | |
| | | | Ethylacetat | |
| | | | o-Xylol | |
| Ir(L522)₃ * | L522 | 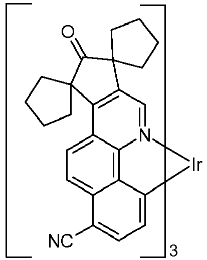 | C | 11 |
| | | | --- | |
| | | | 300°C | |
| | | | 36 h | |
| | | | Ethylacetat | |
| | | | o-Xylol | |
| Ir(L523)₃ * | L523 | 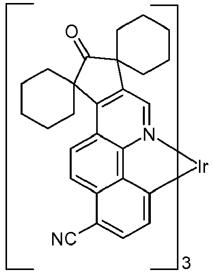 | C | 8 |
| | | | --- | |
| | | | 320°C | |
| | | | 36 h | |
| | | | Ethylacetat | |
| | | | o-Xylol | |
| Ir(L524)₃ * | L524 | 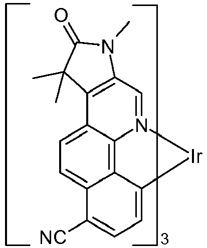 | C | 23 |
| | | | --- | |
| | | | 280°C | |
| | | | 24 h | |
| | | | Ethylacetat | |
| | | | o-Xylol | |
| Ir(L525)₃ | L525 | 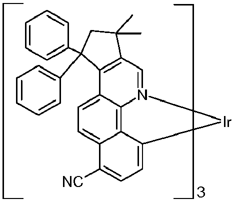 | C | 11 |
| | | | --- | |
| | | | 300°C 24 h | |
| | | | Ethylacetat | |
| | | | o-Xylol | |
| Ir(L526)₃ | L526 | 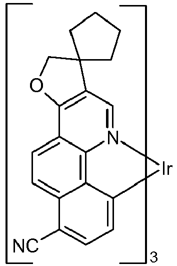 | C | 22 |
| | | | --- | |
| | | | 300°C 24 h | |
| | | | Ethylacetat | |
| | | | o-Xylol | |
| Ir(L527)₃ | L527 | 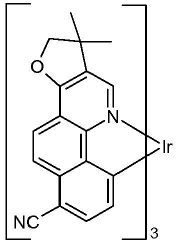 | C | 27 |
| | | | --- | |
| | | | 280°C | |
| | | | 24 h | |
| | | | Ethylacetat | |
| | | | o-Xylol | |
| Ir(L528)₃ * | L528 | 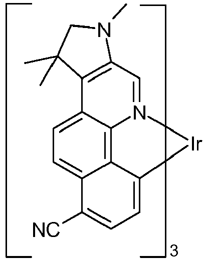 | C | 30 |
| | | | --- | |
| | | | 280°C | |
| | | | 24 h | |
| | | | Ethylacetat | |
| | | | o-Xylol | |
| Ir(L529)₃ * | L529 | 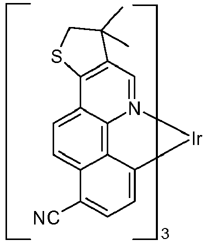 | C | 26 |
| | | | --- | |
| | | | 280°C | |
| | | | 24 h | |
| | | | Ethylacetat | |
| | | | o-Xylol | |
| Ir(L530)₃ | L530 | 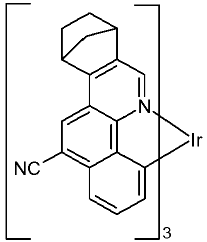 | A | 22 |
| | | | --- | |
| | | | 280°C | |
| | | | 24 h | |
| | | | Ethylacetat | |
| | | | o-Xylol | |
| Ir(L531)₃ | L531 | 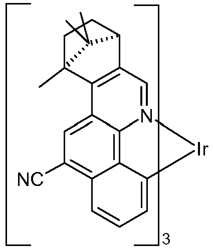 | A | 27 |
| | | | --- | |
| | | | 280°C | |
| | | | 24 h | |
| | | | Ethylacetat | |
| | | | o-Xylol | |
| Ir(L532)₃ | L532 | 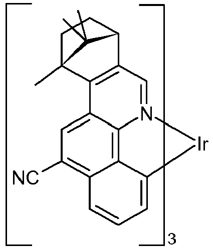 | A | 29 |
| | | | --- | |
| | | | 280°C | |
| | | | 24 h | |
| | | | Ethylacetat | |
| | | | o-Xylol | |
| Ir(L533)₃ | L533 | 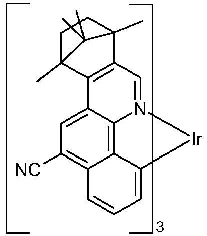 | A | 32 |
| | | | --- | |
| | | | 280°C | |
| | | | 24 h | |
| | | | Ethylacetat | |
| | | | o-Xylol | |
| Ir(L534)₃ * | L534 | 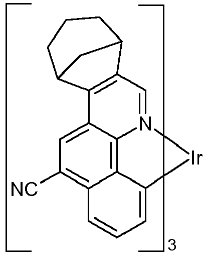 | A | 30 |
| | | | --- | |
| | | | 280°C | |
| | | | 24 h | |
| | | | Ethylacetat | |
| | | | o-Xylol | |
| Ir(L535)₃ | L535 | 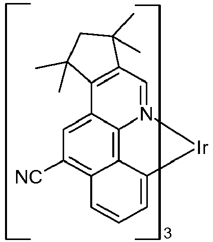 | A | 27 |
| | | | --- | |
| | | | 260°C | |
| | | | 24 h | |
| | | | Ethylacetat | |
| | | | o-Xylol | |
| Ir(L536)₃ * | L536 | 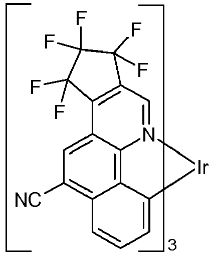 | C | 15 |
| | | | --- | |
| | | | 300°C | |
| | | | 36 h | |
| | | | Ethylacetat | |
| | | | o-Xylol | |
| Ir(L537)₃ * | L537 | 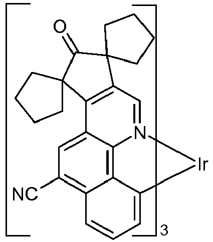 | B | 12 |
| | | | --- | |
| | | | 300°C | |
| | | | 36 h | |
| | | | Ethylacetat | |
| | | | o-Xylol | |
| Ir(L538)₃ * | L538 | 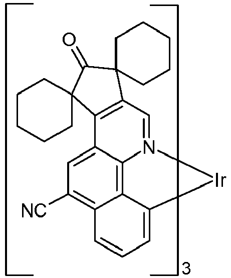 | B | 14 |
| | | | --- | |
| | | | 300°C | |
| | | | 36 h | |
| | | | Ethylacetat | |
| | | | o-Xylol | |
| Ir(L539)₃ * | L539 | 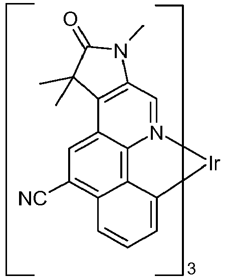 | C | 27 |
| | | | --- | |
| | | | 260°C | |
| | | | 24 h | |
| | | | Ethylacetat | |
| | | | o-Xylol | |
| Ir(L540)₃ | L540 | 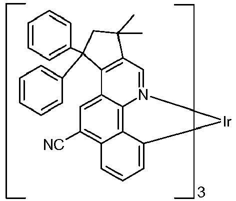 | B | 14 |
| | | | --- | |
| | | | 300°C 24 h | |
| | | | Ethylacetat | |
| | | | o-Xylol | |
| Ir(L541)₃ | L541 | 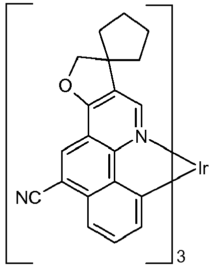 | C | 22 |
| | | | --- | |
| | | | 280°C | |
| | | | 24 h | |
| | | | Ethylacetat | |
| | | | o-Xylol | |
| Ir(L542)₃ | L542 | 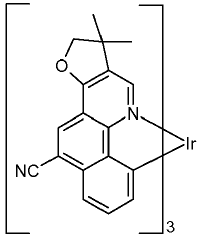 | C | 29 |
| | | | --- | |
| | | | 280°C | |
| | | | 24 h | |
| | | | Ethylacetat | |
| | | | o-Xylol | |
| Ir(L543)₃ * | L543 | 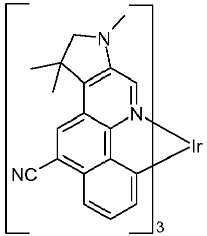 | C | 32 |
| | | | --- | |
| | | | 280°C | |
| | | | 24 h | |
| | | | Ethylacetat | |
| | | | o-Xylol | |
| Ir(L544)₃ * | L544 | 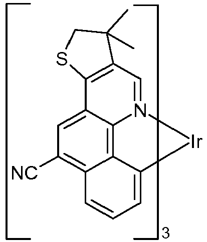 | C | 34 |
| | | | --- | |
| | | | 280°C | |
| | | | 24 h | |
| | | | Ethylacetat | |
| | | | o-Xylol | |
| Ir(L545)₃ | L545 | 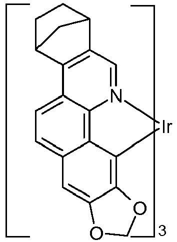 | A | 28 |
| | | | --- | |
| | | | 280°C | |
| | | | 24 h | |
| | | | Ethanol | |
| | | | o-Xylol | |
| Ir(L546)₃ | L546 | 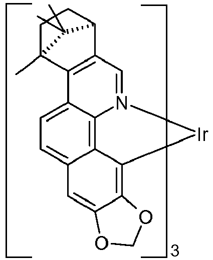 | A | 26 |
| | | | --- | |
| | | | 280°C | |
| | | | 24 h | |
| | | | Ethanol | |
| | | | o-Xylol | |
| Ir(L547)₃ | L547 | 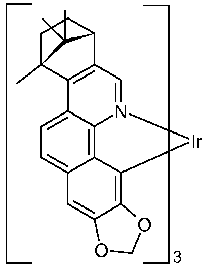 | A | 27 |
| | | | --- | |
| | | | 280°C | |
| | | | 24 h | |
| | | | Ethanol | |
| | | | o-Xylol | |
| Ir(L548)₃ | L548 | 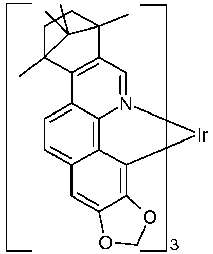 | A | 24 |
| | | | --- | |
| | | | 280°C | |
| | | | 24 h | |
| | | | Ethanol | |
| | | | o-Xylol | |
| Ir(L549)₃ | L549 | 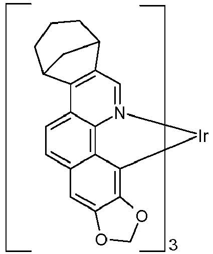 | A | 26 |
| | | | --- | |
| | | | 280°C | |
| | | | 24 h | |
| | | | Ethanol | |
| | | | o-Xylol | |
| Ir(L550)₃ | L550 | 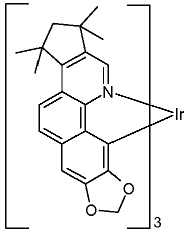 | C | 26 |
| | | | --- | |
| | | | 280°C | |
| | | | 24 h | |
| | | | Ethanol | |
| | | | o-Xylol | |
| Ir(L551)₃ * | L551 | 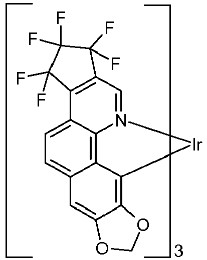 | C | 12 |
| | | | --- | |
| | | | 300°C | |
| | | | 24 h | |
| | | | Ethanol | |
| | | | o-Xylol | |
| Ir(L552)₃ * | L552 | 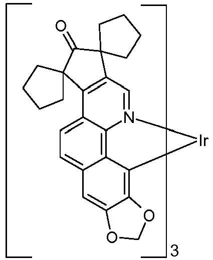 | B | 13 |
| | | | --- | |
| | | | 320°C | |
| | | | 36 h | |
| | | | Ethanol | |
| | | | o-Xylol | |
| Ir(L553)₃ * | L553 | 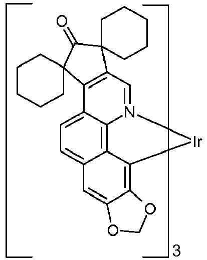 | B | 15 |
| | | | --- | |
| | | | 320°C | |
| | | | 36 h | |
| | | | Ethanol | |
| | | | o-Xylol | |
| Ir(L554)₃ * | L554 | 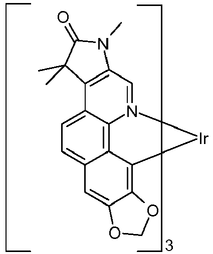 | C | 18 |
| | | | --- | |
| | | | 260°C | |
| | | | 24 h | |
| | | | Ethylacetat | |
| | | | o-Xylol | |
| Ir(L555)₃ | L555 | 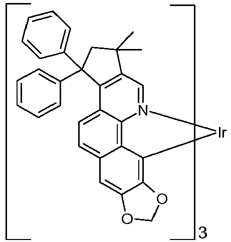 | C | 14 |
| | | | --- | |
| | | | 300°C | |
| | | | 24 h | |
| | | | Ethanol | |
| | | | o-Xylol | |
| Ir(L556)₃ | L556 | 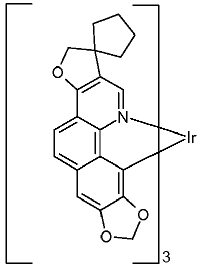 | A | 18 |
| | | | --- | |
| | | | 280°C | |
| | | | 36 h | |
| | | | Ethanol | |
| | | | o-Xylol | |
| Ir(L557)₃ | L557 | 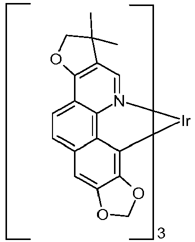 | A | 12 |
| | | | --- | |
| | | | 280°C | |
| | | | 24 h | |
| | | | Ethanol | |
| | | | o-Xylol | |
| Ir(L558)₃ * | L558 | 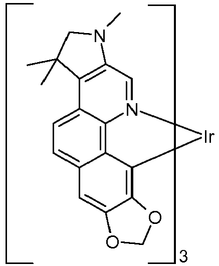 | A | 12 |
| | | | --- | |
| | | | 280°C | |
| | | | 24 h | |
| | | | Ethanol | |
| | | | o-Xylol | |
| Ir(L559)₃ * | L559 | 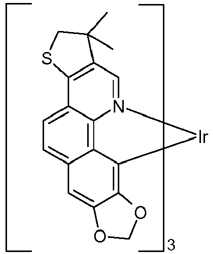 | A | 11 |
| | | | --- | |
| | | | 280°C | |
| | | | 24 h | |
| | | | Ethanol | |
| | | | o-Xylol | |
| Ir(L560)₃ | L560 | 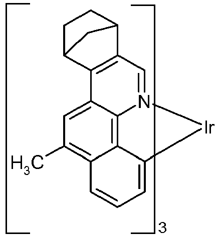 | B | 33 |
| | | | --- | |
| | | | 300°C 24 h | |
| | | | Ethanol | |
| | | | o-Xylol | |
| Ir(L561)₃ | L561 | 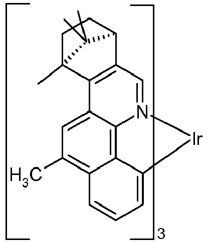 | B | 30 |
| | | | --- | |
| | | | 300°C 24 h | |
| | | | Ethanol | |
| | | | o-Xylol | |
| Ir(L562)₃ | L562 | 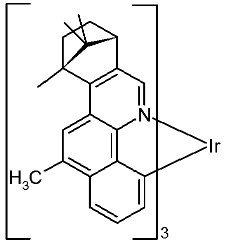 | B | 32 |
| | | | --- | |
| | | | 300°C 24 h | |
| | | | Ethanol | |
| | | | o-Xylol | |
| Ir(L563)₃ | L563 | 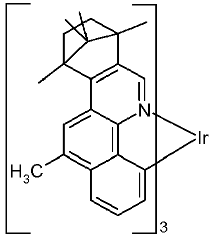 | B | 29 |
| | | | --- | |
| | | | 300°C 24 h | |
| | | | Ethanol | |
| | | | o-Xylol | |
| Ir(L564)₃ * | L564 | 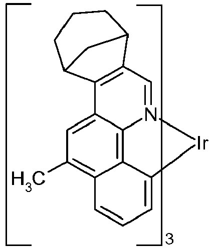 | B | 34 |
| | | | --- | |
| | | | 300°C 24 h | |
| | | | Ethanol | |
| | | | o-Xylol | |
| Ir(L565)₃ | L565 | 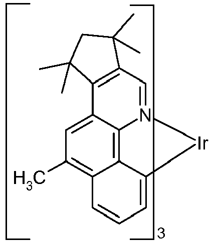 | A | 37 |
| | | | --- | |
| | | | 280°C | |
| | | | 36 h | |
| | | | Ethanol | |
| | | | o-Xylol | |
| Ir(L566)₃ * | L566 | 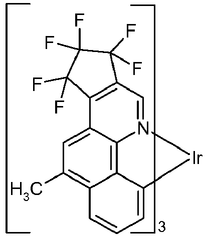 | C | 18 |
| | | | --- | |
| | | | 300°C | |
| | | | 36 h | |
| | | | Ethanol | |
| | | | o-Xylol | |
| Ir(L567)₃ * | L567 | 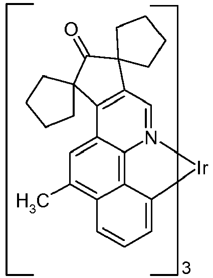 | C | 15 |
| | | | --- | |
| | | | 320°C | |
| | | | 48 h | |
| | | | Ethanol | |
| | | | o-Xylol | |
| Ir(L568)₃ * | L568 | 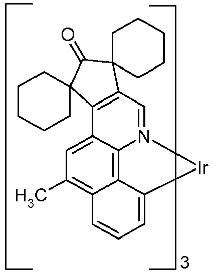 | C | 12 |
| | | | --- | |
| | | | 320°C | |
| | | | 48 h | |
| | | | Ethanol | |
| | | | o-Xylol | |
| Ir(L569)₃ * | L569 | 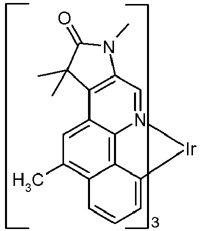 | A | 19 |
| | | | --- | |
| | | | 280°C | |
| | | | 24 h | |
| | | | Ethanol | |
| | | | o-Xylol | |
| Ir(L570)₃ | L570 | 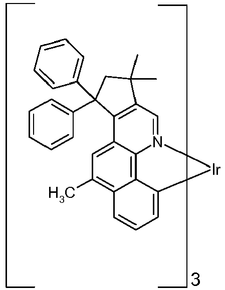 | C | 14 |
| | | | --- | |
| | | | 340°C | |
| | | | 36 h | |
| | | | Ethanol | |
| | | | o-Xylol | |
| Ir(L571)₃ | L571 | 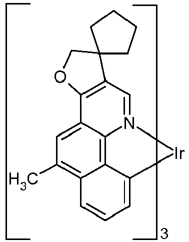 | A | 33 |
| | | | --- | |
| | | | 260°C | |
| | | | 48 h | |
| | | | Ethylacetat | |
| | | | o-Xylol | |
| Ir(L572)₃ | L572 | 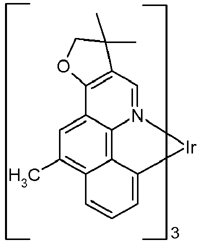 | A | 35 |
| | | | --- | |
| | | | 260°C | |
| | | | 24 h | |
| | | | Ethylacetat | |
| | | | o-Xylol | |
| Ir(L573)₃ * | L573 | 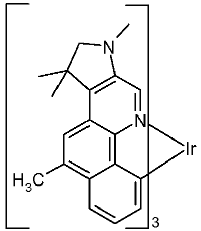 | A | 38 |
| | | | --- | |
| | | | 260°C | |
| | | | 24 h | |
| | | | Ethylacetat | |
| | | | o-Xylol | |
| Ir(L574)₃ * | L574 | 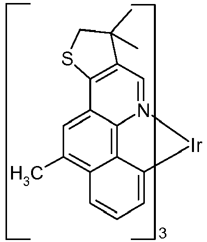 | A | 36 |
| | | | --- | |
| | | | 260°C | |
| | | | 24 h | |
| | | | Ethylacetat | |
| | | | o-Xylol | |
| Ir(L575)₃ | L575 | 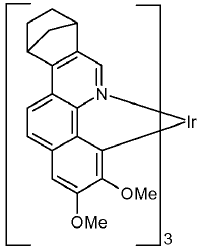 | C | 19 |
| | | | --- | |
| | | | 280°C | |
| | | | 24 h | |
| | | | Ethanol | |
| | | | o-Xylol | |
| Ir(L576)₃ | L576 | 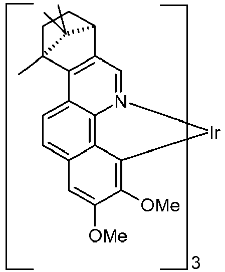 | C | 21 |
| | | | --- | |
| | | | 280°C | |
| | | | 24 h | |
| | | | Ethanol | |
| | | | o-Xylol | |
| Ir(L577)₃ | L577 | 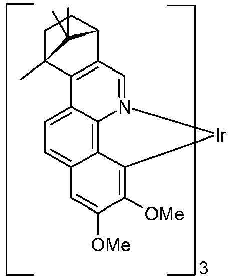 | C | 20 |
| | | | --- | |
| | | | 280°C | |
| | | | 24 h | |
| | | | Ethanol | |
| | | | o-Xylol | |
| Ir(L578)₃ | L578 | 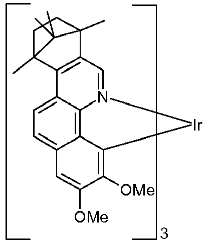 | C | 21 |
| | | | --- | |
| | | | 280°C | |
| | | | 24 h | |
| | | | Ethanol | |
| | | | o-Xylol | |
| Ir(L579)₃ * | L579 | 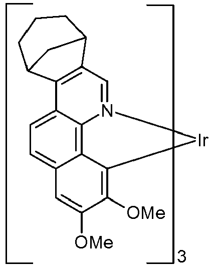 | C | 16 |
| | | | --- | |
| | | | 280°C | |
| | | | 24 h | |
| | | | Ethanol | |
| | | | o-Xylol | |
| Ir(L580)₃ | L580 | 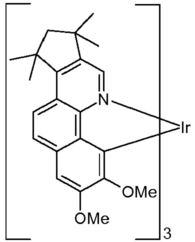 | B | 21 |
| | | | --- | |
| | | | 280°C | |
| | | | 24 h | |
| | | | Ethanol | |
| | | | o-Xylol | |
| Ir(L581)₃ * | L581 | 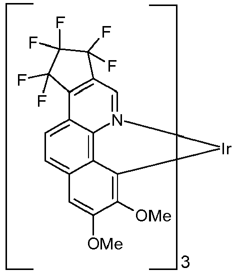 | B | 8 |
| | | | --- | |
| | | | 300°C | |
| | | | 48 h | |
| | | | Ethylacetat | |
| | | | o-Xylol | |
| Ir(L582)₃ * | L582 | 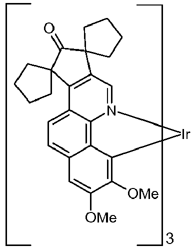 | C | 6 |
| | | | --- | |
| | | | 320°C | |
| | | | 48 h | |
| | | | Ethanol | |
| | | | o-Xylol | |
| Ir(L583)₃ * | L583 | 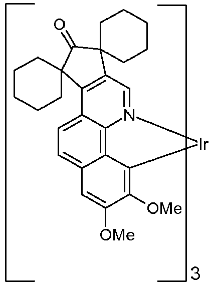 | C | 5 |
| | | | --- | |
| | | | 320°C | |
| | | | 48 h | |
| | | | Ethanol | |
| | | | o-Xylol | |
| Ir(L584)₃ * | L584 | 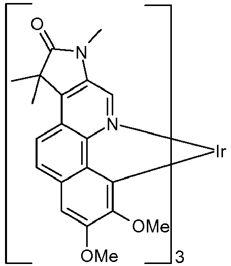 | C | 9 |
| | | | --- | |
| | | | 280°C | |
| | | | 24 h | |
| | | | Ethanol | |
| | | | o-Xylol | |
| Ir(L585)₃ | L585 | 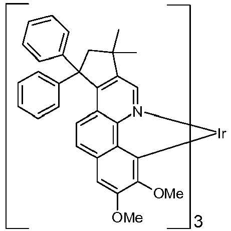 | C | 11 |
| | | | --- | |
| | | | 320°C | |
| | | | 24 h | |
| | | | Ethanol | |
| | | | o-Xylol | |
| Ir(L586)₃ | L586 | 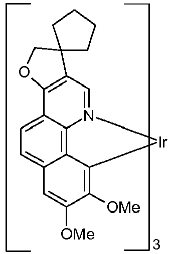 | A | 9 |
| | | | --- | |
| | | | 260°C | |
| | | | 24 h | |
| | | | Ethanol | |
| | | | o-Xylol | |
| Ir(L587)₃ | L587 | 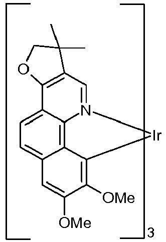 | A | 12 |
| | | | --- | |
| | | | 260°C | |
| | | | 24 h | |
| | | | Ethanol | |
| | | | o-Xylol | |
| Ir(L588)₃ * | L588 | 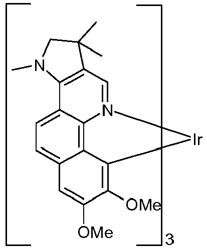 | A | 11 |
| | | | --- | |
| | | | 260°C | |
| | | | 24 h | |
| | | | Ethanol | |
| | | | o-Xylol | |
| Ir(L589)₃ * | L589 | 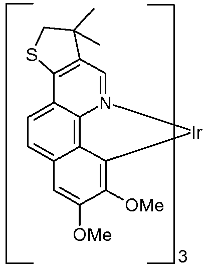 | A | 12 |
| | | | --- | |
| | | | 260°C | |
| | | | 24 h | |
| | | | Ethanol | |
| | | | o-Xylol | |
| Ir(L1000)₃ | L1000 | 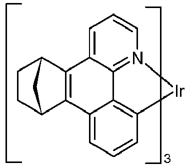 | C | 27 |
| | | | --- | |
| | | | 280°C | |
| | | | 24 h | |
| | | | Aceton | |
| | | | o-Xylol | |
| Ir(L1001)₃ | L1001 | 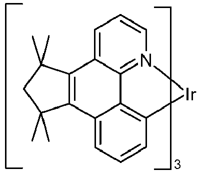 | C | 25 |
| | | | --- | |
| | | | 280°C | |
| | | | 24 h | |
| | | | Aceton | |
| | | | o-Xylol | |
| Ir(L1002)₃ | L1002 | 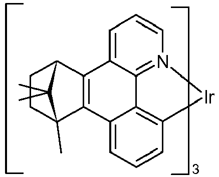 | C | 29 |
| | | | --- | |
| | | | 280°C | |
| | | | 24 h | |
| | | | Aceton | |
| | | | o-Xylol | |
| Ir(L1500)₃ | L1500 | 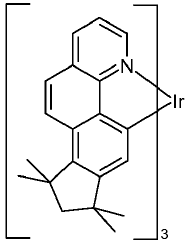 | A | 38 |
| | | | - | |
| | | | 260°C | |
| | | | 24h | |
| | | | Ethylacetat | |
| | | | Mesitylen | |
| Ir(L1501)₃ | L1501 | 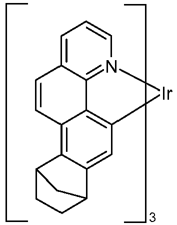 | A | 35 |
| | | | - | |
| | | | 280°C | |
| | | | 24h | |
| | | | Ethylacetat | |
| | | | Mesitylen | |
| Ir(L1502)₃ | L1502 | 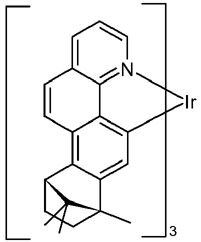 | A | 36 |
| | | | - | |
| | | | 280°C | |
| | | | 24h | |
| | | | Ethylacetat | |
| | | | Mesitylen | |
| Ir(L1503)₃ | L1503 | 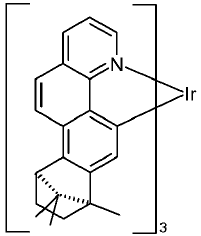 | A | 32 |
| | | | - | |
| | | | 280°C | |
| | | | 24 h | |
| | | | Ethylacetat | |
| | | | Mesitylen | |
| Ir(L1504)₃ | L1504 | 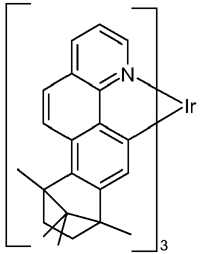 | A | 34 |
| | | | - | |
| | | | 280°C | |
| | | | 24 h | |
| | | | Ethylacetat | |
| | | | Mesitylen | |
| Ir(L 1505)₃ * | L1505 | 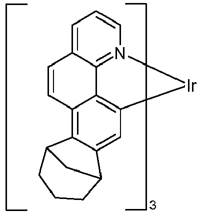 | A | 31 |
| | | | - | |
| | | | 280°C | |
| | | | 24 h | |
| | | | Ethylacetat | |
| | | | Mesitylen | |
| Ir(L 1506)₃ * | L1506 | 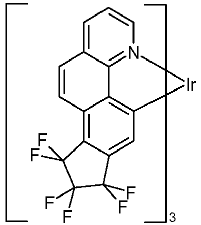 | A | 17 |
| | | | - | |
| | | | 300°C | |
| | | | 48 h | |
| | | | Ethylacetat | |
| | | | Mesitylen | |
| Ir(L1507)₃ | L1507 | 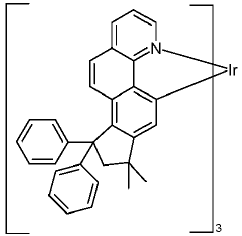 | A | 12 |
| | | | - | |
| | | | 320°C | |
| | | | 36 h | |
| | | | Ethylacetat | |
| | | | Mesitylen | |
| Ir(L1508)₃ | L1508 | 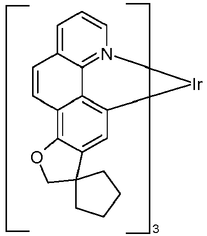 | A | 29 |
| | | | - | |
| | | | 260°C | |
| | | | 24 h | |
| | | | Ethanol | |
| | | | Mesitylen | |
| Ir(L1509)₃ | L1509 | 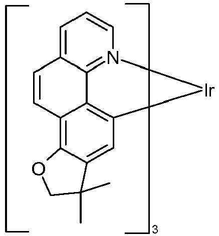 | A | 33 |
| | | | - | |
| | | | 260°C 24 h | |
| | | | Ethanol | |
| | | | Mesitylen | |
| Ir(L1510)₃ * | L1510 | 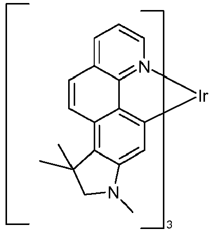 | A | 31 |
| | | | - | |
| | | | 260°C | |
| | | | 24 h | |
| | | | Ethanol | |
| | | | Mesitylen | |
| Ir(L 1512)₃ * | L1511 | 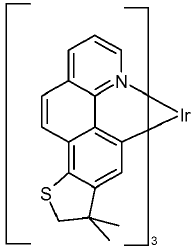 | A | 36 |
| | | | - | |
| | | | 260°C | |
| | | | 24 h | |
| | | | Ethanol | |
| | | | Mesitylen | |

### 2) Heteroleptische Iridium-Komplexe:

### Variante A:

### Schritt 1:

Ein Gemisch aus 10 mmol Natrium-bisacetylacetonato-dichloro-iridat(III) [770720-50-8] und 24 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Nach Erkalten - ACHTUNG: die Ampullen stehen meist unter Druck! - wird die Ampulle geöffnet, der Sinterkuchen wird mit 100 g Glaskugeln (3 mm Durchmesser) in 100 ml des angegebenen Suspensionsmittels (das Suspensionsmittel wird so gewählt, dass der Ligand gut, das Chloro-Dimer der Formel [Ir(L)₂Cl]₂ jedoch schlecht darin löslich ist; typische Suspensionsmittel sind DCM, Aceton, Ethylacetat, Toluol, etc.) 3 h gerührt und dabei mechanisch aufgeschlossen. Man dekantiert die feine Suspension von den Glaskugeln ab, saugt den Feststoff (Ir(L)₂Cl]₂, das noch ca. 2 eq NaCl enthält, nachfolgend das rohe Chloro-Dimer genannt) ab und trocknet diesen im Vakuum.

### Schritt 2:

Das so erhaltene rohe Chloro-Dimer der Formel [Ir(L)₂Cl]₂ wird in einem Gemisch aus 75 ml 2-Ethoxyethanol und 25 ml Wasser suspendiert, mit 13 mmol des Coliganden CL bzw. der Coliganden-Verbindung CL und 15 mmol Natriumcarbonat versetzt. Nach 20 h unter Rückfluss gibt man weitere 75 ml Wasser tropfenweise zu, saugt nach Erkalten vom Feststoff ab, wäscht diesen dreimal mit je 50 ml Wasser und dreimal mit je 50 ml Methanol und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit dem angegebenen Extraktionsmittel (Vorlagemenge ca. 500 ml, das Extraktionsmittel wird so gewählt, dass der Komplex darin in der Hitze gut und in der Kälte schlecht löslich ist; geeignete Extraktionsmittel sind Kohlenwasserstoffe wie Toluol, Xylole, Mesitylen, Naphthalin, o-Dichlorbenzol, Tetrahydrofuran, Dichlormethan, 1,2-Dichlorethan, 1,1,2,2-Tetrachlorethan, Chloroform, Tetrachlorkohlenstoff) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metallkomplexes mittels NMR und/oder HPLC bestimmt. Liegt die Reinheit unter 99.5 % wird der Heißextraktionsschritt wiederholt. Ist eine Reinheit von 99.5 - 99.9 % erreicht, wird der Metallkomplex getempert oder sublimiert. Neben dem Heißextraktionsverfahren zur Reinigung kann die Reinigung auch chromatographisch an Kieselgel oder Alox erfolgen. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird.

Die mit * markierten Komplexe sind nicht Gegenstand der Ansprüche.

| **Bsp.** | **Ligand L** | **Co-Ligand CL** | **Ir-Komplex** | **Ausbeute** |
|---|---|---|---|---|
| | | | **Schritt 1: Reaktionstemp. / Reaktionszeit / Suspensionsmittel** | |
| | | | **Schritt 2: Extraktionsmittel** | |
| Ir(L3)₂(CL1) | L3 | 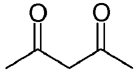 | 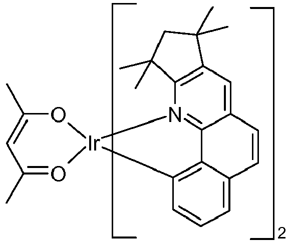 | 28 % |
| | | 123-54-6 CL1 | 260 °C / 60 h / Aceton | |
| | | | Xylol | |
| Ir(L4)₂(CL1) * | L4 | CL1 | 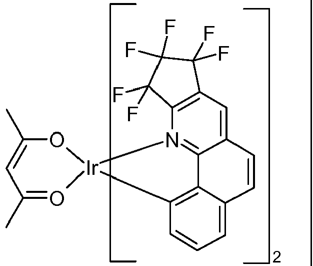 | 25 % |
| | | | 280 °C / 80 h / Aceton | |
| | | | Xylol | |
| Ir(L5)₂(CL1) * | L5 | CL1 | 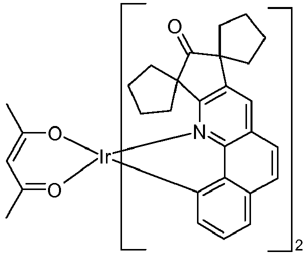 | 16 % |
| | | | 280 °C / 60 h / Ethylacetat | |
| | | | Xylol | |
| Ir(L6)₂(CL1) * | L6 | CL1 | 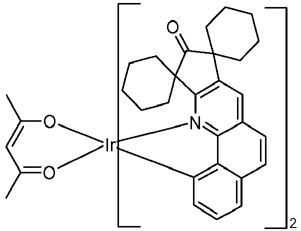 | 15 % |
| | | | 260 °C / 80 h / Aceton | |
| | | | Xylol | |
| Ir(L7)₂(CL1) | L7 | CL1 | 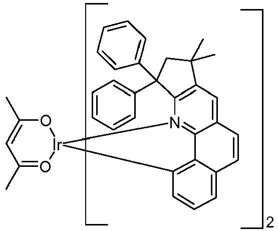 | 16 % |
| | | | 300 °C / 80 h / Aceton | |
| | | | Xylol | |
| Ir(L10)₂(CL1) * | L10 | CL1 | 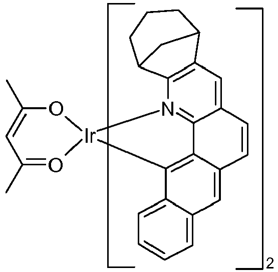 | 15 % |
| | | | 280 °C / 80 h / Ethylacetat | |
| | | | Xylol | |
| Ir(L11)₂(CL1) | L11 | CL1 | 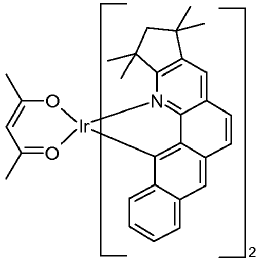 | 16 % |
| | | | 260 °C / 80 h / Ethylacetat | |
| | | | Xylol | |
| Ir(L12)₂(CL1) * | L12 | CL1 | 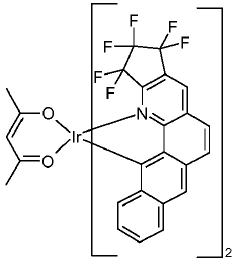 | 25 % |
| | | | 280 °C / 60 h / Aceton | |
| | | | Xylol | |
| Ir(L13)₂(CL1) * | L13 | CL1 | 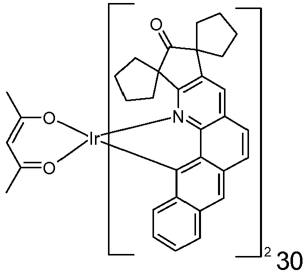 | 16 % |
| | | | 0 °C / 90 h / Aceton | |
| | | | Xylol | |
| Ir(L14)₂(CL2) * | L14 | 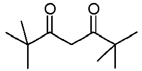 | 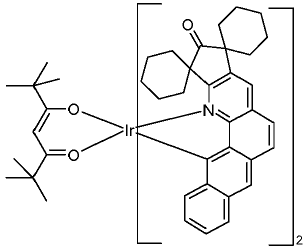 | 19 % |
| | | 1118-71-4 CL2 | 300 °C / 90 h / Ethylacetat | |
| | | | Xylol | |
| Ir(L15)₂(CL2) | L15 | CL2 | 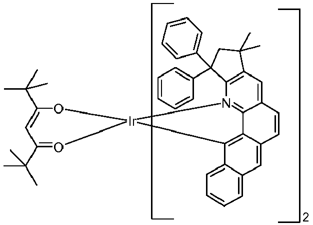 | 14 % |
| | | | 320 °C / 80 h / Aceton | |
| | | | Xylol | |
| Ir(L26)₂(CL2) * | L26 | CL2 | 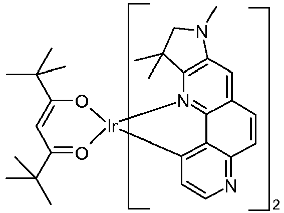 | 30 % |
| | | | 280 °C / 60 h / Aceton | |
| | | | Xylol | |
| Ir(L33)₂(CL2) * | L33 | CL2 | 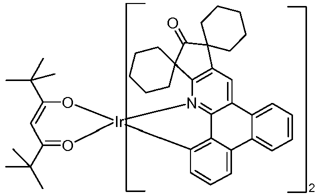 | 14 % |
| | | | 280 °C / 60 h / Aceton | |
| | | | Xylol | |
| Ir(L500)₂(CL2) | L500 | CL2 | 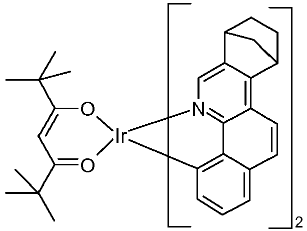 | 60 % |
| | | | 280 °C / 80 h / Aceton | |
| | | | Xylol | |
| Ir(L503)₂(CL2) | L503 | CL2 | 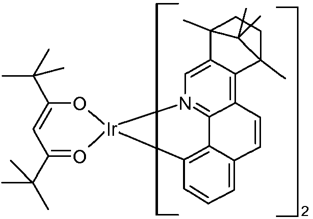 | 54 % |
| | | | 280 °C / 60 h / Aceton | |
| | | | Xylol | |
| Ir(L505)₂(CL2) | L505 | CL2 | 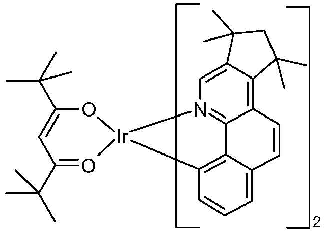 | 60 % |
| | | | 280 °C / 60 h / Aceton | |
| | | | Xylol | |
| Ir(L518)₂(CL3) | L518 | 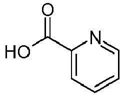 | 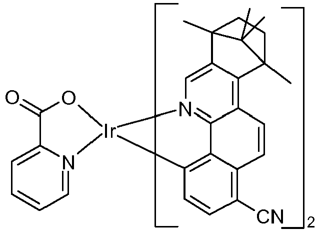 | 27 % |
| | | 98-98-6 CL3 | 280 °C / 80 h / Aceton | |
| | | | Xylol | |
| Ir(L523)₂(CL3) * | L523 | CL3 | 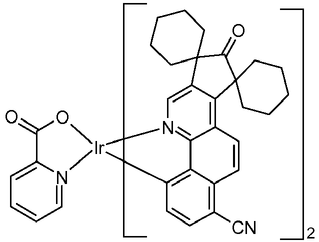 | 22 % |
| | | | 280 °C / 80 h / Aceton | |
| | | | Xylol | |
| Ir(L548)₂(CL3) | L548 | CL3 | 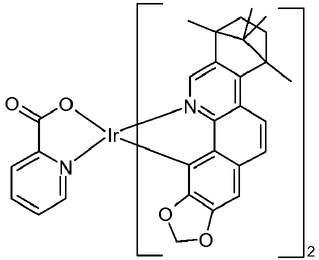 | 27 % |
| | | | 280 °C / 80 h / Aceton | |
| | | | Xylol | |
| Ir(L553)₂(CL3) * | L553 | CL3 | 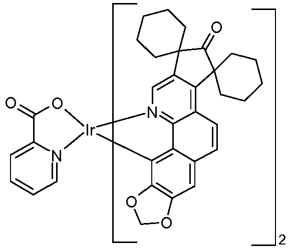 | 24 % |
| | | | 280 °C / 80 h / Aceton | |
| | | | Xylol | |
| Ir(L556)₂(CL3) | L556 | CL3 | 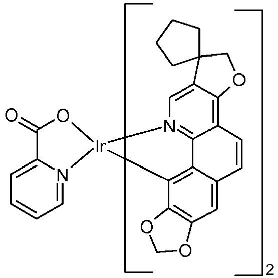 | 29 % |
| | | | 280 °C / 80 h / Aceton | |
| | | | Xylol | |
| Ir(L565)₂(CL4) | L565 | 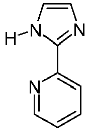 | 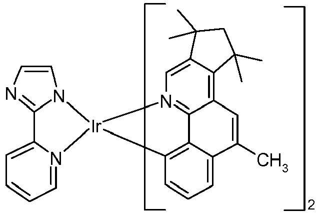 | 44 % |
| | | 18653-75-3 CL4 | 260 °C / 60 h / Aceton | |
| | | | Xylol | |
| Ir(L568)₂(CL4) * | L568 | CL4 | 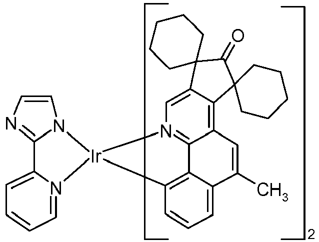 | 44 % |
| | | | 260 °C / 80 h / Aceton | |
| | | | Xylol | |
| Ir(L586)₂(CL5) | L586 | 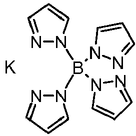 | 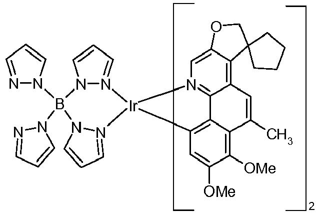 | 50 % |
| | | 14782-58-2 CL5 | 260 °C / 80 h / Aceton | |
| | | | Xylol | |
| Ir(L1508)₂(CL7 ) | L1508 | 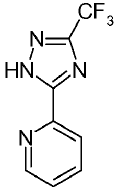 | 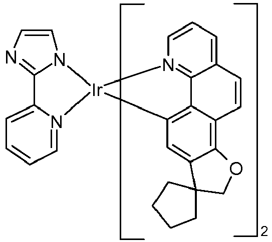 | 47 % |
| | | 219508-27-7 CL6 | 280 °C / 80 h / Aceton | |
| | | | Xylol | |

### Variante B:

### Schritt 1:

Siehe Variante A, Schritt 1.

### Schritt 2:

Das rohe Chloro-Dimer der Formel [Ir(L)₂Cl]₂ wird in 200 ml THF suspendiert, die Suspension wird mit 20 mmol des Coliganden CL, 20 mmol Silber(I)trifluoracetat und 30 mmol Kaliumcarbonat versetzt und 24 h unter Rückfluss erhitzt. Nach Erkalten wird das THF im Vakuum entfernt. Der Rückstand wird in 200 ml eines Gemischs aus Ethanol und konz. Ammoniak-Lösung (1:1, vv) aufgenommen. Die Suspension wird 1 h bei Raumtemperatur gerührt, der Feststoff wird abgesaugt, zweimal mit je 50 ml eines Gemischs aus Ethanol und konz. Ammoniak-Lösung (1:1, vv) und zweimal mit je 50 ml Ethanol gewaschen und dann im Vakuum getrocknet. Heißextraktion und Sublimation wie in Variante A.

| **Bsp.** | **Ligand L** | **Co-Ligand CL** | **Ir-Komplex** | **Ausbeute** |
|---|---|---|---|---|
| | | | **Schritt 1: Reaktionstemp. / Reaktionszeit / Suspensionsmittel** | |
| | | | **Schritt 2: Extraktionsmittel** | |
| Ir(L3)₂(CL7) | L3 | 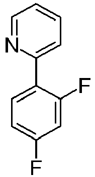 | 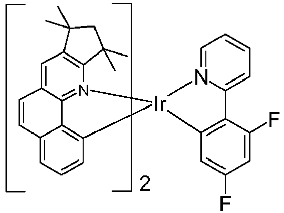 | 39 % |
| | | 391604-55-0 CL7 | wie Bsp. Ir(L98)₂(CL2) | |
| Ir(L503)₂(CL8) | L503 | 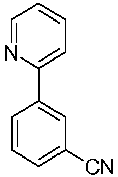 | 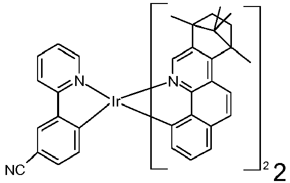 | 31 % |
| | | 4350-51-0 CL8 | 90 °C / 48 h / Aceton Xylol | |
| Ir(L565)₂(CL8) | L565 | 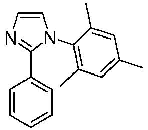 | 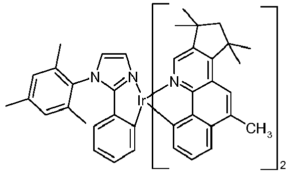 | 39 % |
| | | 1093072-00-4 CL9 | 290 °C / 60 h / Aceton Xylol | |
| Ir(L1508)₂(CL10) | L1508 | 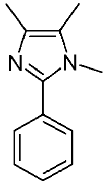 | 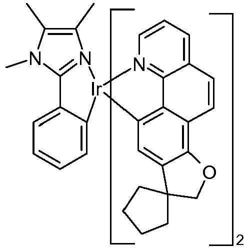 | 38 % |
| | | 152536-39-5 CL10 | 300 °C / 80 h / Aceton Xylol | |

### Variante C:

### Schritt 1:

Siehe Variante A, Schritt 1.

### Schritt 2:

Das rohe Chloro-Dimer der Formel [Ir(L)₂Cl]₂ wird in 1000 ml Dichlormethan und 150 ml Ethanol suspendiert, die Suspension wird mit 20 mmol Silber(I)-trifluormethansulfonat versetzt und 24 h bei Raumtemperatur gerührt. Man saugt vom ausgefallen Feststoff (AgCl) über eine kurzes Celite-Bett ab und engt das Filtrat im Vakuum zur Trockene ein. Der so erhaltene Feststoff wird in 100 ml Ethylenglykol aufgenommen, mit 20 mmol des Coliganden CL versetzt und dann 30 h bei 130 °C gerührt. Nach Erkalten saugt man vom Feststoff ab, wäscht diesen zweimal mit je 50 ml Ethanol und trocknet im Vakuum. Heißextraktion und Sublimation wie in Variante A.

Die mit * markierten Komplexe sind nicht Gegenstand der Ansprüche.

| **Bsp.** | **Ligand L** | **Co-Ligand CL** | **Ir-Komplex** | **Ausbeute** |
|---|---|---|---|---|
| | | | **Schritt 1: Reaktionstemp. / Reaktionszeit / Suspensionsmittel** | |
| | | | **Schritt 2: Extraktionsmittel** | |
| Ir(L586)₂ (CL11) | L586 | 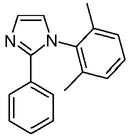 | 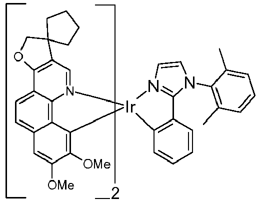 | 46 % |
| | | 914306-48-2 CL11 | 290 °C / 80 h / Aceton Xylol | |
| Ir(L588)₂ (CL11)* | L588 | CL11 | 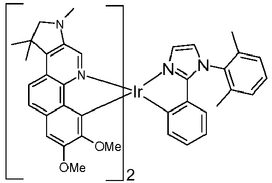 | 39 % |
| | | | 290 °C / 80 h / Aceton Xylol | |
| Ir(L553)₂ (CL12) * | L553 | 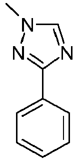 | 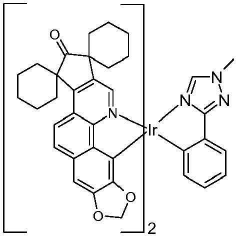 | 44 % |
| | | 39696-58-7 CL12 | 300 °C / 80 h / Aceton Xylol | |

### Variante E:

Ein Gemisch aus 10 mmol des Ir-Komplexes Ir(L)₂(CL1 oder CL2), 20 mmol des Liganden L' und ein glasummantelter Magnetrührkern werden unter Vakuum (10⁻⁵ mbar) in eine 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Weitere Aufarbeitung, Reinigung und Sublimation wie unter 1) Homoleptische tris-faciale Iridium-Komplexe beschrieben.

Die mit * markierten Komplexe sind nicht Gegenstand der Ansprüche.

| **Bsp.** | **Ir-Komplex Ir(L)₂(CL)** | **Ligand L'** | **Ir-Komplex** | **Ausbeute** |
|---|---|---|---|---|
| | | | **Schritt 1: Reaktionstemp. / Reaktionszeit / Suspensionsmittel** | |
| | | | **Schritt 2: Extraktionsmittel** | |
| Ir(L505)₂(L588) | Ir(L505)₂ (CL2) | L588 | 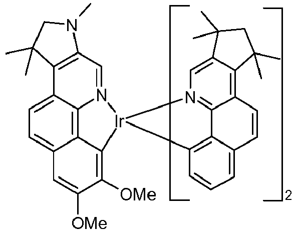 | 39 % |
| | | | 280 °C / 80 h / DCM Mesitylen | |
| Ir(L548)₂(L26) | Ir(L548)₂(CL3) | L26 | 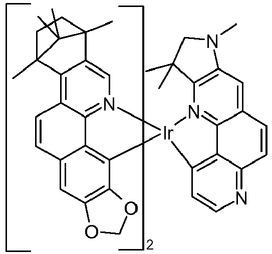 | 43 % |
| | | | 300 °C / 70 h / DCM Mesitylen | |
| Ir(L556)₂(L4) | Ir(L556)₂(CL3) | L4 | 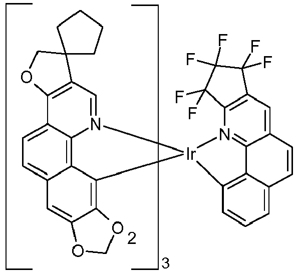 | 44 % |
| | | | 300 °C / 70 h / DCM Mesitylen | |
| Ir(L13)₂(L1508) * | Ir(L13)₂(CL1) | L1508 | 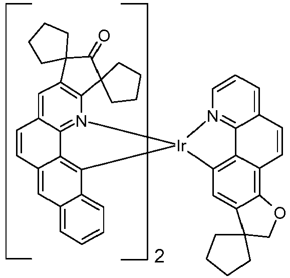 | 36 % |
| | | | 305 °C / 70 h / DCM Mesitylen | |

### E: Derivatisierung der Metallkomplexe

### 1) Halogenierung der Iridium-Komplexe:

Eine Lösung bzw. Suspension von 10 mmol eines Komplexes, der in para-Position zum Iridium A x C-H-Gruppen (mit A = 1, 2 oder 3) trägt, in 3000 ml Dichlormethan wird unter Licht- und Luftausschluss bei 30 °C mit A x 11 mmol N-Halogensuccinimid (Halogen: Cl, Br, I) versetzt und 20 h gerührt. In DCM schlecht lösliche Komplexe können auch in anderen Lösungsmitteln (TCE, THF, DMF, etc.) und bei erhöhter Temperatur umgesetzt werden. Anschließend wird das Lösungsmittel im Vakuum weitgehend entfernt. Der Rückstand wird mit 100 ml MeOH ausgekocht, der Feststoff wird abgesaugt, dreimal mit 30 ml Methanol gewaschen und dann im Vakuum getrocknet.

### Synthese von Ir(L500-Br)₃:

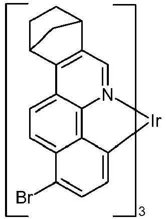

Eine bei 30 °C gerührte Suspension von 11.3 g (10 mmol) Ir(L500)₃ in 3000 ml DCM wird auf ein Mal mit 5.9 g (33 mmol) N-Bromsuccinimid versetzt und 20 h gerührt. Nach Entfernen von ca. 2900 ml des DCMs im Vakuum wird die gelbe Suspension mit 100 ml Methanol versetzt, der Feststoff abgesaugt, dreimal mit ca. 30 ml Methanol gewaschen und dann im Vakuum getrocknet. Ausbeute: 13.8 g (9.5 mmol) 95 %; Reinheit: ca. 99.6 %ig nach NMR.

Analog können folgende Verbindungen dargestellt werden (die mit * markierten Komplexe sind nicht Gegenstand der Ansprüche):

| **Bsp.** | **Komplex** | **Bromierter Komplex** | **Ausbeute** |
|---|---|---|---|
| Ir(L8-Br)₃ * | 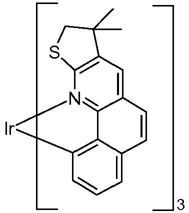 | 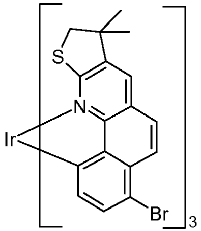 | 93 % |
| | Ir(L8)₃ | Ir(L8-Br)₃ | |
| Ir(L16-Br)₃ | 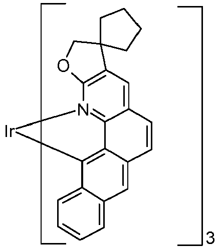 | 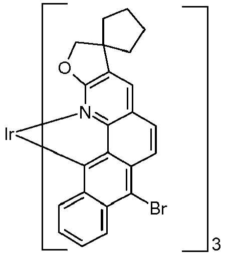 | 95 % |
| | Ir(L16)₃ | Ir(L16-Br)₃ | |
| Ir(L505-Br)₃ | 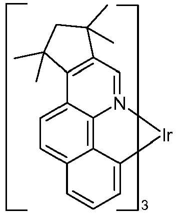 | 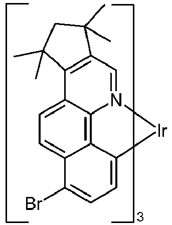 | 97 % |
| | Ir(L505)₃ | Ir(L505-Br)₃ | |

### 2) Suzuki-Kupplung an den Iridium-Komplexen:

### Variante A, zweiphasige Reaktionsmischung:

Eine Suspension von 10 mmol eines bromierten Komplexes, 40 - 80 mmol der Boronsäure bzw. des Boronsäureesters und 80 mmol Trikaliumphosphat in einem Gemisch aus 300 ml Toluol, 100 ml Dioxan und 300 ml Wasser wird mit 0.6 mmol Tri-o-Tolylphosphin und mit 0.1 mmol Palladium(II)acetat versetzt und 16 h unter Rückfluss erhitzt. Nach Erkalten gibt man 500 ml Wasser und 200 ml Toluol zu, trennt die wässrige Phase ab, wäscht die organische Phase dreimal mit 200 ml Wasser, einmal mit 200 ml gesättigter Kochsalzlösung und trocknet über Magnesiumsulfat. Man filtriert über ein Celite-Bett ab, wäscht dieses mit Toluol nach, entfernt das Toluol fast vollständig im Vakuum, gibt 300 ml Ethanol zu, saugt vom ausgefallenen Rohprodukt ab, wäscht dieses dreimal mit je 100 ml EtOH und trocknet im Vakuum. Das Rohprodukt wird mit Toluol zweimal über Kieselgel gesäult. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird.

### Variante B, einphasige Reaktionsmischung:

Eine Suspension von 10 mmol eines bromierten Komplexes, 40 - 80 mmol der Boronsäure bzw. des Boronsäureesters und 60 - 100 mmol der Base (Kaliumfluorid, Trikaliumphosphat, Kaliumcarbonat, Cäsiumcarbonat etc. jeweils wasserfrei) und 100 g Glaskugeln (3 mm Durchmesser) in 100 ml - 500 ml eines aprotischen Lösungsmittels (THF, Dioxan, Xylol, Mesitylen, Dimethylacetamid, NMP, DMSO, etc.) wird mit 0.6 mmol Tri-o-tolylphosphin und mit 0.1 mmol Palladium(II)acetat versetzt und 1 - 24 h unter Rückfluss erhitzt. Alternativ können andere Phosphine wir Tri-tert-butylphosphin, Di-tert-butylphosphin, S-Phos, Xanthphos, etc. eingesetzt werden, wobei bei diesen Phosphinen das bevorzugte Verhältnis Phosphin : Palladium 2:1 bis 1.2 : 1 beträgt. Man entfernt das Lösungsmittel im Vakuum, nimmt das Produkt in einem geeigneten Lösungsmittel (Toluol, Dichlormethan, Ethylacetat, etc.) auf und reinigt wie unter A beschrieben.

### Synthese von Ir(L2000)₃:

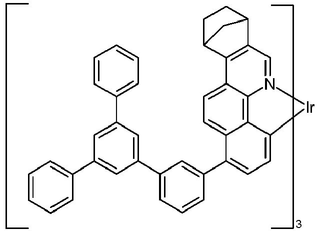

### Variante B:

Einsatz von 11.6 g (10 mmol) Ir(L500-Br)₃ und 14.0 g (40 mmol) Quaterphenylboronsäure [1233200-59-3], Caesiumcarbonat, Tri-ortho-tolylphosphin, NMP, 180 °C, 2 h . Ausbeute: 12.7 g (6.9 mmol) 69 %; Reinheit: ca. 99.8 %ig nach HPLC.

Analog können folgende Verbindungen dargestellt werden (die mit * markierten Komplexe sind nicht Gegenstand der Ansprüche):

| **Bsp.** | **Produkt** | **Ausbeute** |
|---|---|---|
| | **Variante** | |
| Ir(L2001)₃ * | 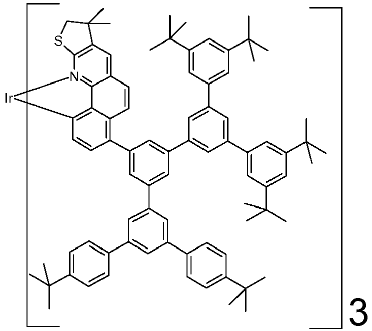 | 57 % |
| | Ir(L8-Br)₃ + [952583-08-3] | |
| | B, wie Ir(L2000)₃ | |
| Ir(L2002)₃ * | 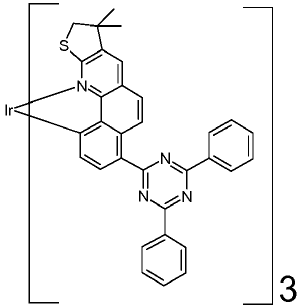 | 55 % |
| | Ir(L8-Br)₃ + [1251825-65-6] | |
| | A | |
| Ir(L2003)₃ | 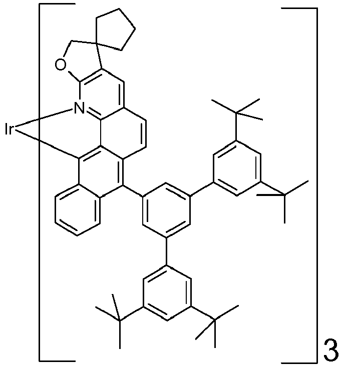 | 46 % |
| | Ir(16-Br)₃ + [1071924-15-6] | |
| | B, wie Ir(L2000)₃ | |
| Ir(L2004)₃ | 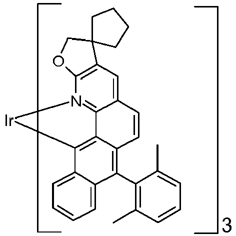 | 52 % |
| | Ir(L16-Br)₃ + [100379-00-8] | |
| | B, wie Ir(L2000)₃, Dioxan statt NMP | |
| Ir(L2005)₃ | 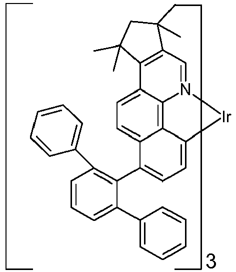 | 23 % |
| | Ir(L505-Br)₃ + [1065663-52-6] | |
| | B, wie Ir(L2000)₃ | |
| Ir(L2006)₃ | 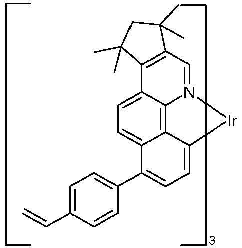 | 25 % |
| | Ir(L505-Br)₃ + [2156-04-9] | |
| | B, wie Ir(L2000)₃, DMAC statt NMP | |

### 3) Buchwald-Kupplung an den Iridium-Komplexen:

Ein Gemisch aus 10 mmol des bromierten Komplexe, 40 mmol des Diarylamins oder Carbazols, 45 mmol Natrium-tert-butylat bei den Aminen bzw. 80 mmol Trikaliumphosphat (wasserfrei) bei Carbazolen, 100 g Glaskugeln (3 mm Durchmesser) und 300 - 500 ml o-Xylol oder Mesitylen wird mit 0.4 mmol Tri-tert-butylphosphin und dann mit 0.3 mmol Palladium(II)acetat versetzt und unter gutem Rühren 16 h unter Rückfluss erhitzt. Nach Erkalten trennt man die wässrige Phase ab, wäscht zweimal mit 200 ml Wasser, einmal mit 200 ml gesättigter Kochsalzlösung und trocknet über Magnesiumsulfat. Man filtriert über ein Celite-Bett ab, wäscht dieses mit o-Xylol oder Mesitylen nach, entfernt das Lösungsmittel fast vollständig im Vakuum, gibt 300 ml Ethanol zu, saugt vom ausgefallenen Rohprodukt ab, wäscht dieses dreimal mit je 100 ml EtOH und trocknet im Vakuum. Das Rohprodukt wird mit Toluol zweimal an Kieselgel gesäult. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird.

### Synthese von Ir(L2500)₃:

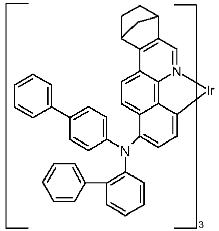

Der Komplex ist nicht Gegenstand der Ansprüche.
Einsatz von 11.6 g (10 mmol) Ir(L500-Br)₃ und 12.9 g (40 mmol) p-Biphenyl-o-biphenyl-amin [1372775-52-4], Mesitylen. Ausbeute: 11.1 g (5.3 mmol) 53 %; Reinheit: ca. 99.8 %ig nach HPLC.

Analog können folgende Verbindungen dargestellt werden (die mit * markierten Komplexe sind nicht Gegenstand der Ansprüche):

| **Bsp.** | **Produkt** | **Ausbeute** |
|---|---|---|
| Ir(L2501)₃ | 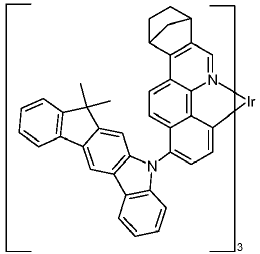 | 49 % |
| | Ir(L500-Br)₃ + [1257220-47-5] | |
| Ir(L2502)₃ * | 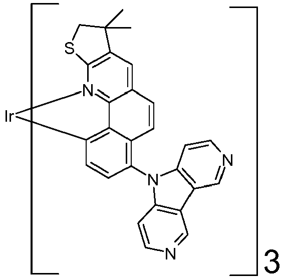 | 53 % |
| | Ir(L8-Br)₃ + [244-78-0] | |
| Ir(L2503)₃ | 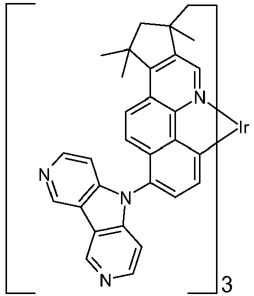 | 53 % |
| | Ir(L505-Br)₃ + [244-78-0] | |
| Ir(L2504)₃ | 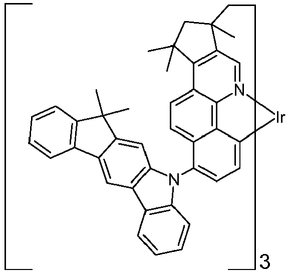 | 47 % |
| | Ir(L505-Br)₃ + [1257220-47-5] | |

### Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird. In den folgenden Beispielen werden die Ergebnisse verschiedener OLEDs vorgestellt. Glasplättchen, mit strukturiertem ITO (Indium Zinn Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 3 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M2:M1:Ir(L1)₃ (55%:35%:10%) bedeutet hierbei, dass das Material M2 in einem Volumenanteil von 55%, M1 in einem Anteil von 35% und Ir(L1)₃ in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 6 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50 % der Startleuchtdichte abgefallen ist, also von z. B. 1000 cd/m² auf 500 cd/m². Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

### Verwendung von erfindungsgemäßen Verbindungen als Emittermaterialien in phosphoreszierenden OLEDs

Die erfindungsgemäßen Verbindungen lassen sich unter anderem als phosphoreszierende Emittermaterialien in der Emissionsschicht in OLEDs einsetzen. Als Vergleich gemäß dem Stand der Technik wird die Verbindung Ir(Ref1)₃ verwendet. Die Ergebnisse der OLEDs sind in Tabelle 2 zusammengefasst.

**Tabelle 1: Aufbau der OLED**

| **Bsp.** | **HTL2 Dicke** | **EBL Dicke** | **EML Dicke** | **HBL Dicke** | **ETL Dicke** |
|---|---|---|---|---|---|
| **Gelbe OLEDs** | | | | | |
| D-Ref. | HTM 220 nm | --- | M2:M1:Ir(Ref1)₃ (75%:15%:10%) 20 nm | HBM 10 nm | ETM1:ETM2 (50%:50%) 40 nm |
| D-Ir(L1)₃ | HTM 220 nm | --- | M2:M1:Ir(L1)₃ (75%:15%:10%) 20 nm | HBM 10 nm | ETM1:ETM2 (50%:50%) 40 nm |
| D-Ir(L9)₃ | HTM 220 nm | --- | M2:M1:Ir(L9)₃ (75%:15%:10%) 20 nm | HBM 10 nm | ETM1:ETM2 (50%:50%) 40 nm |
| D-Ir(L505)₃ | HTM 220 nm | --- | M2:M1:Ir(L505)₃ (75%:15%:10%) 20 nm | HBM 10 nm | ETM1:ETM2 (50%:50%) 40 nm |
| D-Ir(L527)₃ | HTM 220 nm | --- | M2:M1:Ir(L527)₃ (75%:15%:10%) 20 nm | HBM 10 nm | ETM1:ETM2 (50%:50%) 40 nm |
| D-Ir(L1001)₃ | HTM 220 nm | --- | M2:M1:Ir(L1001)₃ (75%:15%:10%) 20 nm | HBM 10 nm | ETM1:ETM2 (50%:50%) 40 nm |
| D-Ir(L1500)₃ | HTM 220 nm | --- | M2:M1:Ir(L1500)₃ (75%:15%:10%) 20 nm | HBM 10 nm | ETM1:ETM2 (50%:50%) 40 nm |
| D-Ir(L3)₂(CL1) | HTM 220 nm | --- | M2:M1: Ir(L3)₂(CL1) (75%:15%:10%) 20 nm | HBM 10 nm | ETM1:ETM2 (50%:50%) 40 nm |
| D-Ir(L500)₂(CL2) | HTM 220 nm | --- | M2:M1: Ir(L500)₂(CL2) (75%:15%:10%) 20 nm | HBM 10 nm | ETM1:ETM2 (50%:50%) 40 nm |
| D-Ir(L565)₂(CL4) | HTM 220 nm | --- | M2:M1: Ir(503)₂(CL8) (75%:15%:10%) 20 nm | HBM 10 nm | ETM1 :ETM2 (50%:50%) 40 nm |
| D-Ir(L503)₂(CL8) | HTM 220 nm | --- | M2:M1: Ir(L503)₂(CL8) (75%:15%:10%) 20 nm | HBM 10 nm | ETM1 :ETM2 (50%:50%) 40 nm |

**Tabelle 2: Ergebnisse der Vakuum-prozessierten OLEDs**

| **Bsp.** | **EQE (%) 1000 cd/m²** | **Spannung (V) 1000 cd/m²** | **CIE x/y 1000 cd/m²** | **LD50 (h) 1000 cd/m²** |
|---|---|---|---|---|
| **Gelbe OLEDs** | | | | |
| D-Ref. | 19.7 | 3.1 | 0.44/0.55 | 210000 |
| D-Ir(L1)₃ | 22.7 | 3.2 | 0.50/0.48 | 330000 |
| D-Ir(L9)₃ | 13.1 | 3.0 | 0.30/0.69 | --- |
| D-Ir(L505)₃ | 23.0 | 3.2 | 0.48/0.50 | 320000 |
| D-Ir(L527)₃ | 19.8 | 3.6 | 0.61/0.38 | --- |
| D-Ir(L1001)₃ | 22.3 | 3.1 | 0.46/0.53 | 340000 |
| D-Ir(L1500)₃ | 21.9 | 3.4 | 0.54/0.44 | 390000 |
| D-Ir(L3)₂(CL1) | 23.3 | 3.2 | 0.47/0.51 | 230000 |
| D-Ir(L500)₂(CL2) | 22.7 | 3.3 | 0.48/0.51 | 250000 |
| D-Ir(L565)₂(CL4) | 19.5 | 3.2 | 0.60/0.38 | --- |
| D-Ir(L503)₂(CL8) | 21.8 | 3.4 | 0.51/0.48 | --- |

### 2) Lösungs-prozessierte Devices

### A: Aus löslichen Funktionsmaterialien

Die erfindungsgemäßen Iridium-Komplexe können auch aus Lösung verarbeitet werden und führen dort zu prozesstechnisch wesentlich einfacheren OLEDs im Vergleich zu den vakuumprozessierten OLEDs mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die Iösungsprozessierten Devices enthalten eine Emissionsschicht aus (Polystyrol):M4:M5:Ir(L)₃ (25%:25%:40%:10%). Die Emissionsschicht wird in einer Inertgasatmosphäre, im vorliegenden Fall Argon, aufgeschleudert und 30 min bei 130 °C ausgeheizt. Zuletzt wird eine Kathode aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z. B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Ir(L)₃** | **EQE (%) 1000 cd/m²** | **Spannung (V) 1000 cd/m²** | **CIE x/y 1000 cd/m²** |
|---|---|---|---|---|
| **Gelbe OLEDs** | | | | |
| S-Ir(L25)₃ | Ir(L25)₃ | 17.8 | 5.6 | 0.59/0.40 |
| S-Ir(L28)₃ | Ir(L28)₃ | 19.0 | 5.3 | 0.48/0.48 |
| S-Ir(L510)₃ | Ir(L510)₃ | 19.5 | 5.4 | 0.48/0.50 |
| S-Ir(L520)₃ | Ir(L520)₃ | 18.4 | 5.5 | 0.60/0.38 |
| S-Ir(L550)₃ | Ir(L550)₃ | 17.8 | 5.2 | 0.47/0.52 |
| S-Ir(L562)₃ | Ir(L562)₃ | 20.4 | 5.5 | 0.48/0.50 |
| S-Ir(L1000)₃ | Ir(L1000)₃ | 19.7 | 5.3 | 0.46/0.53 |
| S-Ir(L1507)₃ | Ir(L1507)₃ | 18.0 | 5.5 | 0.54/0.44 |
| S-Ir(L7)₂(CL1) | Ir(L7)₂(CL1) | 19.8 | 5.4 | 0.45/0.53 |
| S-Ir(L505)₂(CL2) | Ir(L505)₂(CL2) | 19.4 | 5.3 | 0.46/0.52 |
| S-Ir(L565)₂(CL8) | Ir(L565)₂(CL8) | 16.5 | 5.4 | 0.60/0.38 |
| S-Ir(L556)₂(L4) | Ir(L556)₂(L4) | 17.6 | 5.5 | 0.48/0.49 |
| S-Ir(L2000)₃ | Ir(L2000)₃ | 18.9 | 5.4 | 0.49/0.50 |
| S-Ir(L2504)₃ | Ir(L2504)₃ | 14.3 | 5.2 | 0.53/0.46 |

### 3) Weiß emittierende OLEDs

Gemäß den allgemeinen Verfahren aus 1) wird eine weiß emittierende OLED mit folgendem Schichtaufbau hergestellt:

**Tabelle 4: Aufbau der weißen OLEDs**

| **Bsp.** | **HTL2** | **EML Rot** | **EML Blau** | **EML Grün** | **HBL** | **ETL** |
|---|---|---|---|---|---|---|
| | **Dicke** | **Dicke** | **Dicke** | **Dicke** | **Dicke** | **Dicke** |
| D-W1 | HTM 230 nm | EBM:Ir-R (97%:3%) 9 nm | M3:M2:Ir-B (40%:50%:10%) 8 nm | M2:Ir(L527)₃ (90%:10%) 7 nm | HBM 10 nm | ETM1 :ET M2 (50%:50 %) 30 nm |

**Tabelle 5: Deviceergebnisse**

| **Bsp.** | **EQE (%)** | **Spannung (V) 1000 cd/m²** | **CIE x/y 1000 cd/m² CRI** | **LD50 (h) 1000 cd/m²** |
|---|---|---|---|---|
| | **1000 cd/m²** | | | |
| D-W1 | 20.3 | 6.5 | 0.43/0.43 | 3000 |
| | | | 77 | |

**Tabelle 6: Strukturformeln der verwendeten Materialien**

| | |
|---|---|
| 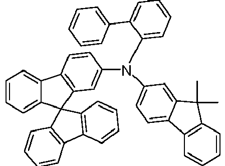 | 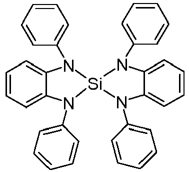 |
| HTM | EBM |
| 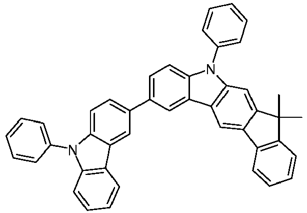 | 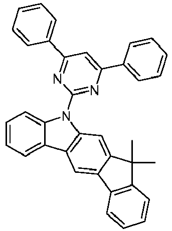 |
| M1 | M2 |
| 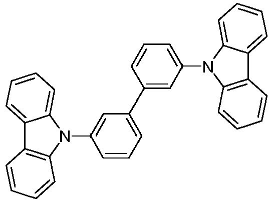 | 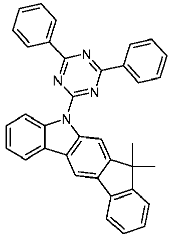 |
| M3 | HBM |
| 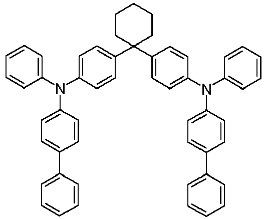 | 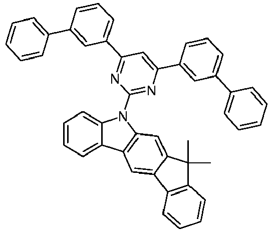 |
| M4 | M5 |
| 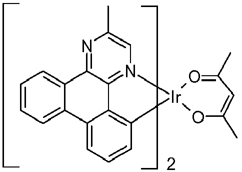 | 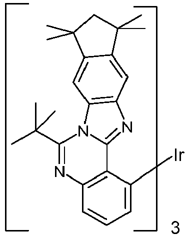 |
| Ir-R | Ir-B |
| 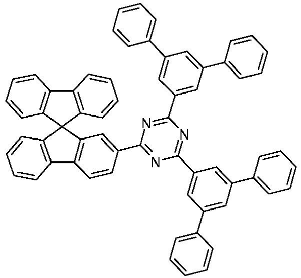 | 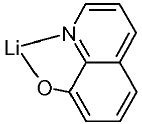 |
| ETM1 | ETM2 |
| 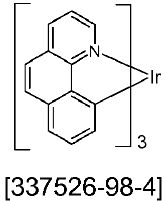 | |
| Ir(Ref1)₃ | |

## Patentansprüche

1. Verbindung gemäß Formel (1),
[Ir(L)ₙ(L')ₘ] Formel (1)
wobei die Verbindung der allgemeinen Formel (1) eine Teilstruktur Ir(L)ₙ der Formel (2) enthält: 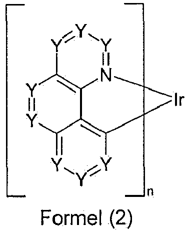 wobei für die verwendeten Symbole und Indizes gilt:
Y ist bei jedem Auftreten gleich oder verschieden CR oder N mit der Maßgabe, dass maximal ein Symbol Y pro Cyclus für N steht, oder zwei benachbarte Symbole Y stehen zusammen für eine Gruppe der folgenden Formel (3), 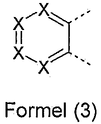 wobei die gestrichelten Bindungen die Verknüpfung dieser Gruppe im Liganden symbolisieren;
X ist bei jedem Auftreten gleich oder verschieden CR oder N mit der Maßgabe, dass maximal zwei Symbole X pro Ligand für N stehen;
R ist bei jedem Auftreten gleich oder verschieden H, D, Cl, Br, I, CN, eine geradkettige Alkyl- oder Alkoxygruppe mit 1 bis 40 C-Atomen oder eine verzweigte oder cyclische Alkyl- oder Alkoxygruppe mit 3 bis 40 C-Atomen, oder ein aromatisches oder heteroaromatisches Ringsystem mit 5 bis 60 aromatischen Ringatomen, das jeweils durch einen oder mehrere Reste R¹ substituiert sein kann; dabei können zwei oder mehrere benachbarte Reste R auch miteinander ein mono- oder polycyclisches, aliphatisches Ringsystem bilden;
R¹ ist bei jedem Auftreten gleich oder verschieden H, eine geradkettige Alkylgruppe mit 1 bis 40 C-Atomen oder eine verzweigte oder cyclische Alkylgruppe mit 3 bis 40 C-Atomen;
L' ist gleich oder verschieden bei jedem Auftreten ein mono- oder bidentater Ligand;
n ist 2 oder 3;
m ist 0 oder 1;
**dadurch gekennzeichnet, dass** in der Teilstruktur der Formel (2) zwei benachbarte Gruppen Y für CR stehen und die jeweiligen Reste R zusammen mit den C-Atomen einen Ring der folgenden Formel (4) oder Formel (5) aufspannen und/oder dass zwei benachbarte Gruppen Y für eine Gruppe der Formel (3) stehen und in dieser Gruppe der Formel (3) zwei benachbarte Gruppen X für CR stehen und die die jeweiligen Reste R zusammen mit den C-Atomen einen Ring der folgenden Formel (4) oder Formel (5) aufspannen, 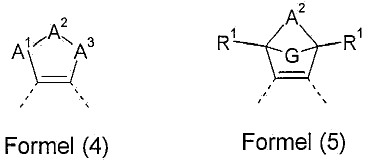 wobei R¹ und R² die oben genannten Bedeutungen aufweisen, die gestrichelten Bindungen die Verknüpfung der beiden Kohlenstoffatome im Liganden andeuten und weiterhin gilt:
A¹, A³ ist gleich oder verschieden bei jedem Auftreten C(R³)₂ oder O;
A² ist C(R¹)₂;
G ist eine Alkylengruppe mit 2 C-Atomen;
R³ ist gleich oder verschieden bei jedem Auftreten eine geradkettige Alkylgruppe mit 1 bis 10 C-Atomen, eine verzweigte oder cyclische Alkylgruppe mit 3 bis 10 C-Atomen, oder ein aromatisches Ring-system mit 5 bis 24 aromatischen Ringatomen; dabei können zwei Reste R³, welche an dasselbe Kohlenstoffatom gebunden sind, miteinander ein aliphatisches Ringsystem bilden und so ein Spirosystem aufspannen.

2. Verbindung nach Anspruch 1, **dadurch gekennzeichnet, dass** n = 3 und m = 0 ist oder dass n = 2 und m = 1 ist, wobei L' ein bidentater Ligand ist, der über ein Kohlenstoffatom und ein Stickstoffatom, ein Kohlenstoffatom und ein Sauerstoffatom, zwei Sauerstoffatome, zwei Stickstoffatome oder ein Sauerstoff- und ein Stickstoffatom an das Iridium koordiniert.

3. Verbindung nach Anspruch 1 oder 2, **dadurch gekennzeichnet, dass** die Teilstruktur Ir(L)ₙ ausgewählt ist aus den Strukturen der Formeln (6) bis (15), 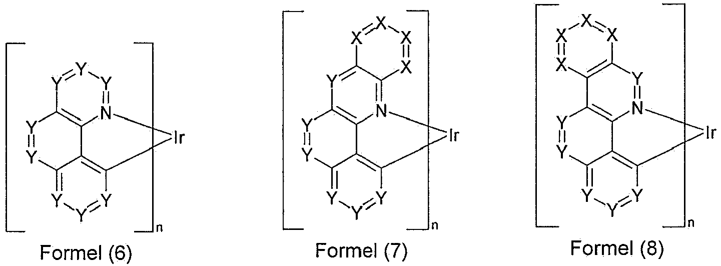 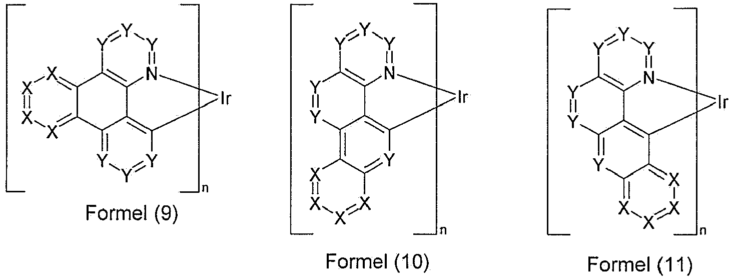 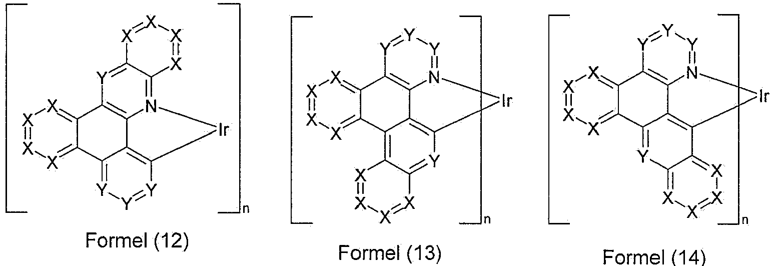 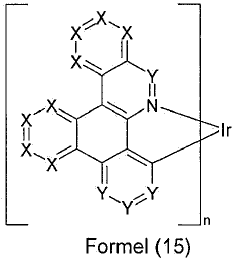 wobei Y bei jedem Auftreten gleich oder verschieden für CR oder N steht und die weiteren Symbole und Indizes die in Anspruch 1 genannten Bedeutungen aufweisen.

4. Verbindung nach einem oder mehreren der Ansprüche 1 bis 3, **dadurch gekennzeichnet, dass** im Liganden L insgesamt 0, 1 oder 2 der Symbole Y und, falls vorhanden, X für N stehen.

5. Verbindung nach einem oder mehreren der Ansprüche 1 bis 4, **dadurch gekennzeichnet, dass** die Teilstruktur Ir(L)ₙ ausgewählt ist aus den Strukturen der Formeln (6-1) bis (6-7), (7-1) bis (7-6), (8-1) bis (8-5), (9-1) bis (9-5), (10-1) bis (10-5), (11-1) bis (11-6), (12-1) bis (12-4), (13-1) bis (13-3), (14-1) bis (14-4) und (15-1) bis (15-3), 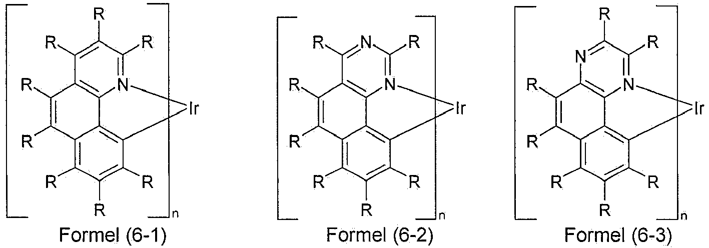 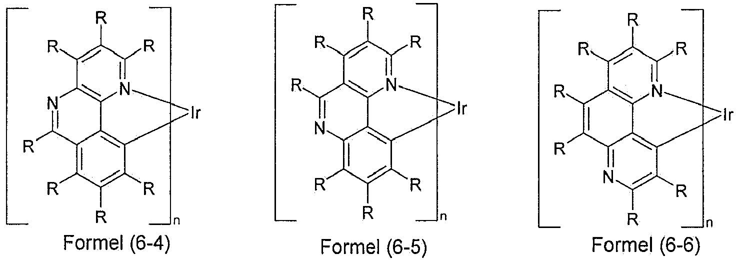 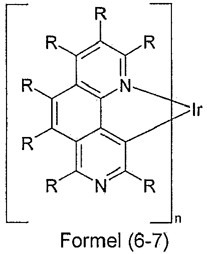 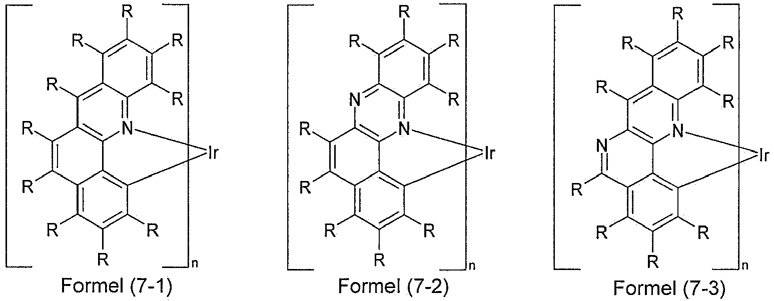 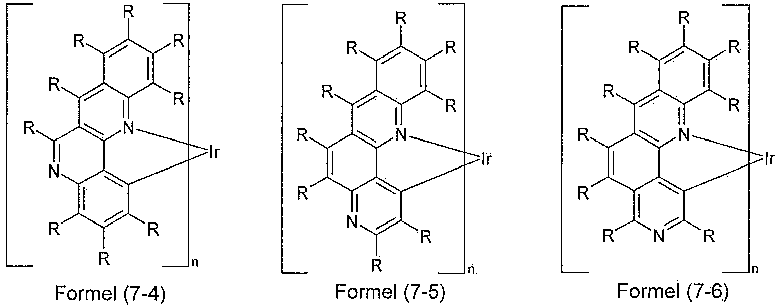 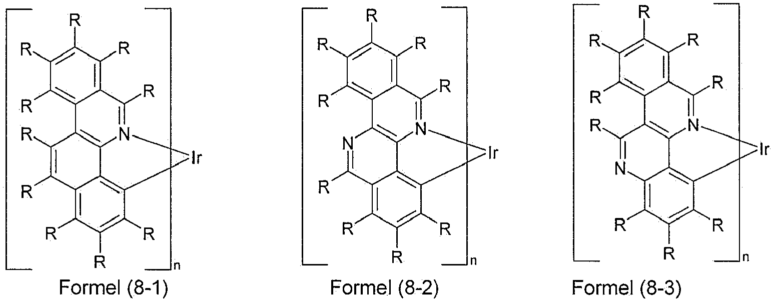 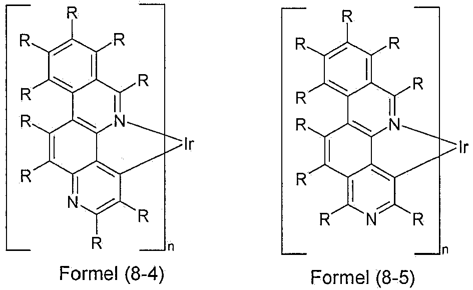 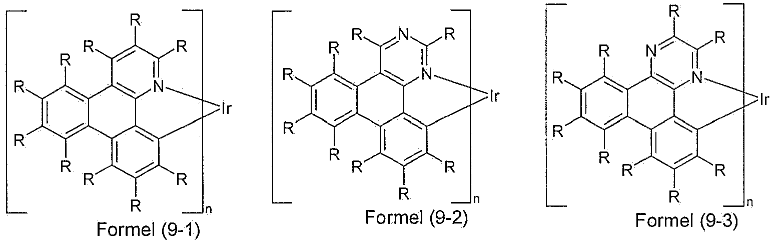 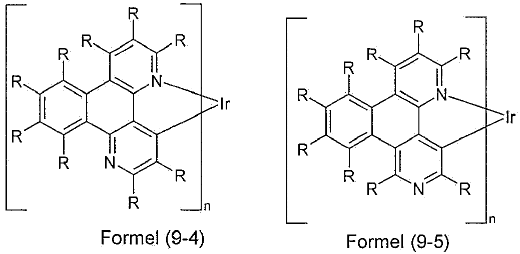 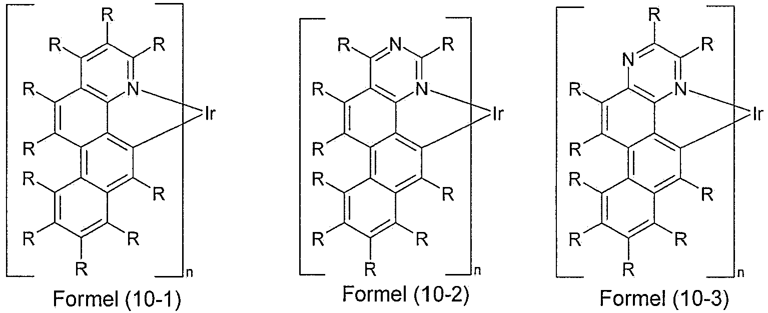 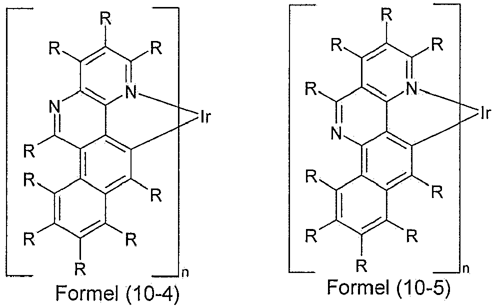 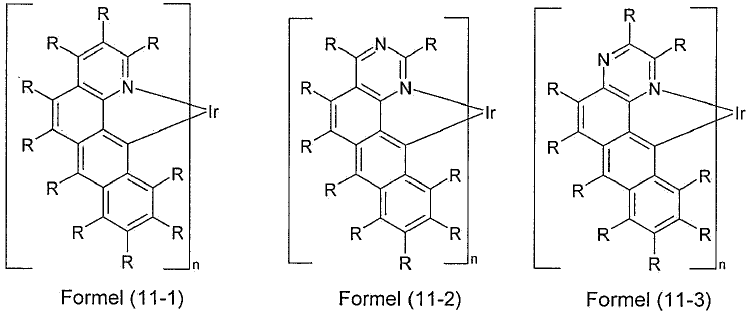 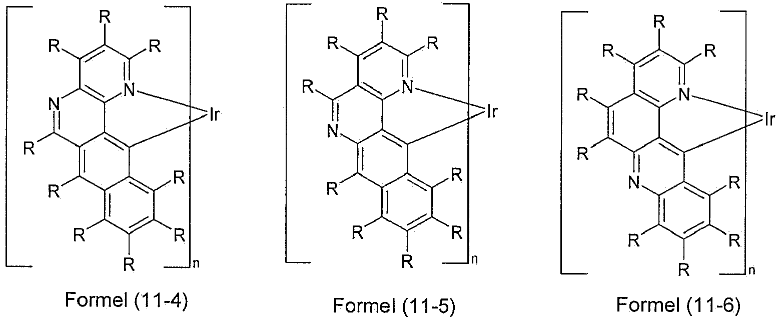 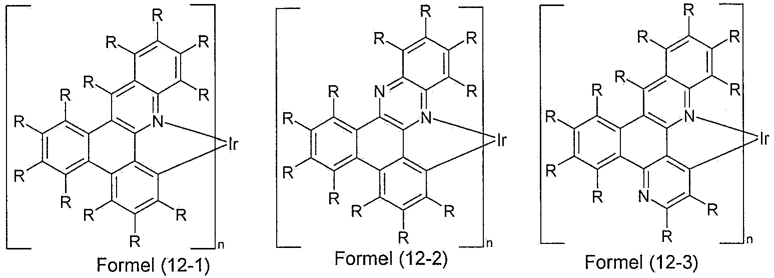 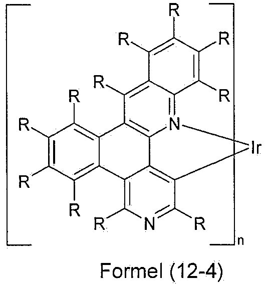 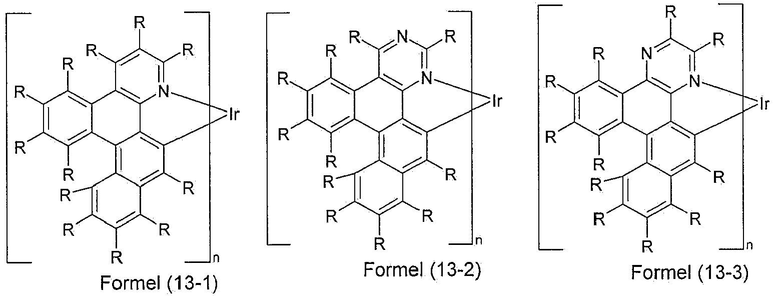 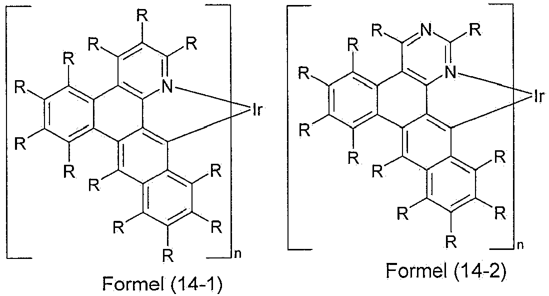 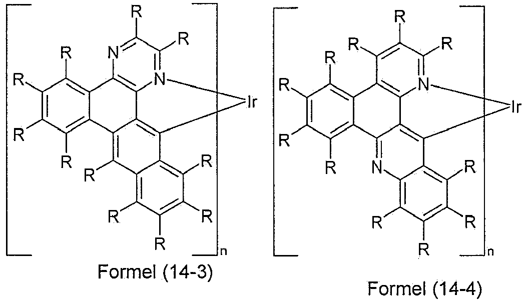 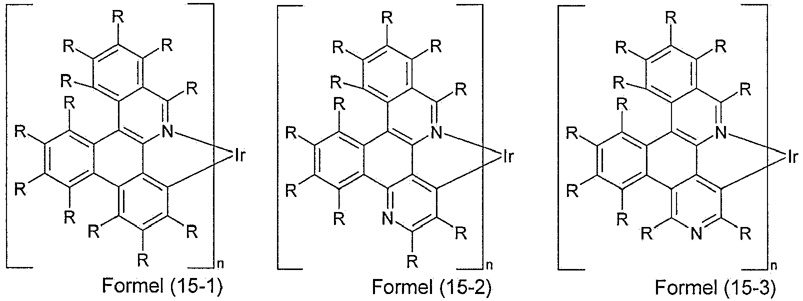 wobei die verwendeten Symbole und Indizes die in Anspruch 1 genannten Bedeutungen aufweisen.

6. Verbindung nach einem oder mehreren der Ansprüche 1 bis 5, **dadurch gekennzeichnet, dass** ein oder mehrere der Atome Y und/oder X für N stehen und benachbart zu diesem Stickstoffatom ein Substituent R gebunden ist, welcher ausgewählt ist aus der Gruppe bestehend aus Alkyl- oder Alkoxygruppen mit 1 bis 10 C-Atomen oder aromatischen bzw. heteroaromatischen Ringsystemen, die durch einen oder mehrere Substituenten R¹ substituiert sein können oder dass dieser Rest R in einer Struktur der Formel (4) oder (5) eingebunden ist.

7. Verbindung nach einem oder mehreren der Ansprüche 1 bis 6, **dadurch gekennzeichnet, dass** die Teilstruktur Ir(L)ₙ ausgewählt ist aus den Teilstrukturen der Formeln (6a) bis (15h), 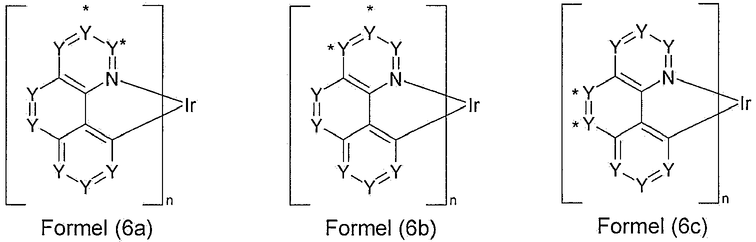 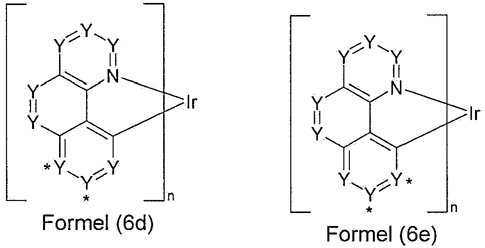 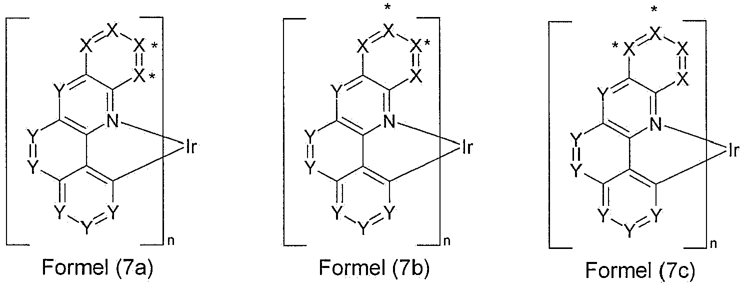 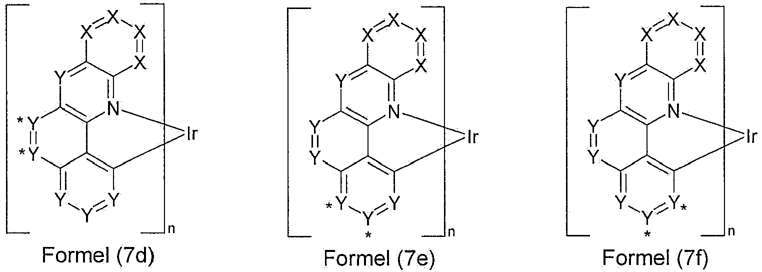 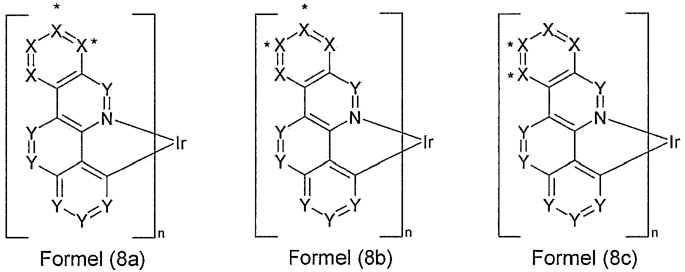 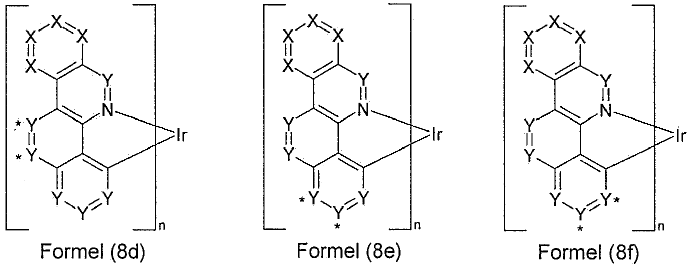 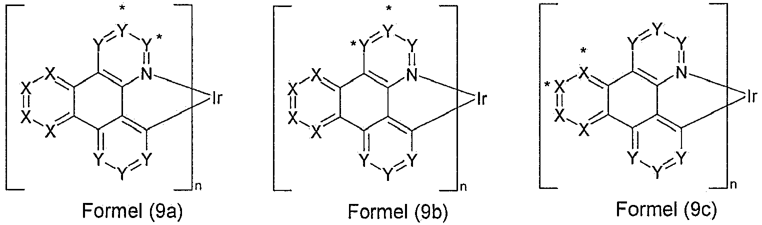 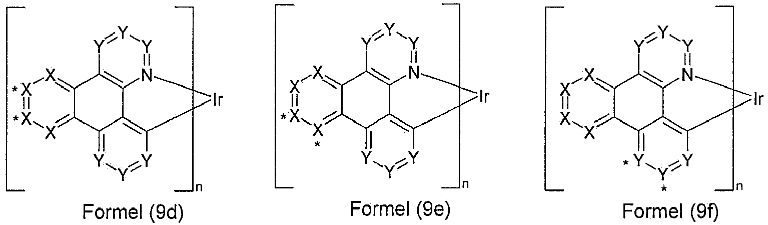 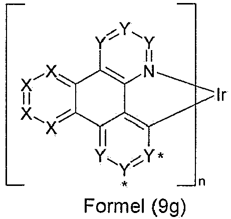 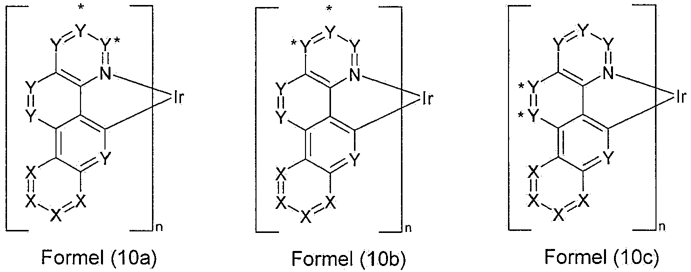 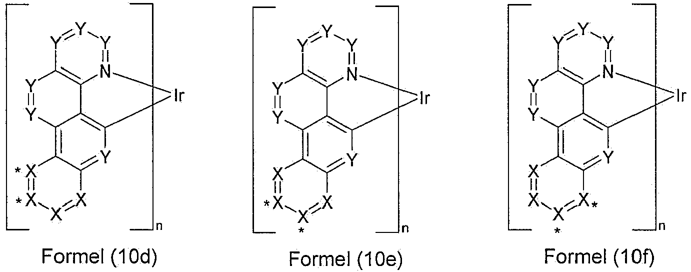 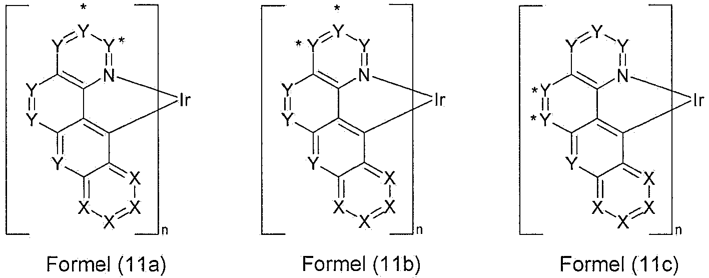 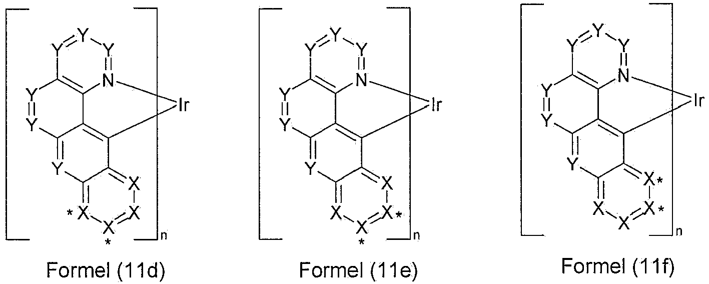 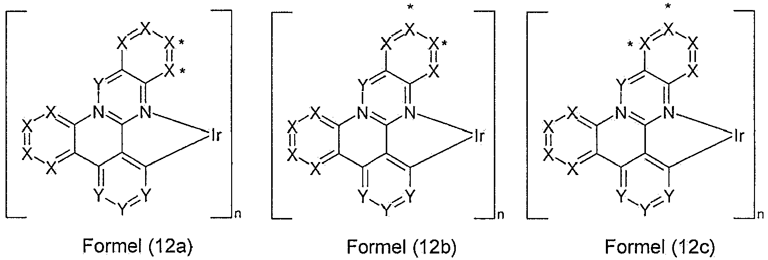 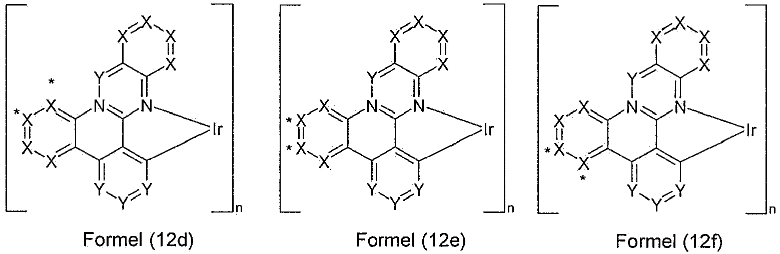 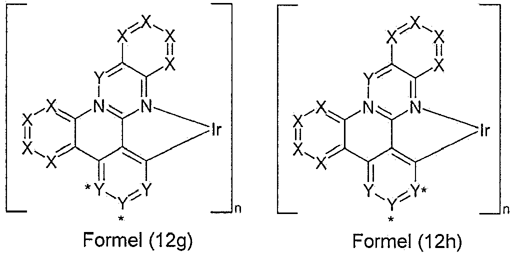 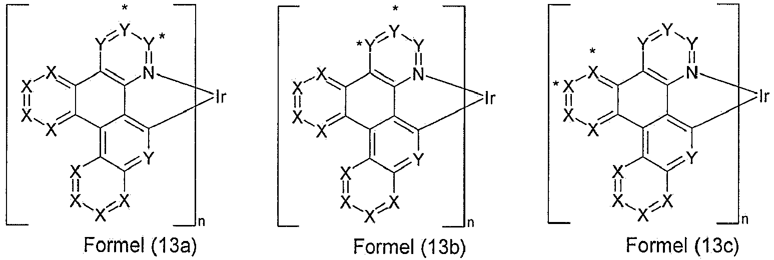 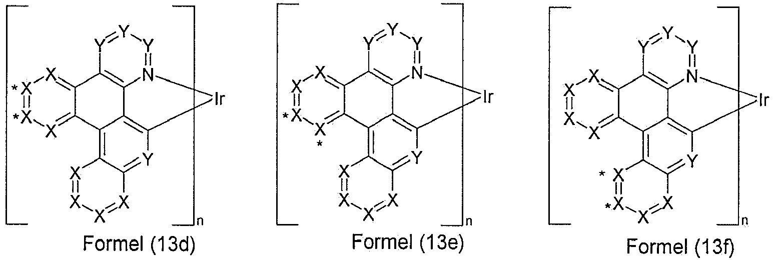 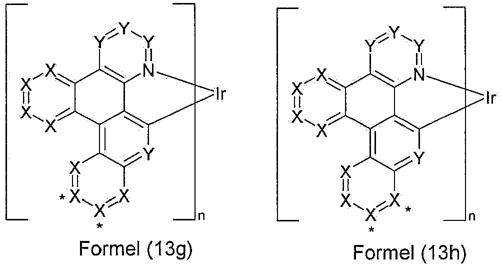 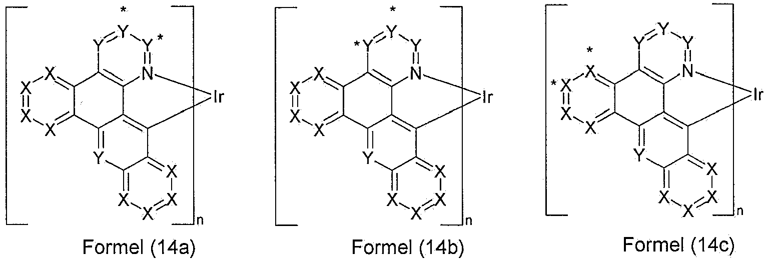 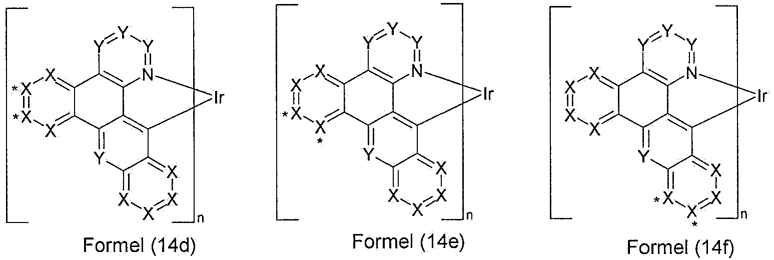 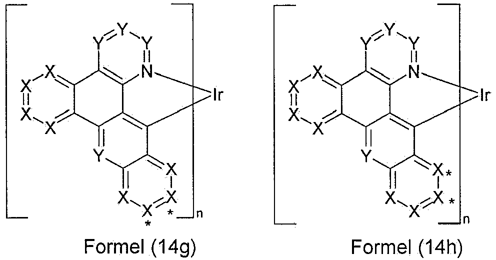 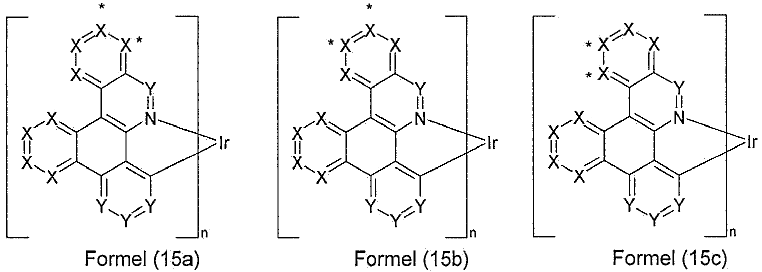 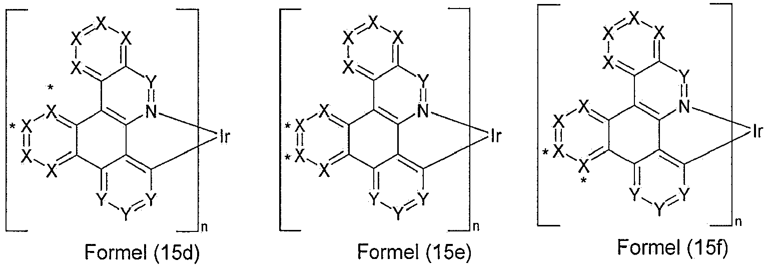 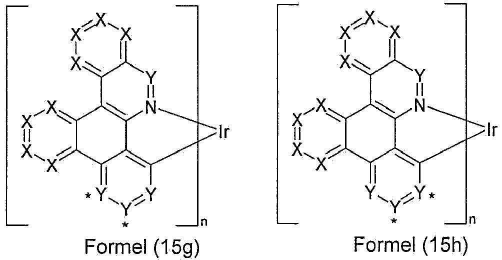 wobei die verwendeten Symbole und Indizes die in Anspruch 1 genannten Bedeutungen aufweisen und * jeweils die Position anzeigt, an der die beiden benachbarten Gruppen Y bzw. X für CR stehen und die jeweiligen Reste R zusammen mit den C-Atomen einen Ring der Formel (4) oder Formel (5) aufspannen.

8. Verbindung nach einem oder mehreren der Ansprüche 1 bis 7, **dadurch gekennzeichnet, dass** die Struktur gemäß Formel (4) ausgewählt ist aus den Formeln (4-A), (4-B), (4-D) und (4-E), 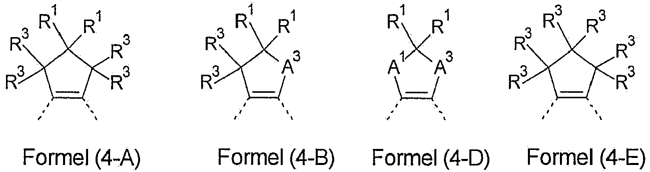 wobei R¹ und R³ die in Anspruch 1 genannten Bedeutungen aufweisen und A¹ und A³ für O stehen,
und dass die Struktur gemäß Formel (5) ausgewählt ist aus den Formeln (5-A) und (5-C), 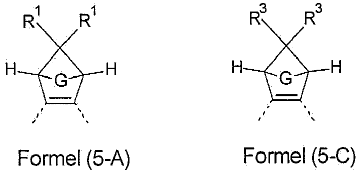 wobei die verwendeten Symbole die in Anspruch 1 genannten Bedeutungen aufweisen.

9. Verbindung nach einem oder mehreren der Ansprüche 1 bis 8, **dadurch gekennzeichnet, dass** R³ gleich oder verschieden bei jedem Auftreten für eine geradkettige Alkylgruppe mit 1 bis 10 C-Atomen oder eine verzweigte oder cyclische Alkylgruppe mit 3 bis 10 C-Atomen oder ein aromatisches Ringsystem mit 5 bis 14 aromatischen Ringatomen steht; dabei können zwei Reste R³, welche an dasselbe Kohlenstoffatom gebunden sind, miteinander ein aliphatisches Ringsystem bilden und so ein Spirosystem aufspannen.

10. Verfahren zur Herstellung einer Verbindung nach einem oder mehreren der Ansprüche 1 bis 9 durch Umsetzung der Liganden mit Iridiumalkoholaten der Formel (44), mit Iridiumketoketonaten der Formel (45), mit Iridiumhalogeniden der Formel (46) oder mit dimeren Iridiumkomplexen der Formel (47) oder (48), 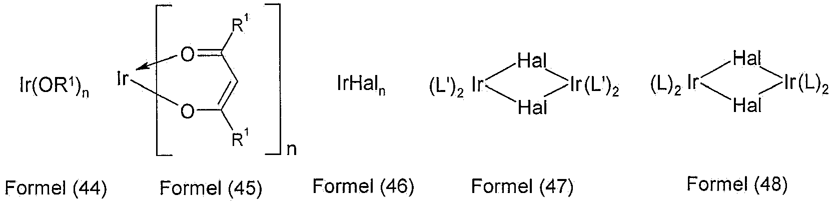 wobei die Symbole und Indizes L', m, n und R¹ die in Anspruch 1 angegebenen Bedeutungen haben und Hal = F, Cl, Br oder I ist, oder durch Umsetzung der Liganden L mit Iridiumkomplexen der Formel [Ir(L')₂(HOMe)₂]A oder [Ir(L')₂(NCMe)₂]A oder durch Umsetzung der Liganden L' mit Iridiumkomplexen der Formel [Ir(L)₂(HOMe)₂]A oder [Ir(L)₂(NCMe)₂]A, wobei A jeweils ein nicht koordinierendes Anion darstellt, oder unter Verwendung von Iridiumverbindungen, die sowohl Alkoholat- und/oder Halogenid- und/oder Hydroxy- wie auch Ketoketonatreste tragen, als Edukte.

11. Formulierung, enthaltend mindestend eine Verbindung nach einem oder mehreren der Ansprüche 1 bis 9 und mindestens eine weitere Verbindung, insbesondere ein Lösemittel.

12. Verwendung einer Verbindung nach einem oder mehreren der Ansprüche 1 bis 9 in einer organischen Elektrolumineszenzvorrichtung.

13. Organische Elektrolumineszenzvorrichtung enthaltend mindestens eine Verbindung nach einem oder mehreren der Ansprüche 1 bis 9.

14. Organische Elektrolumineszenzvorrichtung nach Anspruch 13, **dadurch gekennzeichnet, dass** die Verbindung nach einem oder mehreren der Ansprüche 1 bis 9 als emittierende Verbindung in einer oder mehreren emittierenden Schichten eingesetzt wird.

15. Organische Elektrolumineszenzvorrichtung nach Anspruch 14, **dadurch gekennzeichnet, dass** als Matrixmaterial für die Verbindung nach einem oder mehreren der Ansprüche 1 bis 9 Ketone, Phosphinoxide, Sulfoxide, Sulfone, Triarylamine, Carbazole, Indolocarbazole, Indenocarbazole, Azacarbazole, bipolare Matrixmaterialien, Azaborole, Boronester, Diazasilole, Diazaphosphole, Triazine, Zinkkomplexe, Berylliumkomplexe, Dibenzofurane und/oder verbrückte Carbazolderivate eingesetzt werden.

## Claims

1. Compound of formula (1)
[Ir(L)ₙ(L')ₘ] Formula (1)
where the compound of the general formula (1) contains a substructure Ir(L)ₙ of the formula (2): 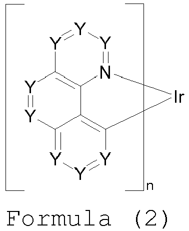 where the symbols and indices used are as follows:
Y is the same or different at each instance and is CR or N, with the proviso that not more than one symbol Y per cycle is N, or two adjacent symbols Y together are a group of the following formula (3) : 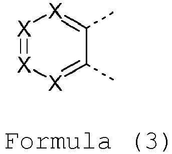 where the dotted bonds symbolize the linkage of this group in the ligand;
X is the same or different at each instance and is CR or N, with the proviso that not more than two symbols X per ligand are N;
R is the same or different at each instance and is H, D, Cl, Br, I, CN, a straight-chain alkyl or alkoxy group having 1 to 40 carbon atoms or a branched or cyclic alkyl or alkoxy group having 3 to 40 carbon atoms, or an aromatic or heteroaromatic ring system which has 5 to 60 aromatic ring atoms and may be substituted in each case by one or more R¹ radicals; at the same time, two or more adjacent R radicals together may also form a mono- or polycyclic, aliphatic ring system;
R¹ is the same or different at each instance and is H, a straight-chain alkyl group having 1 to 40 carbon atoms or a branched or cyclic alkyl group having 3 to 40 carbon atoms;
L' is the same or different at each instance and is a mono- or bidentate ligand;
n is 2 or 3;
m is 0 or 1;
**characterized in that**, in the substructure of the formula (2), two adjacent Y groups are CR and the respective R radicals together with the carbon atoms form a ring of the following formula (4) or formula (5) and/or **in that** two adjacent Y groups are a group of the formula (3) and two adjacent X groups in this group of the formula (3) are CR and the respective R radicals together with the carbon atoms form a ring of the following formula (4) or formula (5): 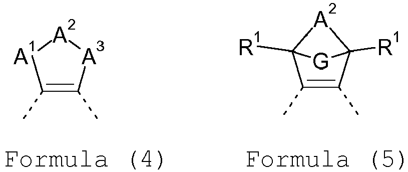 where R¹ and R² are as defined above, the dotted bonds signify the linkage of the two carbon atoms in the ligand and, in addition:
A¹, A³ is the same or different at each instance and is C(R³)₂ or O;
A² is C(R¹)₂;
G is an alkylene group having 2 carbon atoms;
R³ is the same or different at each instance and is a straight-chain alkyl group having 1 to 10 carbon atoms, a branched or cyclic alkyl group having 3 to 10 carbon atoms, or an aromatic ring system having 5 to 24 aromatic ring atoms; at the same time, two R³ radicals bonded to the same carbon atom together may form an aliphatic ring system and thus form a spiro system.

2. Compound according to Claim 1, **characterized in that** n = 3 and m = 0 or **in that** n = 2 and m = 1, where L' is a bidentate ligand which coordinates to the iridium via one carbon atom and one nitrogen atom, one carbon atom and one oxygen atom, two oxygen atoms, two nitrogen atoms or one oxygen atom and one nitrogen atom.

3. Compound according to Claim 1 or 2, **characterized in that** the substructure Ir(L)ₙ is selected from the structures of the formulae (6) to (15) : 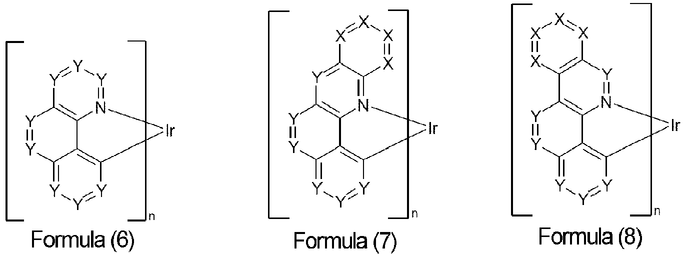 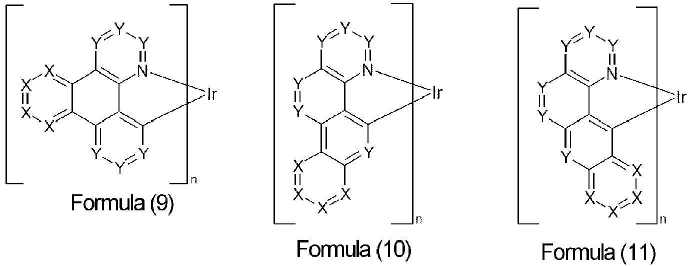 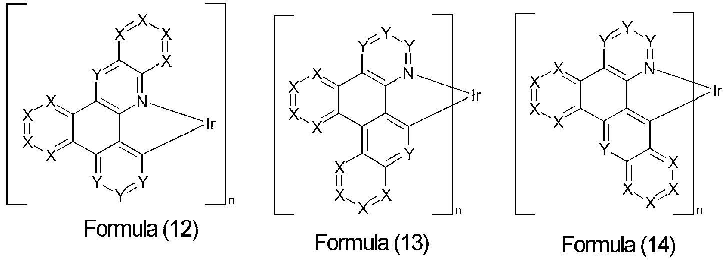 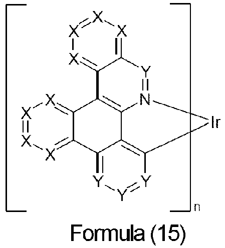 where Y is the same or different at each instance and is CR or N and the further symbols and indices are as defined in Claim 1.

4. Compound according to one or more of Claims 1 to 3, **characterized in that** a total of 0, 1 or 2 of the symbols Y and, if present, X in the ligand L are N.

5. Compound according to one or more of Claims 1 to 4, **characterized in that** the substructure Ir(L)ₙ is selected from the structures of the formulae (6-1) to (6-7), (7-1) to (7-6), (8-1) to (8-5), (9-1) to (9-5), (10-1) to (10-5), (11-1) to (11-6), (12-1) to (12-4), (13-1) to (13-3), (14-1) to (14-4) and (15-1) to (15-3): 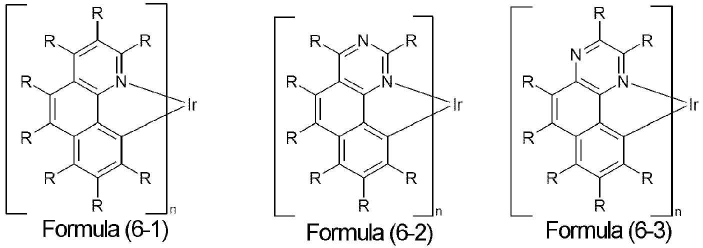 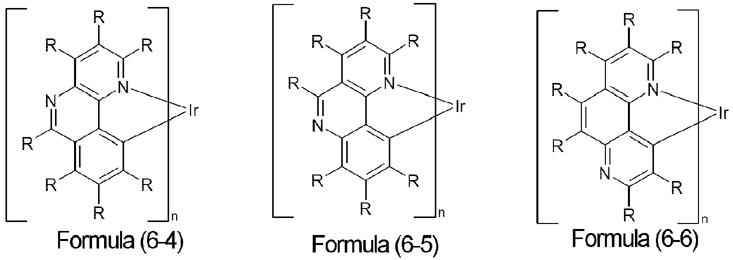 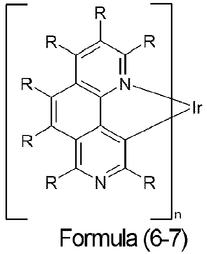 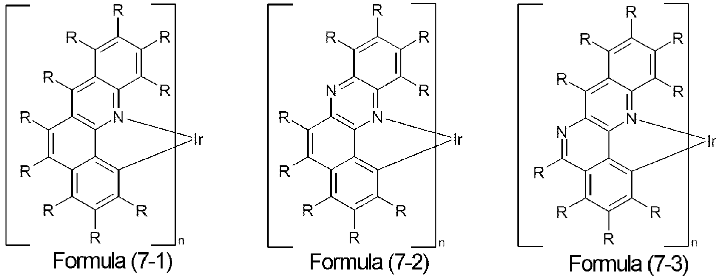 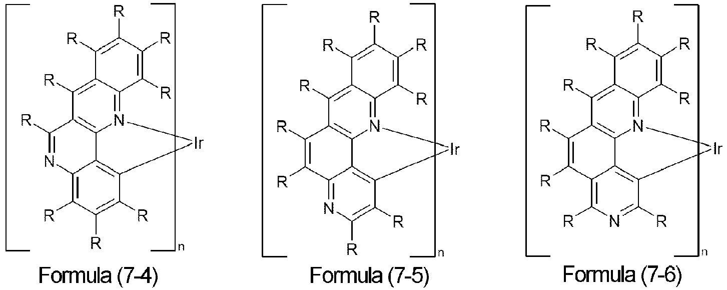 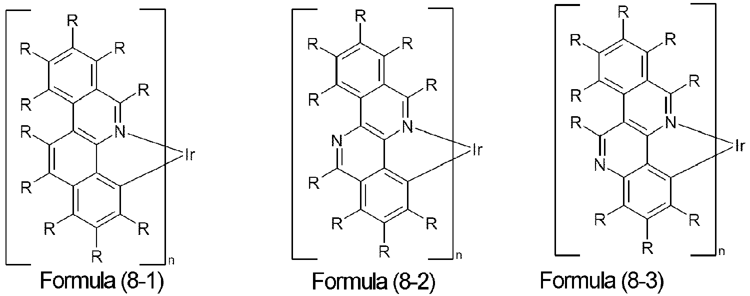 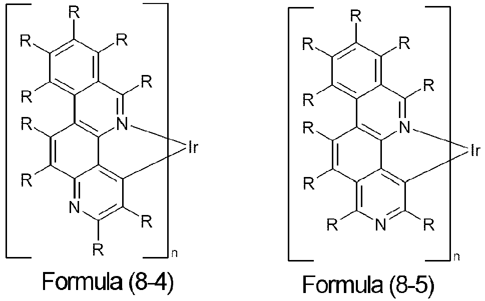 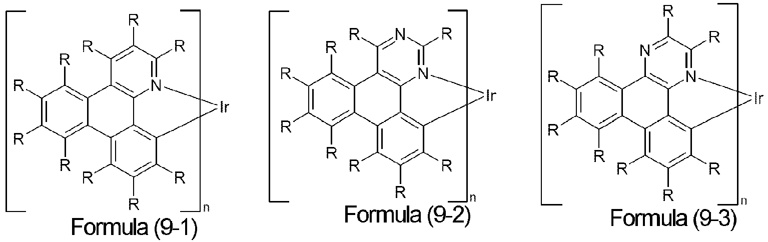 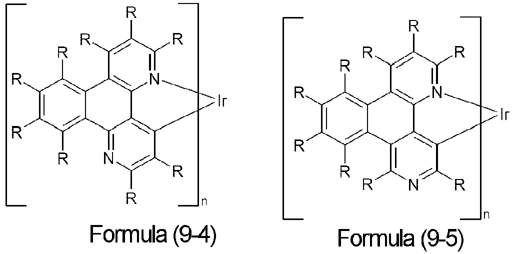 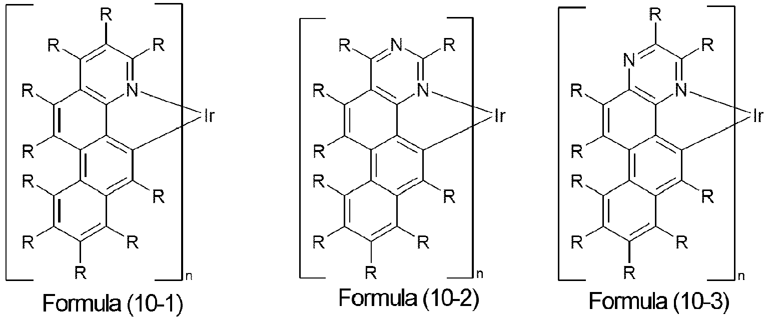 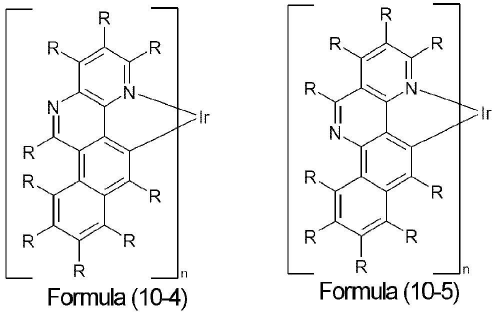 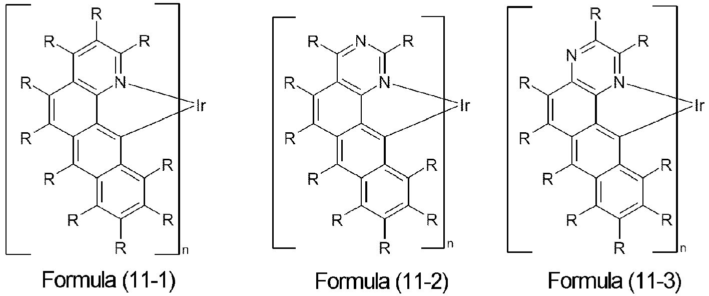 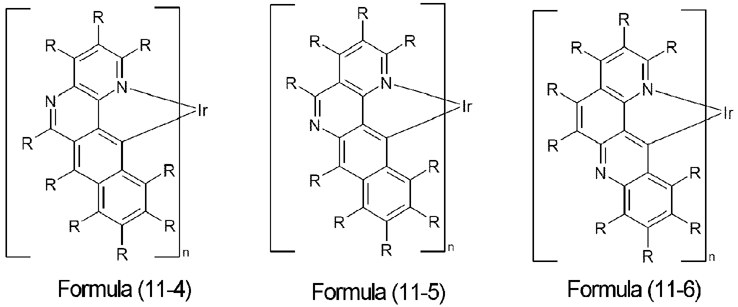 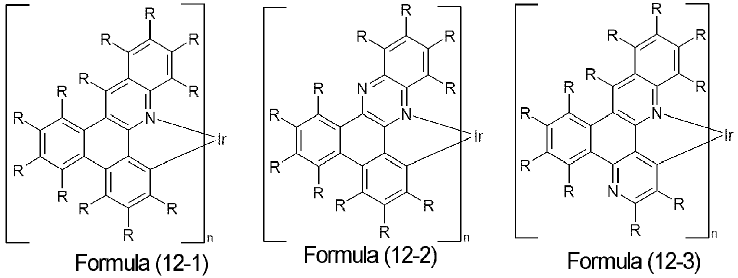 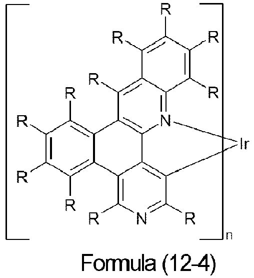 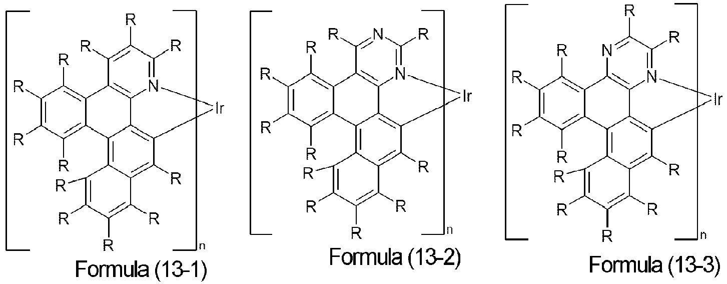 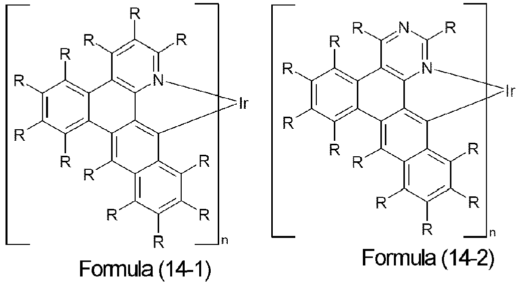 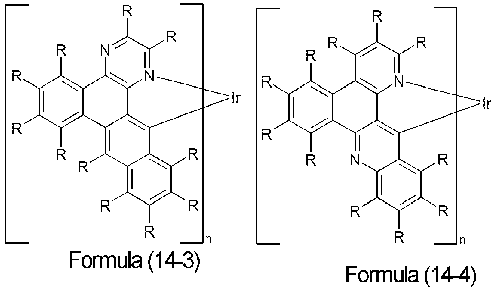 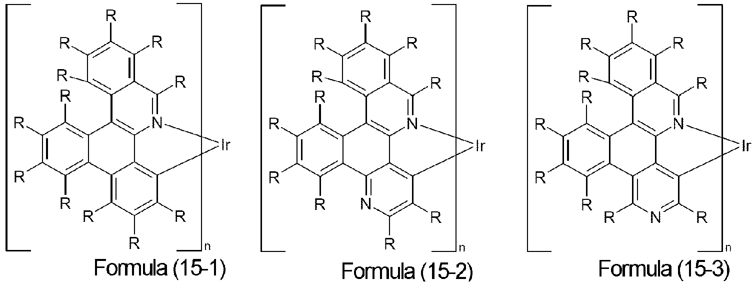 where the symbols and indices used are as defined in Claim 1.

6. Compound according to one or more of Claims 1 to 5, **characterized in that** one or more of the Y and/or X atoms are N and a substituent R bonded adjacent to this nitrogen atom is selected from the group consisting of alkyl or alkoxy groups having 1 to 10 carbon atoms or aromatic or heteroaromatic ring systems which may be substituted by one or more substituents R¹ or **in that** this R radical is incorporated in a structure of the formula (4) or (5) .

7. Compound according to one or more of Claims 1 to 6, **characterized in that** the substructure Ir(L)ₙ is selected from the substructures of the formulae (6a) to (15h): 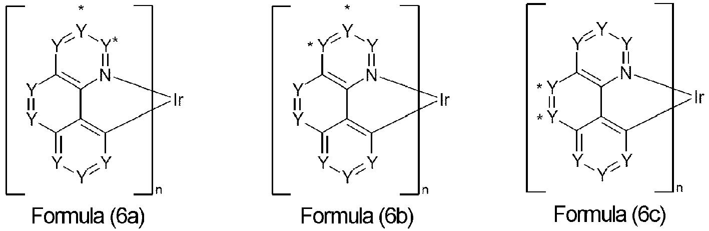 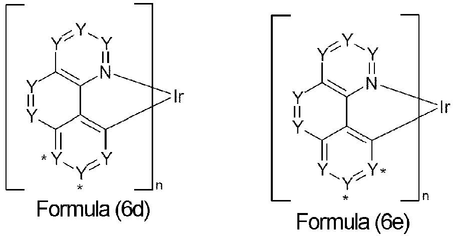 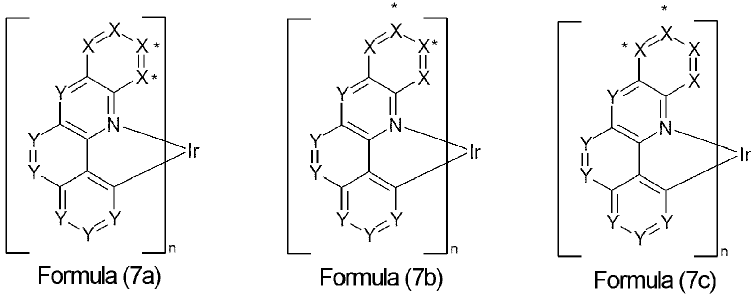 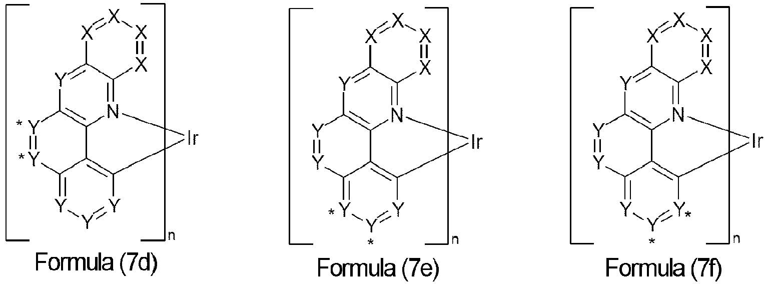 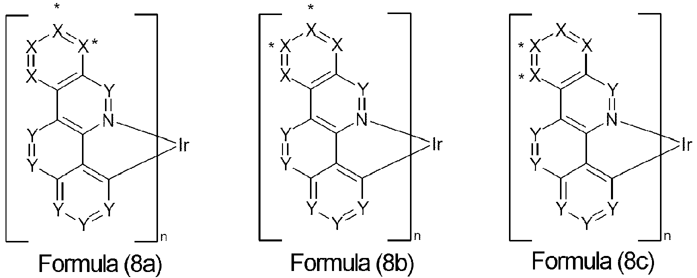 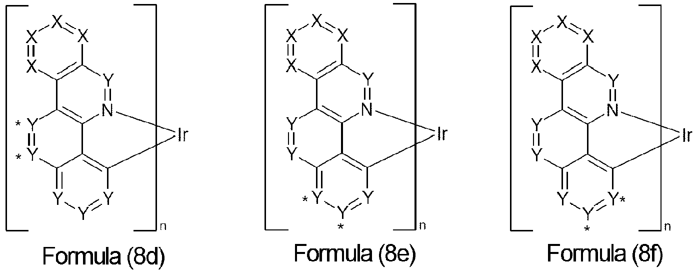 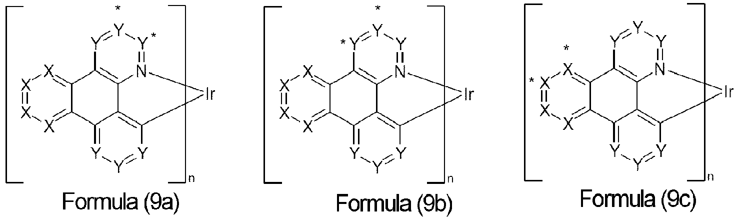 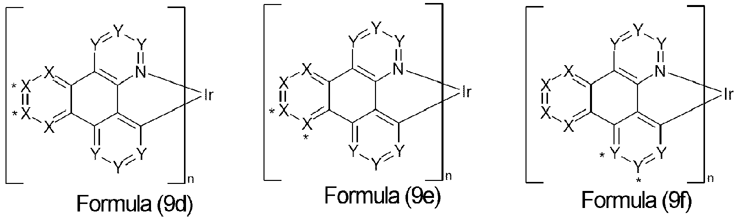 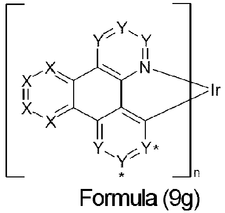 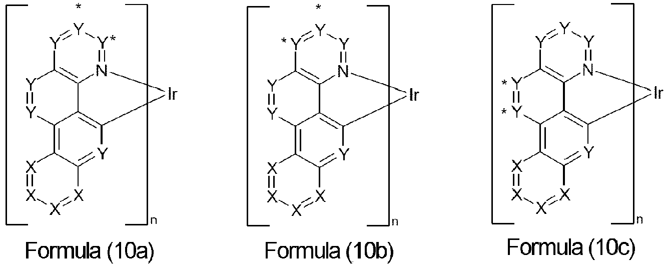 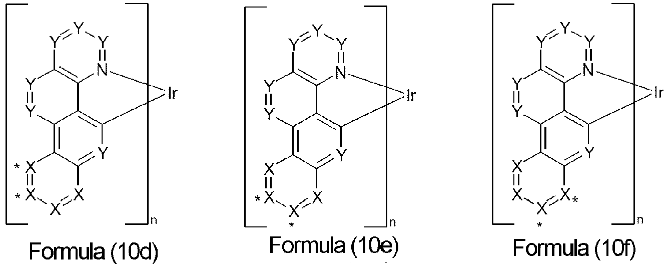 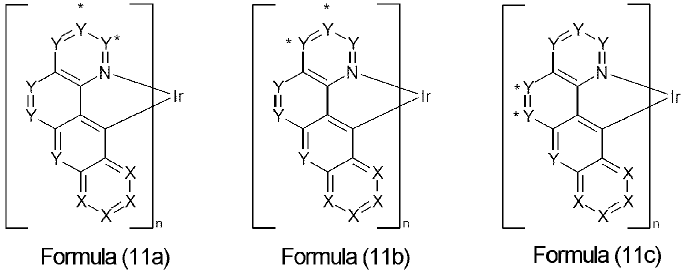 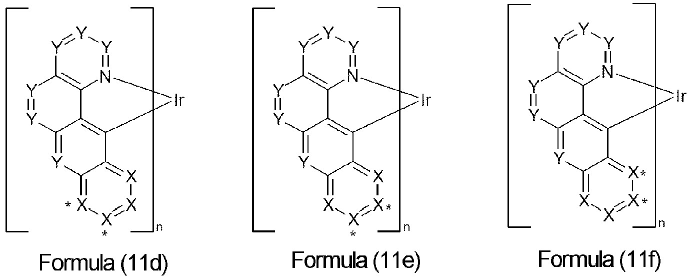 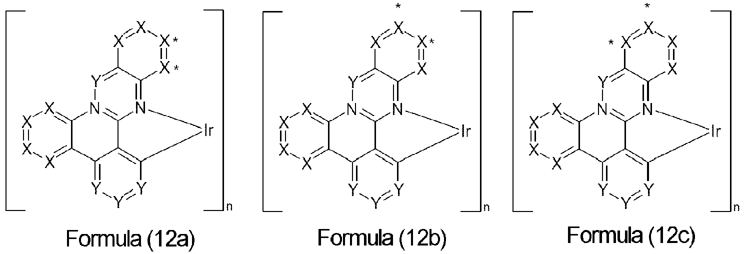 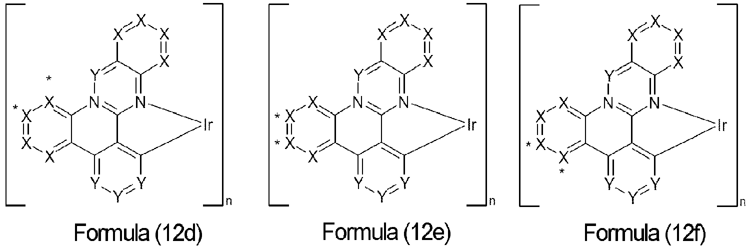 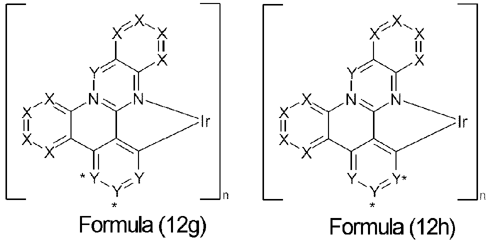 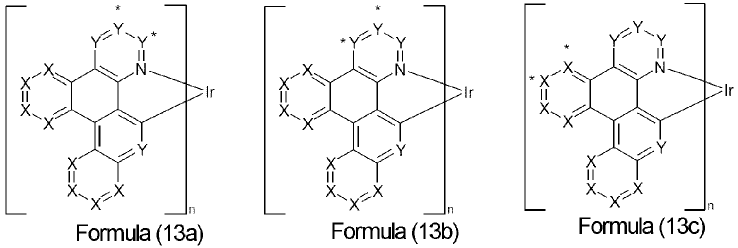 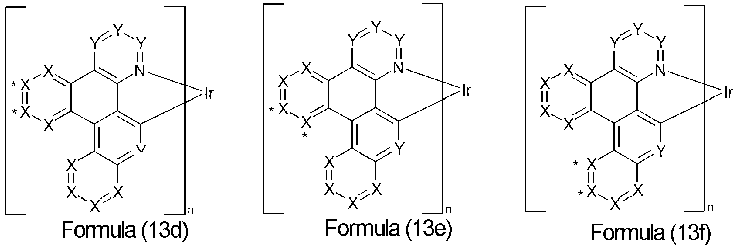 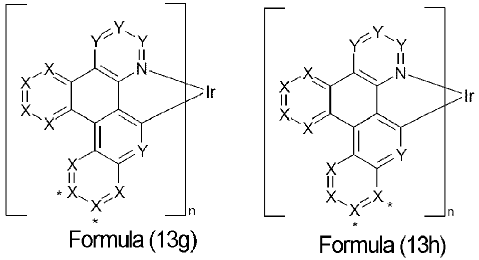 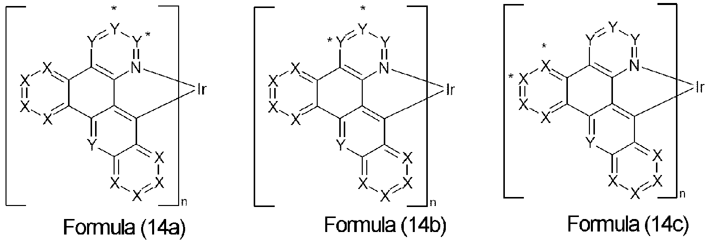 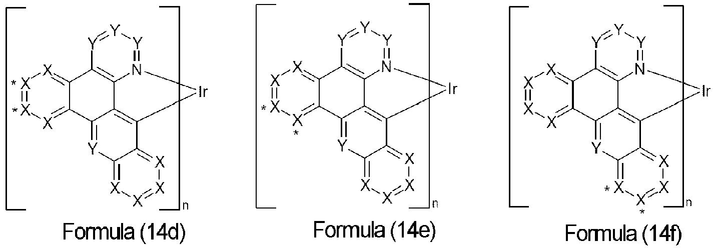 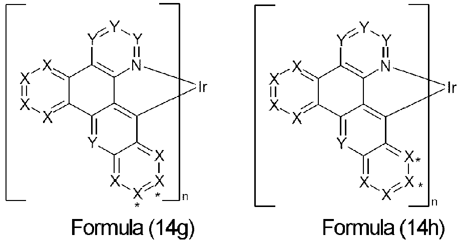 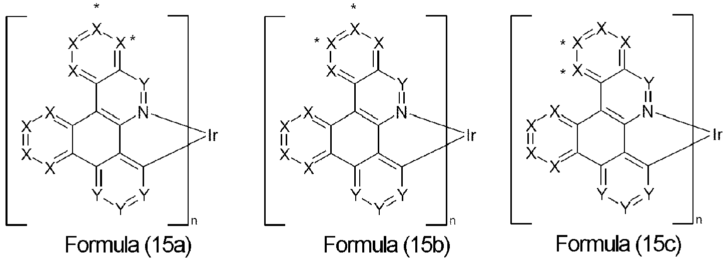 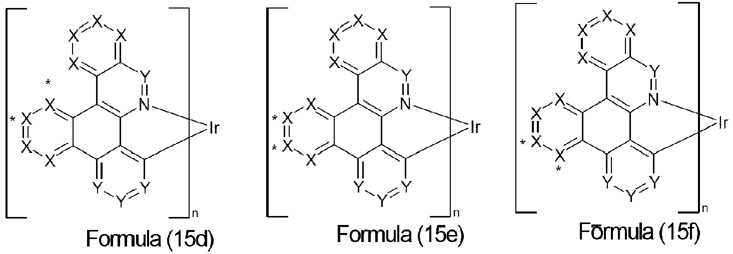 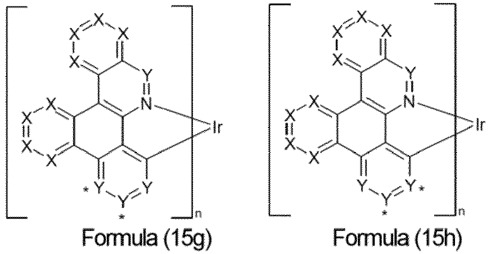 where the symbols and indices used are as defined in claim 1 and * in each case indicates the position at which the two adjacent Y or X groups are CR and the respective R radicals together with the carbon atoms form a ring of the formula (4) or formula (5).

8. Compound according to one or more of Claims 1 to 7, **characterized in that** the structure of formula (4) is selected from the formulae (4-A), (4-B), (4-D) and (4-E): 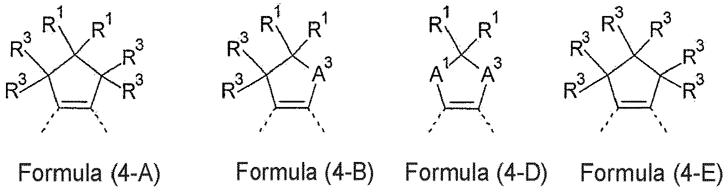 where R¹ and R³ are as defined in Claim 1 and A¹ and A³ are O,
and **in that** the structure of formula (5) is selected from the formulae (5-A) and (5-C): 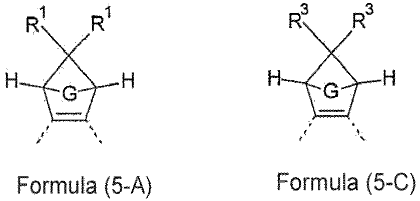 where the symbols used are as defined in Claim 1.

9. Compound according to one or more of Claims 1 to 8, **characterized in that** R³ is the same or different at each instance and is a straight-chain alkyl group having 1 to 10 carbon atoms or a branched or cyclic alkyl group having 3 to 10 carbon atoms or an aromatic ring system having 5 to 14 aromatic ring atoms; at the same time, two R³ radicals bonded to the same carbon atom may together form an aliphatic ring system and thus form a spiro system.

10. Process for preparing a compound according to one or more of Claims 1 to 9 by reacting the ligands with iridium alkoxides of the formula (44), with iridium ketoketonates of the formula (45), with iridium halides of the formula (46) or with dimeric iridium complexes of the formula (47) or (48): Ir(OR¹)ₙ Formula (44) 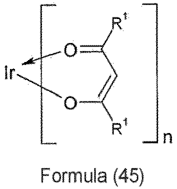 IrHalₙ Formula (46) 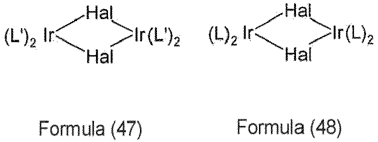 where the symbols and indices L', m, n and R¹ are as defined in Claim 1 and Hal = F, Cl, Br or I, or by reacting the ligands L with iridium complexes of the formula [Ir(L')₂(HOMe)₂]A or [Ir(L')₂(NCMe)₂]A or by reacting the ligands L' with iridium complexes of the formula [Ir(L)₂(HOMe)₂]A or [Ir(L)₂(NCMe)₂]A, where A in each case is a non-coordinating anion, or using iridium compounds having both alkoxide and/or halide and/or hydroxyl radicals and ketoketonate radicals as reactants.

11. Formulation comprising at least one compound according to one or more of Claims 1 to 9 and at least one further compound, especially a solvent.

12. Use of a compound according to one or more of Claims 1 to 9 in an organic electroluminescent device.

13. Organic electroluminescent device comprising at least one compound according to one or more of Claims 1 to 9.

14. Organic electroluminescent device according to Claim 13, **characterized in that** the compound according to one or more of Claims 1 to 9 is used as an emitting compound in one or more emitting layers.

15. Organic electroluminescent device according to Claim 14, **characterized in that** the matrix material used for the compound according to one or more of Claims 1 to 9 comprises ketones, phosphine oxides, sulfoxides, sulfones, triarylamines, carbazoles, indolocarbazoles, indenocarbazoles, azacarbazoles, bipolar matrix materials, azaboroles, boronic esters, diazasiloles, diazaphospholes, triazines, zinc complexes, beryllium complexes, dibenzofurans and/or bridged carbazole derivatives.

## Revendications

1. Composé selon la formule (1) :
[Ir(L)ₙ(L')ₘ] Formule (1)
le composé de la formule générale (1) contenant une structure partielle Ir(L)ₙ de la formule (2) : 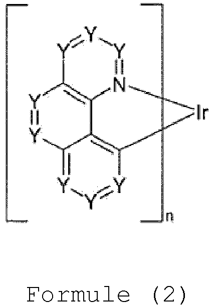 avec, pour les symboles et les indices utilisés :
les Y représentent, à chaque occurrence de manière identique ou différente, CR ou N, à condition qu'au plus un symbole Y par cycle représente N, ou deux symboles Y voisins représentent ensemble un groupe de la formule (3) suivante : 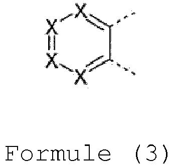
dans laquelle les liaisons en pointillés symbolisent la liaison de ce groupe dans le ligand ;
les X représentent, à chaque occurrence de manière identique ou différente, CR ou N, à condition qu'au plus deux symboles X par ligand représentent N ;
les R représentent, à chaque occurrence de manière identique ou différente, H, D, Cl, Br, I, CN, un groupe alkyle ou alcoxy linéaire de 1 à 40 atomes C, ou un groupe alkyle ou alcoxy ramifié ou cyclique de 3 à 40 atomes C, ou un système cyclique aromatique ou hétéroaromatique de 5 à 60 atomes de cycle aromatique, qui peut à chaque fois être substitué par un ou plusieurs radicaux R¹ ; deux radicaux R voisins ou davantage pouvant également former les uns avec les autres un système cyclique aliphatique mono- ou polycyclique ;
les R¹ représentent, à chaque occurrence de manière identique ou différente, H, un groupe alkyle linéaire de 1 à 40 atomes C, ou un groupe alkyle ramifié ou cyclique de 3 à 40 atomes C ;
les L' représentent, de manière identique ou différente à chaque occurrence, un ligand mono- ou bidentate ;
n représente 2 ou 3 ;
m représente 0 ou 1 ;
**caractérisé en ce que**, dans la structure partielle de la formule (2), deux groupes Y voisins représentent CR et les radicaux R respectifs établissent conjointement avec les atomes C un cycle de la formule (4) ou de la formule (5) suivante, et/ou **en ce que** deux groupes Y voisins représentent un groupe de la formule (3) et, dans ce groupe de la formule (3), deux groupes X voisins représentent CR et les radicaux R respectifs établissent conjointement avec les atomes C un cycle de la formule (4) ou de la formule (5) suivante, 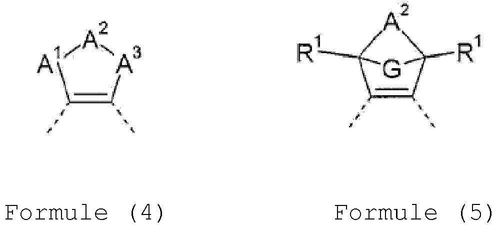
dans lesquelles R¹ et R² ont les significations indiquées précédemment, les liaisons en pointillés indiquent la liaison des deux atomes de carbone dans le ligand, et par ailleurs :
les A¹, A³ représentent, de manière identique ou différente à chaque occurrence, C(R³)₂ ou O ;
A² représente C(R¹)₂ ;
G représente un groupe alkylène contenant 2 atomes C ;
les R³ représentent, de manière identique ou différente à chaque occurrence, un groupe alkyle linéaire de 1 à 10 atomes C, un groupe alkyle ramifié ou cyclique de 3 à 10 atomes C, ou un système cyclique aromatique de 5 à 24 atomes de cycle aromatique ; deux radicaux R³ qui sont reliés au même atome de carbone pouvant former l'un avec l'autre un système cyclique aliphatique, et établir ainsi un système spiro.

2. Composé selon la revendication 1, **caractérisé en ce que** n = 3 et m = 0 ou **en ce que** n = 2 et m = 1, L' étant un ligand bidentate qui est coordonné par un atome de carbone et un atome d'azote, un atome de carbone et un atome d'oxygène, deux atomes d'oxygène, deux atomes d'azote ou un atome d'oxygène et un atome d'azote à l'iridium.

3. Composé selon la revendication 1 ou 2, **caractérisé en ce que** la structure partielle Ir(L)ₙ est choisie parmi les structures des formules (6) à (15) : 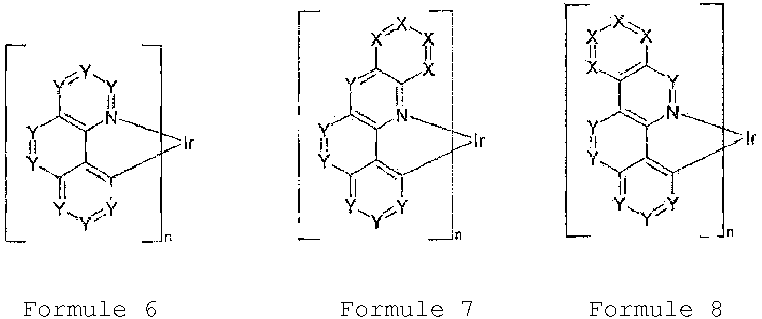 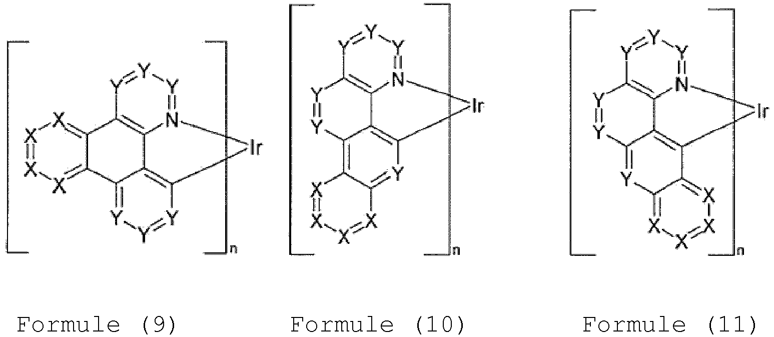 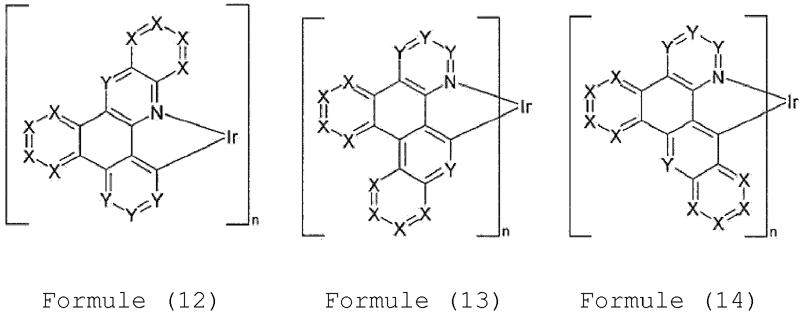 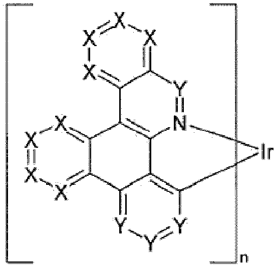 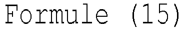 dans lesquelles les Y représentent, à chaque occurrence de manière identique ou différente, CR ou N, et les autres symboles et indices ont les significations indiquées dans la revendication 1.

4. Composé selon une ou plusieurs des revendications 1 à 3, **caractérisé en ce que**, dans le ligand L, au total 0, 1 ou 2 des symboles Y et, le cas échéant, X représentent N.

5. Composé selon une ou plusieurs des revendications 1 à 4, **caractérisé en ce que** la structure partielle Ir(L)ₙ est choisie parmi les structures des formules (6-1) à (6-7), (7-1) à (7-6), (8-1) à (8-5), (9-1) à (9-5), (10-1) à (10-5), (11-1) à (11-6), (12-1) à (12-4), (13-1) à (13-3), (14-1) à (14-4) et (15-1) à (15-3) : 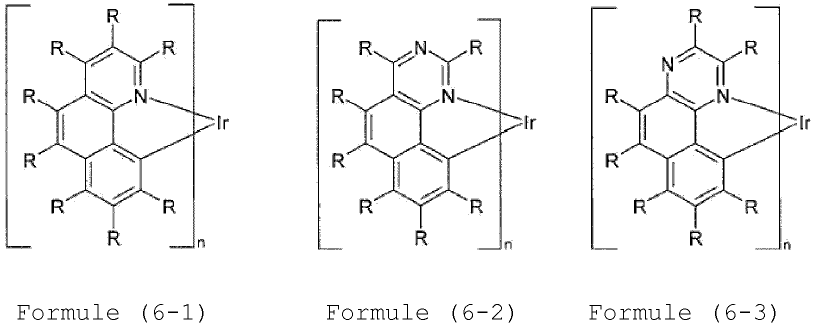 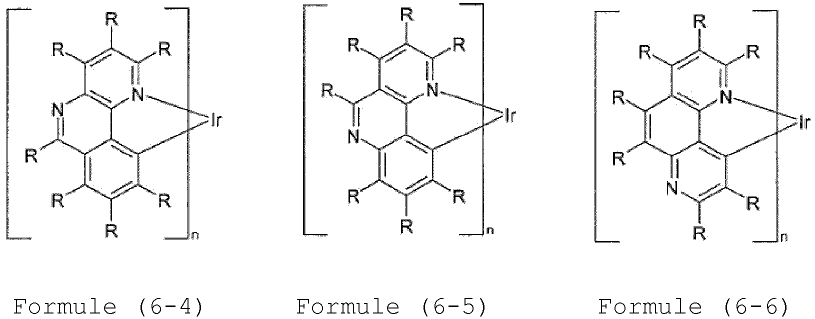 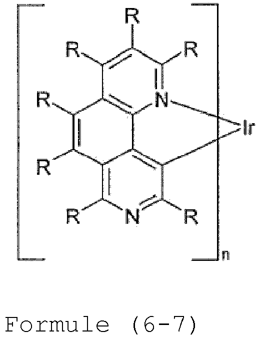 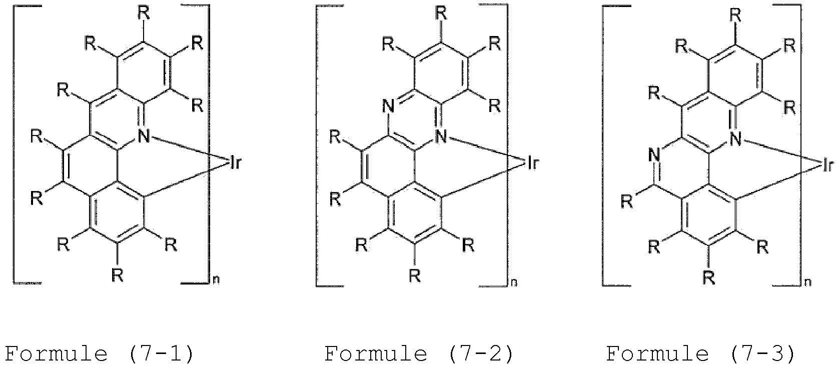 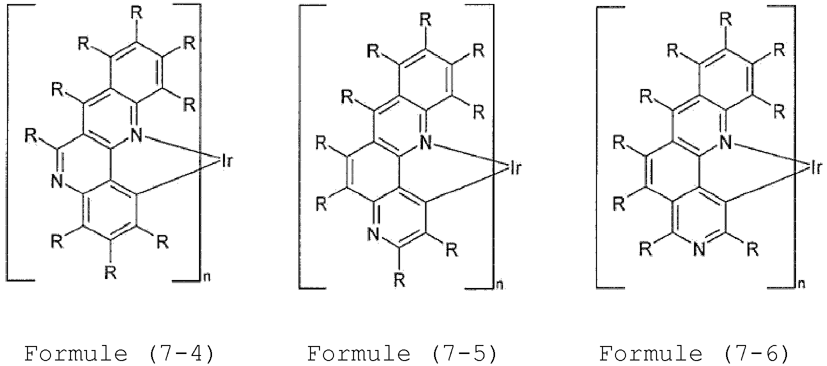 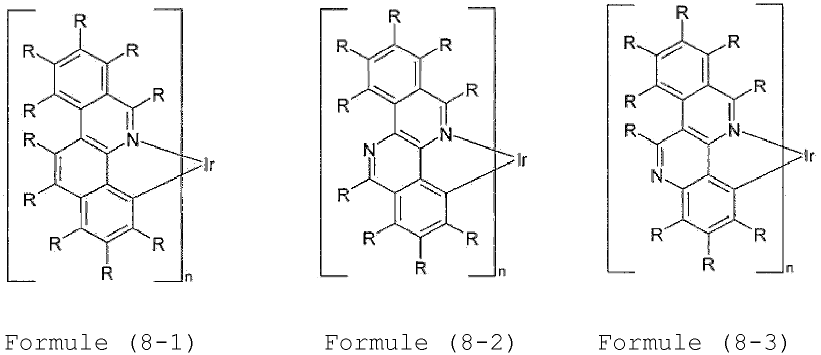 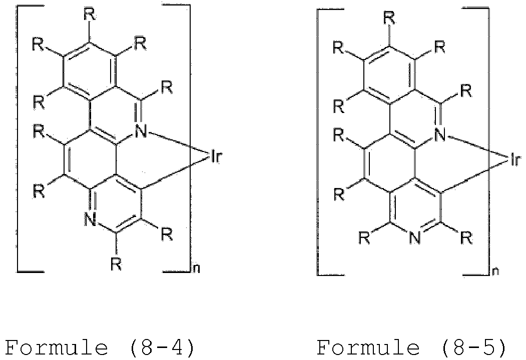 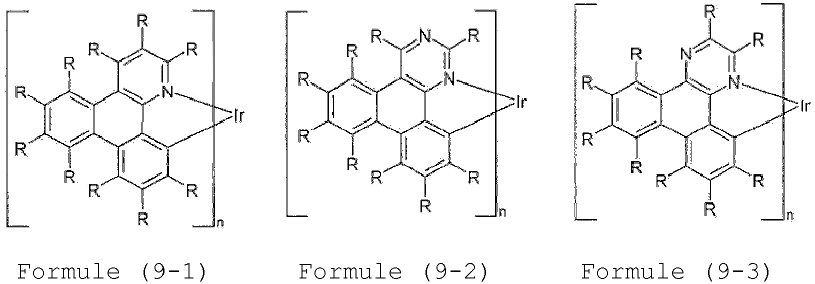 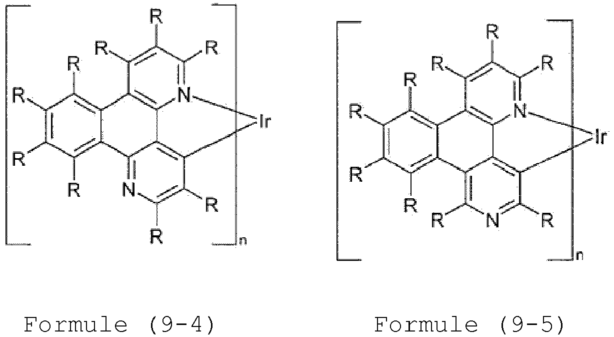 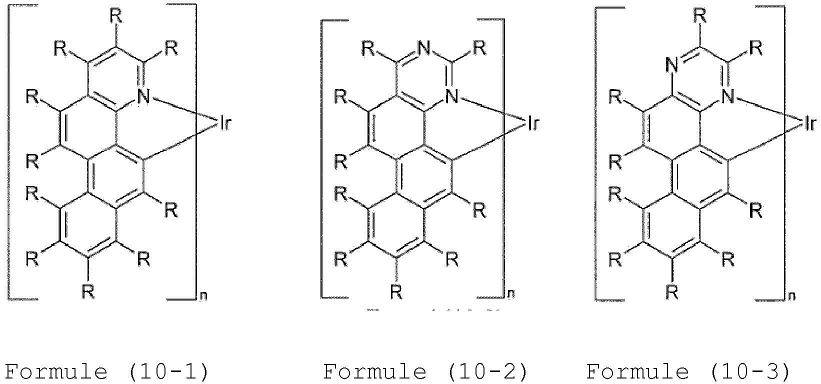 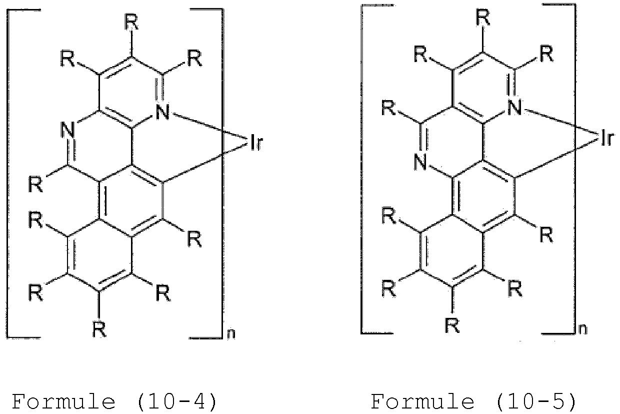 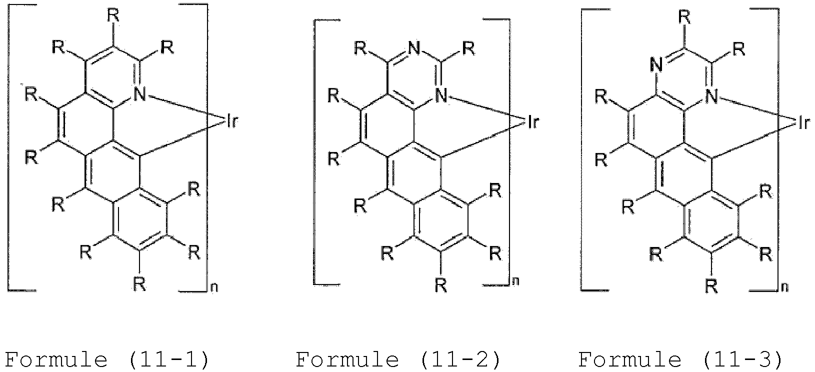 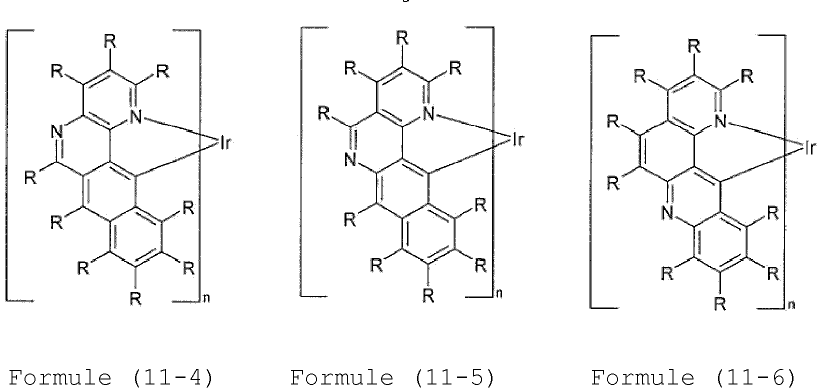 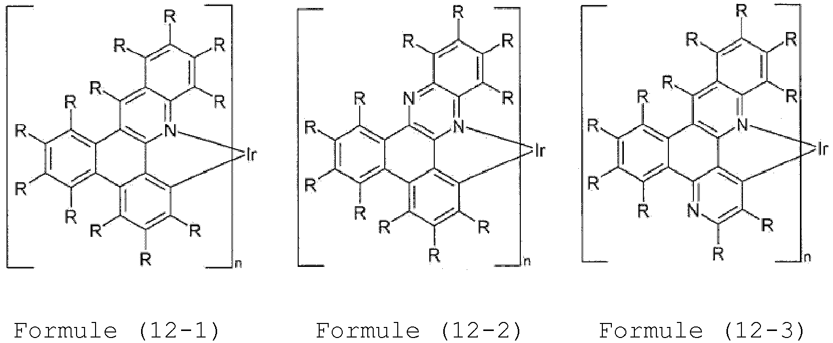 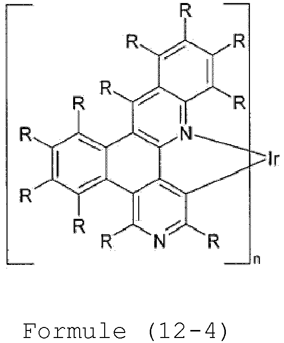 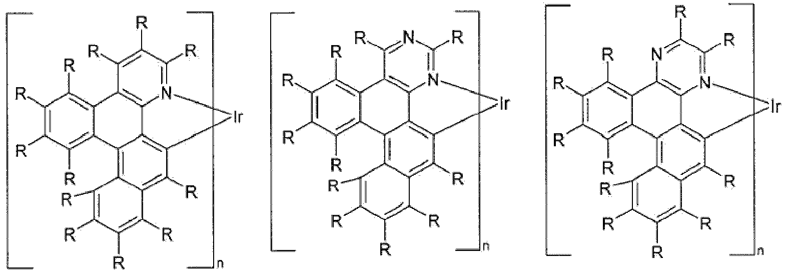 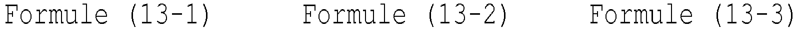 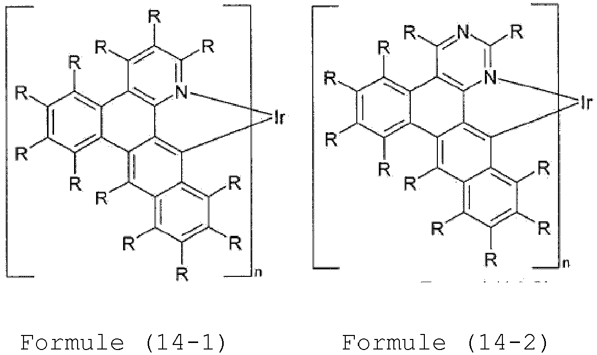 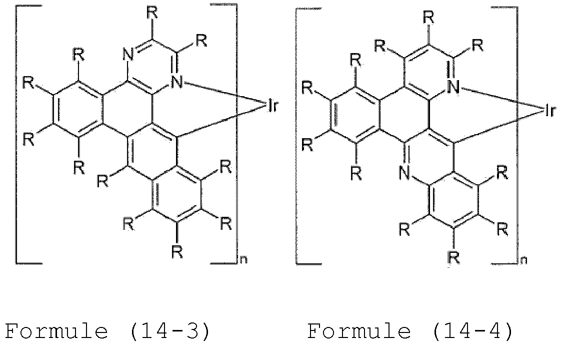 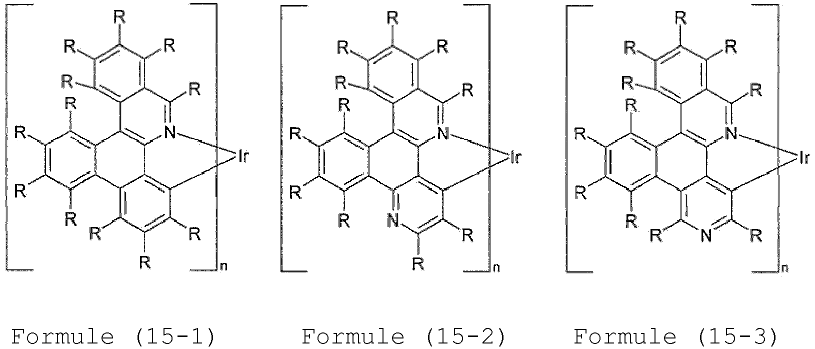 dans lesquelles les symboles et les indices utilisés ont les significations indiquées dans la revendication 1.

6. Composé selon une ou plusieurs des revendications 1 à 5, **caractérisé en ce qu'**un ou plusieurs des atomes Y et/ou X représentent N, et un substituant R est relié en position voisine de cet atome d'azote, qui est choisi dans le groupe constitué par les groupes alkyle ou alcoxy de 1 à 10 atomes C ou les systèmes cycliques aromatiques ou hétéroaromatiques qui peuvent être substitués par un ou plusieurs substituants R¹, ou **en ce que** ce radical R est relié dans une structure de la formule (4) ou (5).

7. Composé selon une ou plusieurs des revendications 1 à 6, **caractérisé en ce que** la structure partielle Ir(L)ₙ est choisie parmi les structures partielles des formules (6a) à (15h) : 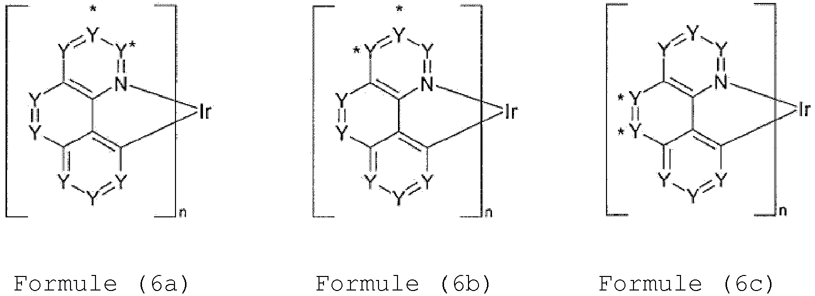 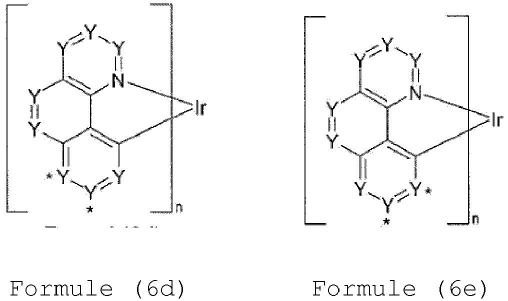 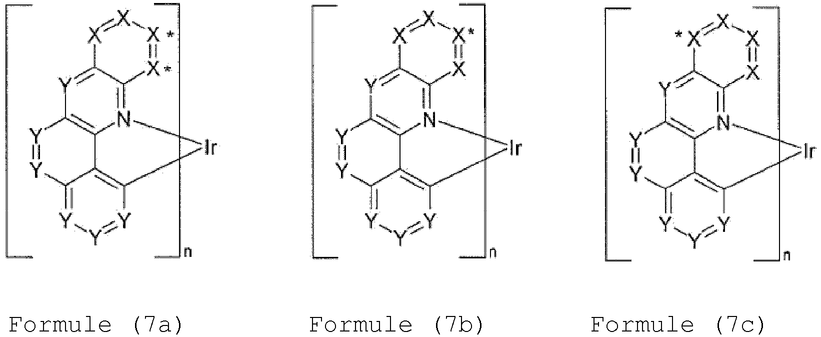 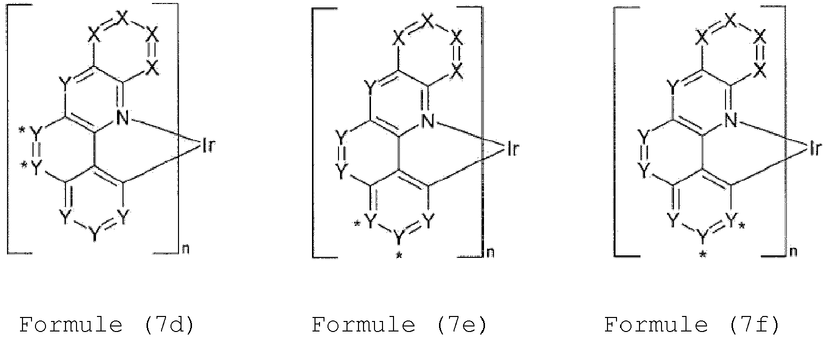 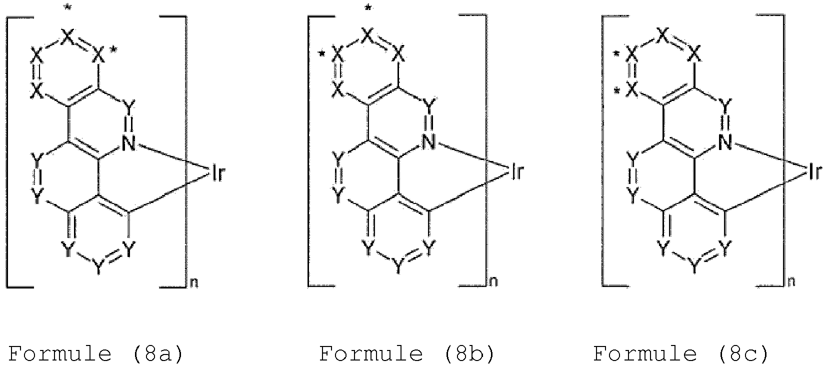 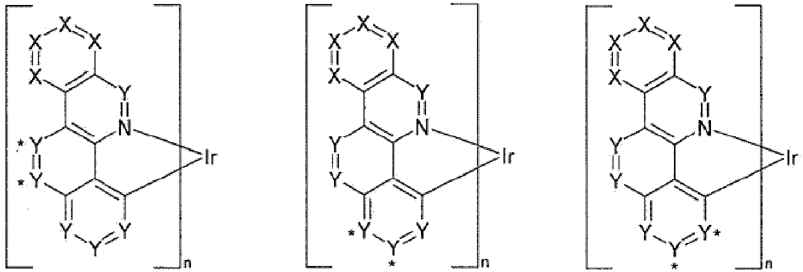 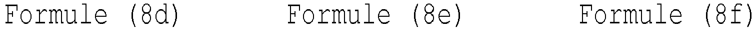 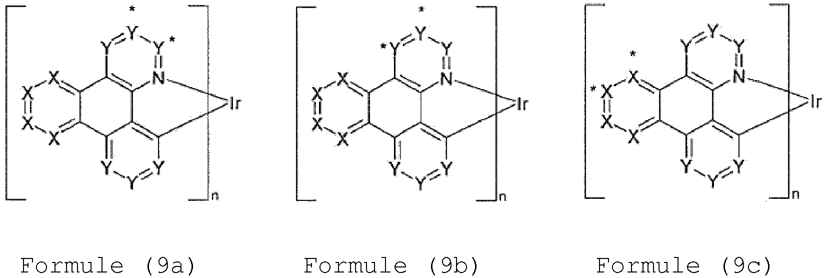 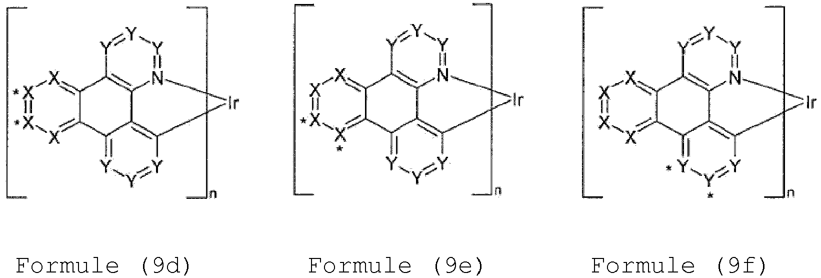 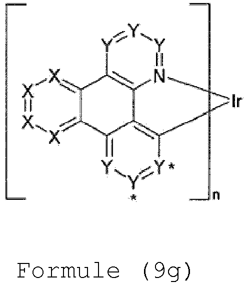 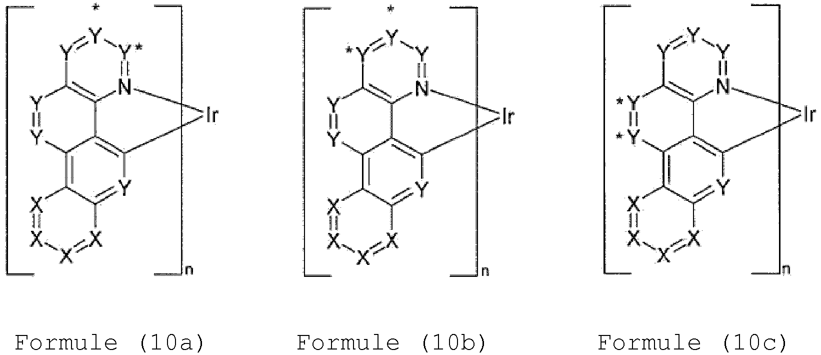 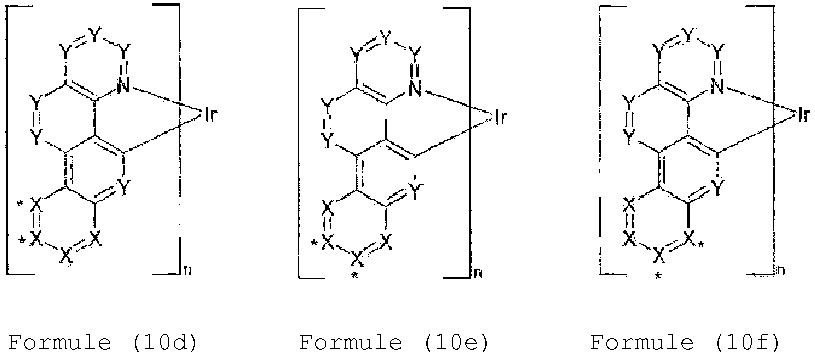 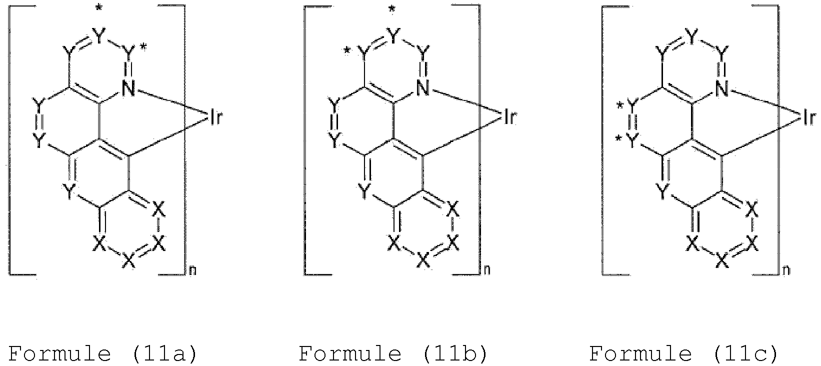 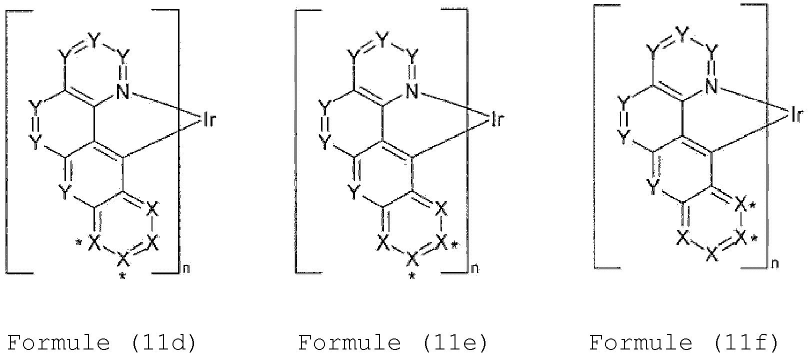 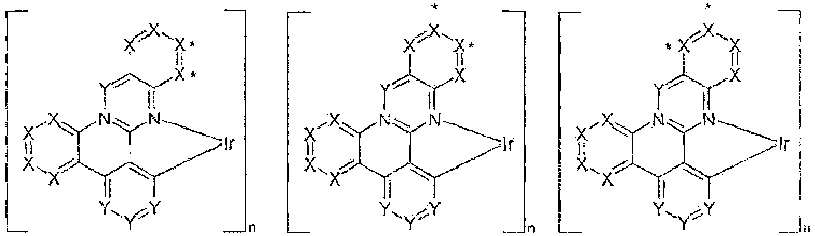 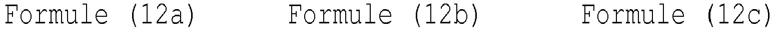 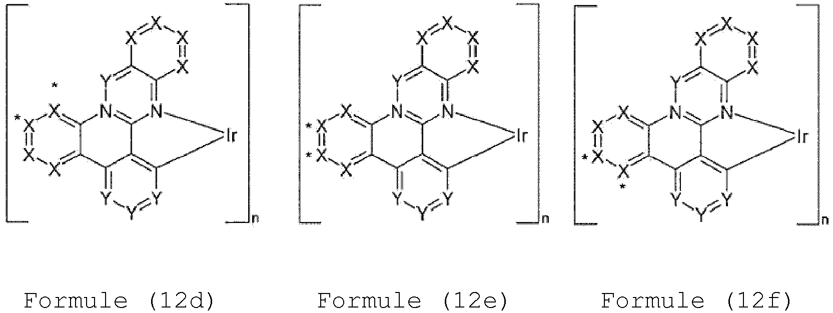 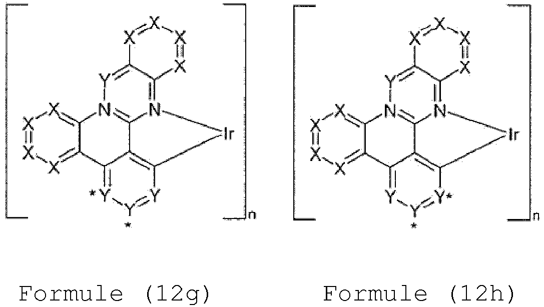 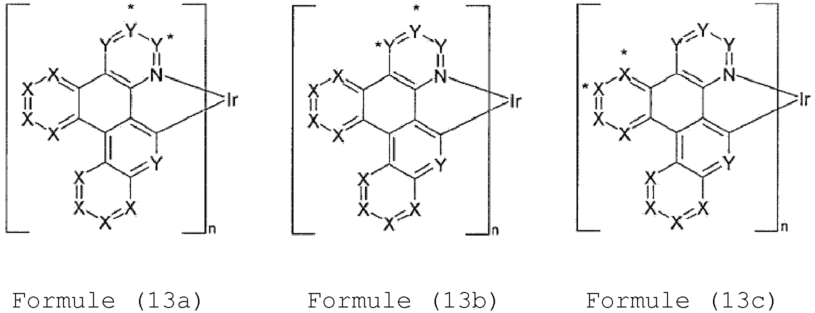 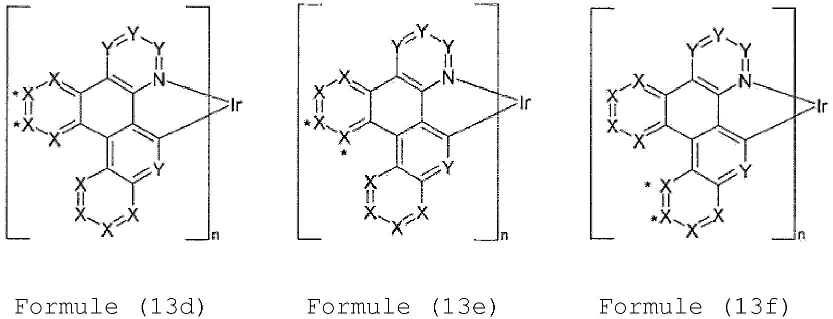 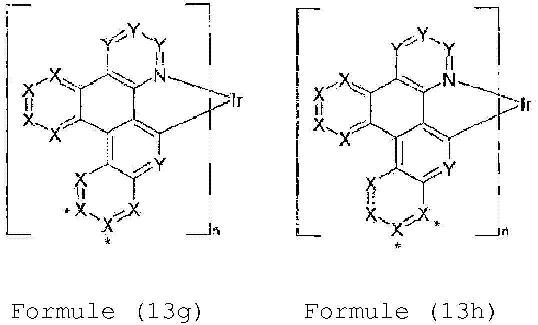 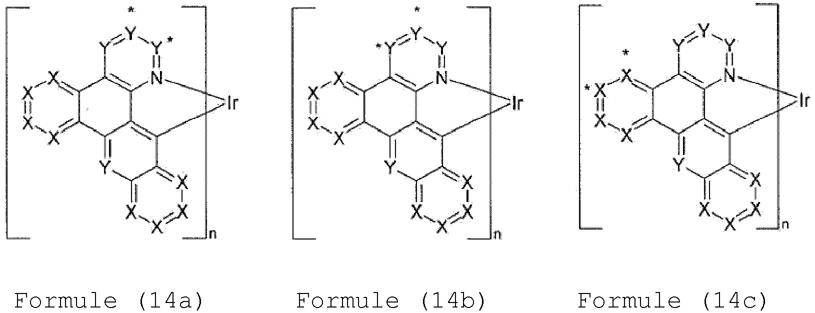 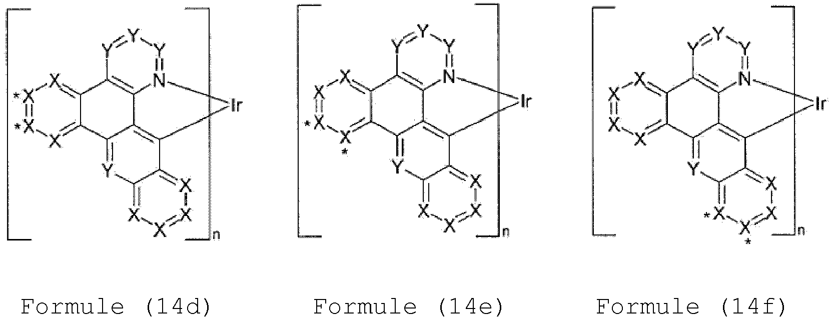 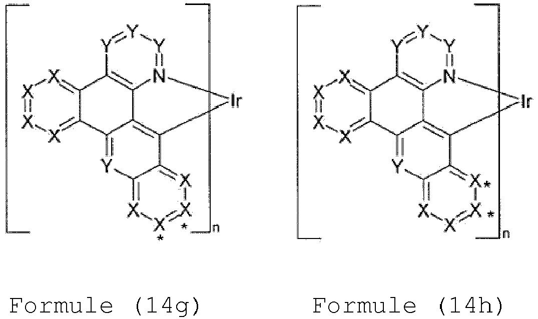 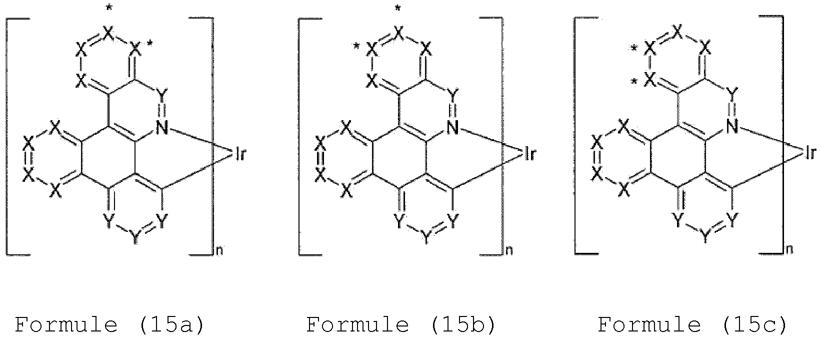 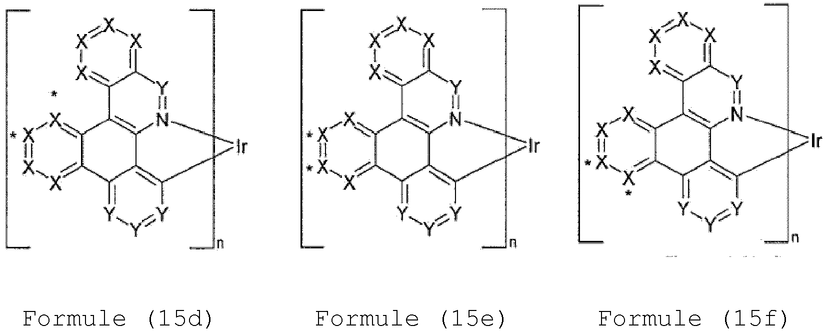 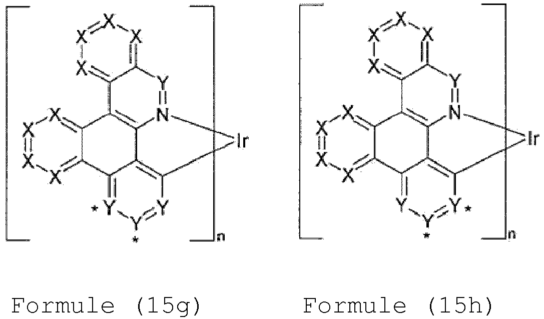 dans lesquelles les symboles et les indices utilisés ont les significations indiquées dans la revendication 1, et * désigne à chaque fois la position à laquelle les deux groupes Y ou X voisins représentent CR, et les radicaux R respectifs établissent conjointement avec les atomes C un cycle de la formule (4) ou de la formule (5).

8. Composé selon une ou plusieurs des revendications 1 à 7, **caractérisé en ce que** la structure selon la formule (4) est choisie parmi les formules (4-A), (4-B), (4-D) et (4-E) : 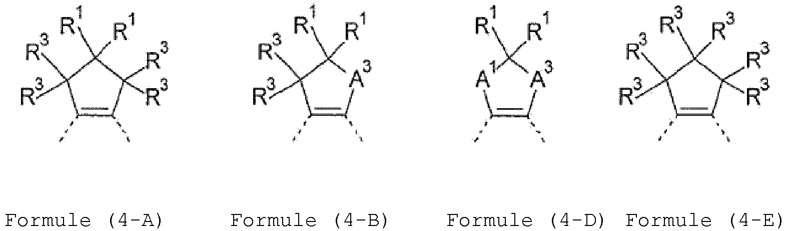 dans lesquelles R¹ et R³ ont les significations indiquées dans la revendication 1, et A¹ et A³ représentent O,
et **en ce que** la structure selon la formule (5) est choisie parmi les formules (5-A) et (5-C) : 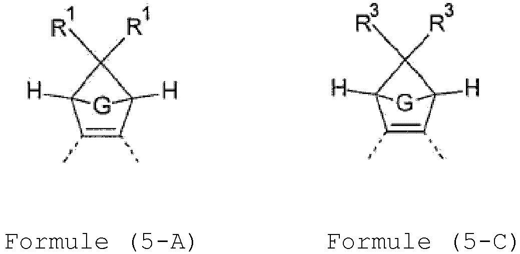 dans lesquelles les symboles utilisés ont les significations indiquées dans la revendication 1.

9. Composé selon une ou plusieurs des revendications 1 à 8, **caractérisé en ce que** les R³ représentent, de manière identique ou différente à chaque occurrence, un groupe alkyle linéaire de 1 à 10 atomes C, ou un groupe alkyle ramifié ou cyclique de 3 à 10 atomes C, ou un système cyclique aromatique de 5 à 14 atomes de cycle aromatique ; deux radicaux R³ qui sont reliés au même atome de carbone pouvant former l'un avec l'autre un système cyclique aliphatique, et établir ainsi un système spiro.

10. Procédé de fabrication d'un composé selon une ou plusieurs des revendications 1 à 9 par mise en réaction des ligands avec des alcoolates d'iridium de la formule (44), avec des cétocétonates d'iridium de la formule (45), avec des halogénures d'iridium de la formule (46) ou avec des complexes d'iridium dimères de la formule (47) ou (48) : 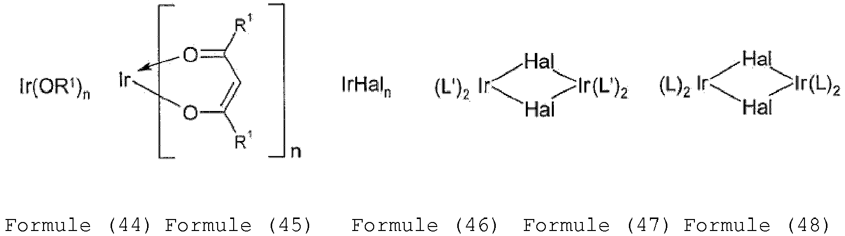 dans lesquelles les symboles et les indices L', m, n et R¹ ont les significations indiquées dans la revendication 1, et Hal = F, Cl, Br ou I, ou par mise en réaction des ligands L avec des complexes d'iridium de la formule [Ir(L')₂(HOMe)₂]A ou [Ir(L')₂(NCMe)₂]A, ou par mise en réaction des ligands L' avec des complexes d'iridium de la formule [Ir(L)₂(HOMe)₂]A ou [Ir(L)₂(NCMe)₂]A, A représentant à chaque fois un anion non coordinant, ou par utilisation de composés d'iridium qui portent aussi bien des radicaux alcoolate et/ou halogénure et/ou hydroxy que cétocétonate, en tant que réactifs.

11. Formulation, contenant au moins un composé selon une ou plusieurs des revendications 1 à 9 et au moins un composé supplémentaire, notamment un solvant.

12. Utilisation d'un composé selon une ou plusieurs des revendications 1 à 9 dans un dispositif électroluminescent organique.

13. Dispositif électroluminescent organique contenant au moins un composé selon une ou plusieurs des revendications 1 à 9.

14. Dispositif électroluminescent organique selon la revendication 13, **caractérisé en ce que** le composé selon une ou plusieurs des revendications 1 à 9 est utilisé en tant que composé d'émission dans une ou plusieurs couches d'émission.

15. Dispositif électroluminescent organique selon la revendication 14, **caractérisé en ce que** des cétones, des phosphinoxydes, des sulfoxydes, des sulfones, des triarylamines, des carbazoles, des indolocarbazoles, des indénocarbazoles, des azacarbazoles, des matériaux de matrice bipolaires, des azaboroles, des esters boroniques, des diazasiloles, des diazaphospholes, des triazines, des complexes de zinc, des complexes de béryllium, des dibenzofuranes et/ou des dérivés de carbazole pontés sont utilisés en tant que matériau de matrice pour le composé selon une ou plusieurs des revendications 1 à 9.
